(19) Europäisches Patentamt
European Patent Office
Office européen des brevets

(11) **EP 4 559 913 A1**

(12) **EUROPEAN PATENT APPLICATION**
published in accordance with Art. 153(4) EPC


(43) Date of publication:
**28.05.2025 Bulletin 2025/22**

(21) Application number: **23842356.0**

(22) Date of filing: **19.07.2023**

(51) International Patent Classification (IPC):
***C07D 471/04*** *(2006.01)* ***C07D 487/00*** *(2006.01)*
***C07D 487/04*** *(2006.01)* ***A61K 31/513*** *(2006.01)*
***A61K 31/517*** *(2006.01)* ***A61K 31/519*** *(2006.01)*
***A61P 35/00*** *(2006.01)*

(52) Cooperative Patent Classification (CPC):
**A61K 31/513; A61K 31/517; A61K 31/519; A61P 35/00; C07D 471/04; C07D 487/00; C07D 487/04**

(86) International application number:
**PCT/CN2023/108142**

(87) International publication number:
**WO 2024/017294 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**19.07.2022 CN 202210848185**
**30.08.2022 CN 202211051796**
**21.10.2022 CN 202211292824**
**16.11.2022 CN 202211432056**
**03.01.2023 CN 202310003195**
**30.03.2023 CN 202310329117**
**06.05.2023 CN 202310502696**



(71) Applicant: **Haisco Pharmaceutical Group Co., Ltd.**
**Shannan, Tibet 856000 (CN)**

(72) Inventors:
- **LI, Yao**
**Lhoka, Tibet 856099 (CN)**
- **SHI, Zongjun**
**Lhoka, Tibet 856099 (CN)**
- **CHEN, Lei**
**Lhoka, Tibet 856099 (CN)**
- **PEI, Yunpeng**
**Lhoka, Tibet 856099 (CN)**
- **SHU, Tianbo**
**Lhoka, Tibet 856099 (CN)**
- **WANG, Pengcheng**
**Lhoka, Tibet 856099 (CN)**
- **SHI, Shaohui**
**Lhoka, Tibet 856099 (CN)**
- **HE, Linkun**
**Lhoka, Tibet 856099 (CN)**
- **TANG, Pingming**
**Lhoka, Tibet 856099 (CN)**
- **ZHANG, Chen**
**Lhoka, Tibet 856099 (CN)**
- **YAN, Pangke**
**Lhoka, Tibet 856099 (CN)**

(74) Representative: **IP TRUST SERVICES**
**Europole - Le Grenat**
**3, avenue Doyen Louis Weil**
**38000 Grenoble (FR)**


(54) **PREPARATION AND USE OF QUINAZOLINONE DERIVATIVE AS KINASE INHIBITOR**


(57) Disclosed are a compound represented by formula I, a stereoisomer, deuterated product or pharmaceutically acceptable salt thereof, or a pharmaceutical composition containing same, as well as a use thereof as a BRAF modulator in the preparation of a drug for the treatment of related diseases. Each group shown in formula (I) is as defined in the description.


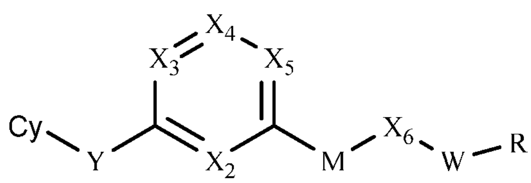

I

EP 4 559 913 A1

## Description

### Technical Field

The present invention belongs to the field of medicine, and particularly relates to a small molecule compound related to kinase (such as but not limited to BRAF kinase) disorder, and a stereoisomer, a deuterated product or a pharmaceutical acceptable salt thereof and a use thereof in the preparation of a drug for treatment of a related disease.

### Background Art

Kinases are enzymes that catalyze the transfer of phosphate groups from high-energy, phosphate-donating molecules to specific substrates. This process is called phosphorylation, in which the substrate gains a phosphate group and the high-energy ATP molecule donates a phosphate group. Kinases are divided into the following broad categories based on the substrates on which they act: protein kinases, lipid kinases, and carbohydrate kinases. Kinases are found in a variety of species, from bacteria to molds, to worms, and to mammals. More than five hundred different kinases have been identified in humans.

MAP kinases (MAPKs) are a family of serine/threonine kinases that respond to a variety of extracellular growth signals. For example, growth hormone, epidermal growth factor, platelet-derived growth factor, and insulin are all thought to participate in mitogenic stimulation of the MAPK pathway. Activation of this pathway at the receptor level initiates a signaling cascade whereby Ras GTPase exchanges GDP for GTP. Next, Ras activates Raf kinase (also known as MAPKKK), which activates MEK (MAPKK).

BRAF protein is a member of the RAF family of serine/threonine kinases that participates in cascades of the Ras Raf MEK extracellular signal-regulated kinase (ERK) pathway or the mitogen-activated protein kinase (MAPK)/ERK signaling pathway that affect cell division and differentiation. Mutations in the BRAF gene can lead to uncontrolled growth and subsequent tumor formation. BRAF is mutated and/or overactivated in common human cancers, such as melanoma, colorectal cancer, thyroid cancer, non-small cell lung cancer, and ovarian cancer and their metastatic cancers, and primary brain tumors. Although some BRAF inhibitors produce excellent extracranial responses, cancers may still develop brain metastases during or subsequent to BRAF inhibitor therapy. An estimated 20% of subjects with cancer will develop brain metastases, with the majority of brain metastases occurring in those with melanoma, colorectal cancer, lung cancer, and renal cell carcinoma. Brain metastases remain a substantial contributor to overall cancer mortality in subjects with advanced cancers, and despite multimodality treatments and advances in systemic therapies, including combinations of surgery, radiotherapy, chemotherapy, immunotherapy, and/or targeted therapies, the prognosis remains poor.

Additionally, BRAF has been identified as a potential target for the treatment of primary brain tumors. The prevalence of BRAF V600E mutations in primary brain tumors has been reported. Schindler et al analyzed 1,320 central nervous system (CNS) tumors and Behling et al analyzed 969 CNS tumors in pediatric and adult populations. These studies combined with others have reported the presence of BRAF V600E mutations in various cancers, including papillary craniopharyngioma, pleomorphic xanthoastrocytoma (PXA), ganglioglioma, astroblastoma, etc.

The blood-brain barrier (BBB) is a highly selective physical transport and metabolic barrier that separates the CNS from the blood. The BBB prevents certain drugs from entering brain tissue and is a limiting factor in the delivery of many peripherally administered agents to the CNS. Many drugs commonly used to treat cancer cannot cross the blood-brain barrier. This means that these drugs cannot penetrate the brain and therefore cannot effectively kill cancer cells in the brain. Current treatments for subjects with brain tumors include surgical resection, radiotherapy, and/or chemotherapy with agents such as temozolomide and/or bevacizumab. However, surgical treatment of brain cancer is not always possible; for example, the tumor may not be accessible or the subject may not be able to withstand the trauma of neurosurgery. Additionally, radiotherapy and treatments with cytotoxic agents are known to have undesirable side effects. For example, there is growing evidence that the use of temozolomide itself can induce mutations and worsen prognosis in a significant proportion of subjects, and the bevacizumab label has a boxed warning for gastrointestinal perforation, surgical and wound healing complications, and bleeding. Kinase inhibitors are used to treat many peripheral cancers. However, many kinase inhibitors such as BRAF inhibitors (e.g., vemurafenib and dabrafenib), due to their structural properties, are substrates of active transporters such as P-glycoprotein (P gp) or breast cancer resistance protein (BCRP). For example, dabrafenib was reported to have an MDR1 efflux ratio of 11.4, a BCRP efflux ratio of 21.0, and a total brain-to-plasma ratio of 0.023; Vemurafenib was reported to have an MDR1 efflux ratio of 83, a BCRP efflux ratio of 495, and a total brain-to-plasma ratio of 0.004.

Given that both P gp and BCRP are expressed in the endothelial cells lining blood-brain capillaries, the activity of both P gp and BCRP in the BBB plays a critical role in preventing the distribution of most kinase inhibitors in the brain parenchyma. Therefore, kinase inhibitors are generally not suitable for the treatment of tumors or cancers in the brain (which is protected by the BBB). Therefore, treatments against tumors harboring BRAF mutations are still needed. Additionally, there remains an unmet need for the treatment of CNS tumors, including CNS tumors harboring BRAF

mutations.

Summary of the Invention

The present invention provides a small molecule compound with BRAF regulating activity, a stereoisomer, a deuterated product or a pharmaceutically acceptable salt thereof. The compound can better penetrate the blood-brain barrier, has a higher brain-blood ratio, and has good activity, few side effects, excellent pharmacokinetics and high bioavailability.

The present invention provides a compound of the following formula I, a stereoisomer, a deuterated product or a pharmaceutically acceptable salt thereof,

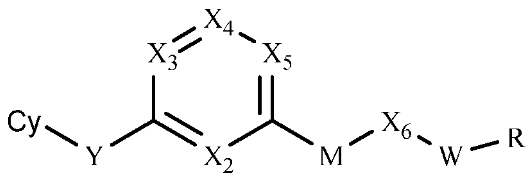

I

wherein, Cy is selected from P1, P2, P3, P4, P5, P6 or selected from P7 and P8;

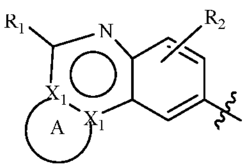
P1 ,

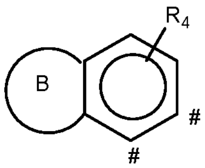
P2 ,

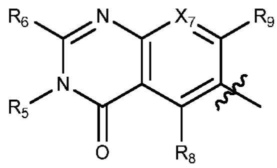
P3 ,

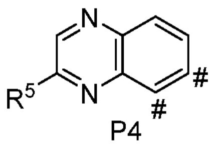
P4 ,

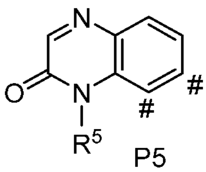
P5 , and

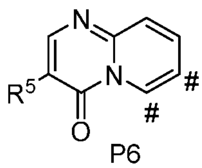
P6 ,

or Cy selected from

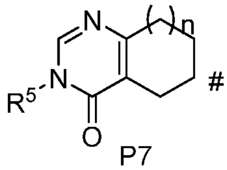
P7

and

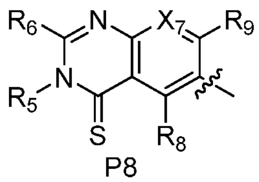
P8 ,

wherein, n = 0 or 1;

ring A is a 5-membered or 6-membered heteroaryl containing 1-3 heteroatoms selected from N, S, and O; in some embodiments, the heteroaryl is a 5-membered heteroaryl; in some embodiments, the heteroaryl is selected from pyrazolyl, oxazolyl, imidazolyl, triazole, thiazolyl, isoxazolyl, isothiazolyl, pyrrolyl, pyridyl, pyrimidinyl, etc., and the heteroaryl is optionally substituted with 1 or 2 groups selected from halogen, $C_{1-4}$ alkoxy, -NH$C_{1-4}$ alkyl, -N($C_{1-4}$ alkyl)$_2$, CN, $C_{1-4}$ alkyl, halo$C_{1-4}$ alkoxy and halo$C_{1-4}$ alkyl;

ring B is 5-membered or 6-membered heterocyclic ring containing 1-3 heteroatoms selected from N, S, and O; in some embodiments, the heterocyclic ring is a 5-membered heterocyclic ring; in some embodiments, the heterocyclic ring is a 6-membered heterocyclic ring; the heterocyclic ring includes heteroaryl and heterocycloalkyl. In some embodiments, ring B is a 5-membered heteroaryl or 5-membered heterocycloalkyl containing 1-3 heteroatoms selected from N, S, and O, such as pyrazolyl, oxazolyl, imidazolyl, triazole, thiazolyl, isoxazolyl, isothiazolyl , pyrrolyl, 1,3-dioxolyl, etc.; in some embodiments, the 6-membered heterocyclic ring includes pyridyl, pyrazolyl, pyrimidinyl, furyl, piperazinyl, etc., and the heterocyclic ring is optionally substituted with 1-3 groups selected from =O, halogen, $C_{1-4}$ alkyl, halo$C_{1-4}$ alkyl, $C_{1-4}$ alkoxy, OH and halo$C_{1-4}$ alkoxy;

# means that one point is selected to connect to Y;

each $X_1$ is independently N or C. In some embodiments, each $X_1$ is independently C;

$X_2$ is N or $CR_3$. In some embodiments, $X_2$ is N. In some embodiments, $X_2$ is $CR_3$;

$X_3$ is N or $CR_{31}$. In some embodiments, $X_3$ is N or CH;

$X_4$ is N or $CR_{32}$. In some embodiments, $X_4$ is N or CH;

$X_5$ is N or $CR_{33}$. In some embodiments, $X_5$ is N or CH;

$X_6$ is C(O), S(O) or S(O)$_2$. In some embodiments, $X_6$ is C(O) or S(O)$_2$. In some embodiments, $X_6$ is S(O)$_2$;

$X_7$ is $CR_7$ or N. In some embodiments, $X_7$ is N. In some embodiments, $X_7$ is CH;

$R_1$, $R_2$ and $R_4$ are independently H, halogen, OH, -NH$C_{1-4}$ alkyl, -N($C_{1-4}$ alkyl)$_2$, CN, $C_{1-4}$ alkyl, $C_{1-4}$ alkoxy, halo$C_{1-4}$ alkoxy, halo$C_{1-4}$ alkyl, $C_{2-4}$ alkenyl or $C_{2-4}$ alkynyl; the -NH$C_{1-4}$ alkyl includes -NH methyl, -NH ethyl, -NH isopropyl, -NH propyl, -NH butyl, and the -N($C_{1-4}$ alkyl)$_2$ includes dimethylamino, diethylamino, etc;

$R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -NH$C_{1-4}$ alkyl, -N($C_{1-4}$ alkyl)$_2$, CN or $C_{3-6}$ cycloalkyl. In some embodiments, $R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -NH$C_{1-4}$ alkyl, -N($C_{1-4}$ alkyl)$_2$ or CN. In some embodiments, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$, or CN. In some embodiments, $R_3$ is $C_{1-4}$ alkyl, halo$C_{1-4}$ alkyl, $C_{1-4}$ alkoxy, halo$C_{1-4}$ alkoxy, OH, $NH_2$, -NH$C_{1-4}$ alkyl, or - N($C_{1-4}$alkyl)$_2$. In some embodiments, $R_3$ is H, $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN.

Alternatively, $R_{31}$ and $R_{32}$, or $R_{32}$ and $R_{33}$ together with the atoms to which they are attached form a $C_{3-6}$ carbocyclic ring or 5-membered or 6-membered heterocyclic ring containing 1-3 heteroatoms selected from N, S, and O. The carbocyclic ring includes aryl and cycloalkyl. The heterocyclic ring includes heteroaryl and heterocycloalkyl, and is optionally substituted with 1-3 groups selected from halogen, $C_{2-4}$ alkenyl, $C_{2-4}$ alkynyl, $C_{1-4}$ alkyl, halo$C_{1-4}$ alkyl, $C_{1-4}$ alkoxy, OH, $NH_2$ and CN. In some embodiments, the heterocyclic ring is optionally substituted with 1-3 groups selected from halogen, $C_{1-4}$ alkyl, halo$C_{1-4}$ alkyl, $C_{1-4}$ alkoxy, $C_{1-4}$ haloalkoxy and CN. In some embodiments, $R_{31}$ and $R_{32}$, or $R_{32}$ and $R_{33}$ together with the atoms to which they are attached form $C_{3-6}$ cycloalkyl, such as cyclopropenyl, cyclobutenyl, cyclopentenyl, or form a 5-membered or 6-membered heterocycloalkyl containing 1-3 heteroatoms selected from N, S, and O, such as azetinyl, oxetenly, azacyclopentyl, oxacyclopentyl, azacyclohexenyl, and oxacyclohexenyl, and the cycloalkyl and the heterocycloalkyl is optionally substituted with 1-3 groups selected from halogen, $C_{1-4}$ alkyl, halo$C_{1-4}$ alkyl, $C_{1-4}$ alkoxy, $C_{1-4}$ haloalkoxy and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and the cycloalkyl includes but is not limited to cyclopropyl, cyclobutyl, cyclopentyl and cyclohexyl, the heterocycloalkyl includes but is not limited to aziridinyl, azetidinyl, tetrahydropyrrolyl, piperidinyl, piperazinyl, oxacyclopropanyl, oxetanyl, tetrahydrofuranyl, tetrahydropyranyl, morpholinyl, etc., and is optionally substituted with 1-3 groups selected from halogen, -NH$C_{1-4}$ alkyl, -N($C_{1-4}$ alkyl)$_2$, $C_{1-4}$ alkoxy, halo$C_{1-4}$ alkoxy, halo$C_{1-4}$ alkyl, CN, $C_{1-4}$ alkyl and =O. In some embodiments, $R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1-3 groups selected from halogen, -NH$C_{1-4}$ alkyl, -N($C_{1-4}$ alkyl)$_2$, $C_{1-4}$ alkoxy, halo$C_{1-4}$ alkoxy, halo$C_{1-4}$ alkyl, CN and $C_{1-4}$ alkyl. In some embodiments, $R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, OH, $NH_2$, -NH$C_{1-4}$ alkyl, -N($C_{1-4}$ alkyl)$_2$, CN and $C_{1-4}$ alkyl. In some embodiments, $R_5$ is optionally substituted with 1 to 3 groups selected from halogen, -NH$C_{1-4}$ alkyl, -N($C_{1-4}$ alkyl)$_2$, $C_{1-4}$ alkoxy, halo$C_{1-4}$ alkoxy, halo$C_{1-4}$ alkyl, CN and $C_{1-4}$ alkyl. In some

embodiments, $R_5$ is substituted or unsubstituted $C_{3-6}$ cycloalkyl, substituted or unsubstituted 4-membered to 7-membered heterocycloalkyl containing 1-3 heteroatoms selected from N, S and O, substituted or unsubstituted $C_{3-6}$ cycloalkyloxy, substituted or unsubstituted $C_{1-4}$ alkoxy or substituted or unsubstituted halo$C_{1-4}$ alkoxy, the substituent is as described above. In some embodiments, $R_5$ is $C_{1-2}$ alkyl, CN substituted $C_{1-2}$ alkyl, halo$C_{1-2}$ alkyl, $C_{3-4}$ cycloalkyl, 4-membered to 7-membered heterocycloalkyl containing 1-3 heteroatoms selected from N, S, and O, $C_{1-2}$ alkoxy, halo$C_{1-2}$ alkoxy or $C_{3-4}$ cycloalkyloxy. In some embodiments, $R_5$ is selected from $C_{1-2}$ alkyl, CN substituted $C_{1-2}$ alkyl, halo$C_{1-2}$ alkyl or $C_{1-2}$ alkoxy. In some embodiments, $R_5$ is selected from $-CH_2-CN$, $-CH_2CH_2F$, $-CH_2CHF_2$, $-CH_2CF_3$, $-CH_2F$, $-CHF_2$ or $-CF_3$.

$R_6$ is H, halogen, $C_{1-4}$ alkyl or halo$C_{1-4}$ alkyl. In some embodiments, $R_6$ is H.

Alternatively, $R_5$ and $R_6$ together with the atoms to which they are attached form 5-membered or 6-membered heterocyclic ring containing 1 to 3 heteroatoms selected from N, S, and O, and the heterocyclic ring includes heteroaryl and heterocycloalkyl. In some embodiments, a 5-membered heterocyclic ring is formed. In some embodiments, 5-membered cycloalkyl is formed, and is optionally substituted with 1 to 3 groups selected from halogen, OH, $NH_2$, $-NHC_{1-4}$ alkyl, $-N(C_{1-4}$ alkyl$)_2$, CN and $C_{1-4}$ alkyl;

$R_7$, $R_8$, and $R_9$ are independently H, halogen, OH, $NH_2$, $-NHC_{1-4}$ alkyl, $-N(C_{1-4}$ alkyl$)_2$, CN, $C_{1-4}$ alkyl, halo$C_{1-4}$ alkyl, $C_{1-4}$ alkoxy or halo$C_{1-4}$ alkoxy. In some embodiments, $R_7$, $R_8$, and $R_9$ are independently H, halogen, $-NHC_{1-4}$ alkyl, $-N(C_{1-4}$ alkyl$)_2$, CN, $C_{1-4}$ alkyl, $C_{2-4}$ alkenyl, $C_{2-4}$ alkynyl, halo$C_{1-4}$ alkyl, $C_{1-4}$ alkoxy or halo$C_{1-4}$ alkoxy. In some embodiments, $R_7$ is H or halogen. In some embodiments, $R_8$ is halogen, OH, $NH_2$, $-NHC_{1-4}$ alkyl, $-N(C_{1-4}$ alkyl$)_2$, CN, $C_{1-4}$ alkoxy or halo$C_{1-4}$ alkoxy. In some embodiments, $R_8$ is halogen, $-NHC_{1-4}$ alkyl, $-N(C_{1-4}$ alkyl$)_2$, CN, $C_{1-4}$ alkoxy or halo$C_{1-4}$ alkoxy. In some embodiments, $R_9$ is H or halogen;

Y is $C_{1-2}$ alkylene, O or $NR_y$. In some embodiments, Y is O. In some embodiments, Y is NH. In some embodiments, Y is methylene or ethylene;

$R_y$ is H or $C_{1-4}$ alkyl. In some embodiments, $R_y$ is H. In some embodiments, $R_y$ is methyl, ethyl, propyl, isopropyl, etc.;

M is O or $NR_m$. In some embodiments, M is NH. In some embodiments, M is methylimino, ethylimino, propylimino or isopropylimino;

W is a bond, O or $NR_w$. In some embodiments, W is a bond, O, NH or $NC_{1-4}$ alkyl;

$R_m$ and $R_w$ are independently H or $C_{1-4}$ alkyl. In some embodiments, $R_m$ and $R_w$ are independently H, methyl, ethyl, propyl or isopropyl;

R is selected from $C_{1-4}$ alkyl, $C_{3-8}$ cycloalkyl, 4-membered to 10-membered heterocyclic ring containing 1 to 3 heteroatoms selected from N, S, and O, or $-O-C_{3-6}$ cycloalkyl, and the alkyl, cycloalkyl or heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, R is selected from $C_{1-4}$ alkyl, $C_{3-8}$ cycloalkyl or 4-membered to 10-membered heterocyclic ring containing 1 to 3 heteroatoms selected from N, S, and O, and the alkyl, cycloalkyl and heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, R is selected from $C_{3-8}$ cycloalkyl or 4-membered to 10-membered heterocyclic ring containing 1 to 3 heteroatoms selected from N, S, and O, and is optionally substituted with 1 to 3 groups selected from halogen, $C_{1-4}$ alkyl, $C_{1-4}$ alkoxy, halo$C_{1-4}$ alkoxy, OH, $NH_2$, $-$ $NHC_{1-4}$ alkyl, $-N(C_{1-4}$ alkyl$)_2$ and halo$C_{1-4}$ alkyl. In some embodiments, R is $C_{3-4}$ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, R is selected from $C_{1-4}$ alkyl, $C_{3-8}$ cycloalkyl or 4-membered to 10-membered heterocycloalkyl containing 1 to 3 heteroatoms selected from N, S, and O, and the alkyl, the cycloalkyl and the heterocycloalkyl is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, R is selected from $C_{1-4}$ alkyl, $C_{3-8}$ cycloalkyl or 4-membered to 7-membered monocyclic heterocycloalkyl containing 1 to 3 heteroatoms selected from N, S, and O, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, R is saturated 4-membered to 7-membered monocyclic heterocycloalkyl containing 1 to 3 heteroatoms selected from N, S, and O, saturated 5-membered to 10-membered fused heterocycloalkyl, saturated 5-membered to 10-membered bridged heterocycloalkyl or saturated 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl. In some embodiments, the cycloalkyl is selected from cyclopropyl, cyclobutyl, cyclopentyl,

cyclohexyl, cyclopentenyl, cyclohexenyl, etc., the monocyclic heterocycloalkyl is selected from aziridinyl, azetidinyl, tetrahydropyrrolyl, piperidinyl, piperazinyl, oxacyclopropanyl, oxetanyl, tetrahydrofuranyl, tetrahydropyranyl, morpholinyl, etc., the fused heterocycloalkyl is selected from

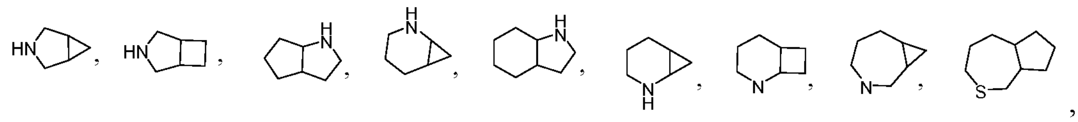

etc., the bridged heterocycloalkyl is selected from

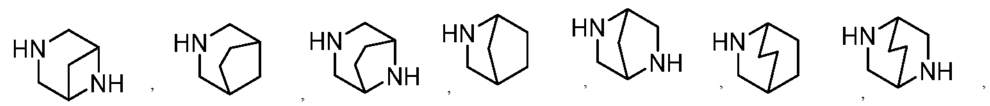

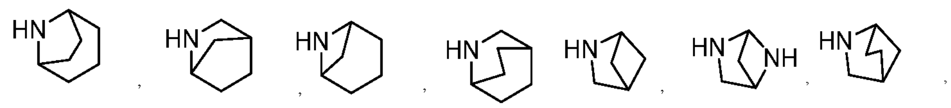

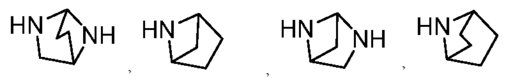

etc., and the spirocyclic heterocycloalkyl is selected from:

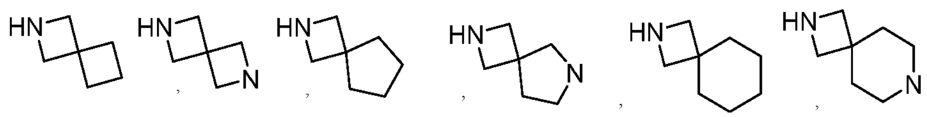

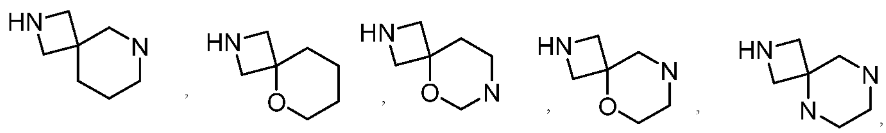

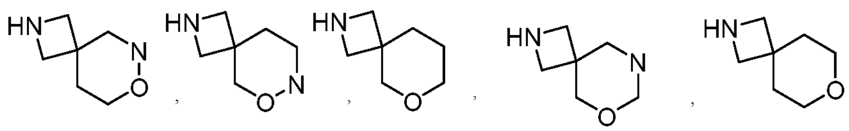

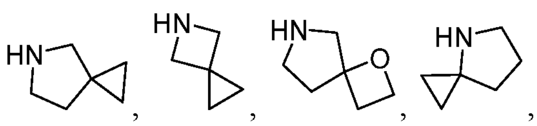

etc.

when Cy is P2, R is 4-membered to 10-membered saturated heterocycloalkyl containing 1 to 3 heteroatoms selected from N, S, and O, and is optionally substituted with 1 to 3 groups selected from halogen, $C_{1-4}$ alkyl, $C_{1-4}$ alkoxy, OH, $NH_2$, $-NHC_{1-4}$ alkyl, $-N(C_{1-4}\ alkyl)_2$ and halo$C_{1-4}$ alkyl.

In some embodiments, when Cy is P3, the compound meets one of the following conditions:

(1) $R_5$ is substituted or unsubstituted $C_{3-6}$ cycloalkyl, substituted or unsubstituted 4-membered to 7-membered heterocycloalkyl containing 1-3 heteroatoms selected from N, S, and O, substituted or unsubstituted $C_{3-6}$ cycloalkyloxy, substituted or unsubstituted $C_{1-4}$ alkoxy or substituted or unsubstituted halo$C_{1-4}$ alkoxy, R is $C_{3-8}$ cycloalkyl, 4-membered to 10-membered heterocyclic ring containing 1 to 3 heteroatoms selected from N, S, and O, or $-O-C_{3-6}$ cycloalkyl, and is optionally substituted with 1 to 3 groups selected from halogen, $C_{1-4}$ alkyl, $C_{1-4}$ alkoxy, halo$C_{1-4}$

alkoxy, OH, $NH_2$, -$NHC_{1-4}$ alkyl, -N($C_{1-4}$ alkyl)$_2$ and halo$C_{1-4}$ alkyl;

(2) $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -$NHC_{1-4}$ alkyl, -N($C_{1-4}$ alkyl)$_2$, CN and $C_{1-4}$ alkyl;

(3) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN, W is O;

(4) $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, $C_{1-4}$ haloalkoxy and CN;

(5) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN, W is $NR_w$, R is $C_{3-8}$ cycloalkyl or 4-membered to 10-membered heterocyclic ring containing 1 to 3 heteroatoms selected from N, S, and O, and is optionally substituted with 1 to 3 groups selected from halogen, $C_{1-4}$ alkyl, $C_{1-4}$ alkoxy, $C_{1-4}$ haloalkoxy, OH, $NH_2$, - $NHC_{1-4}$ alkyl, -N($C_{1-4}$ alkyl)$_2$ and halo$C_{1-4}$ alkyl;

(6) $X_2$ is N or $CR_3$, $X_6$ is SO or $SO_2$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN, R is $C_{3-4}$ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl)$_2$ and halo$C_{1-4}$ alkyl;

(7) $X_6$ is CO, $X_2$ is N or $CR_3$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN;

(8) at least one of $X_2$, $X_3$, $X_4$, and $X_5$ is N, when $X_2$ is $CR_3$, $R_3$ is **H,** $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN;

(9) $X_2$ is N or $CR_3$, $R_3$ is **H,** $C_{1-4}$ alkyl, halo$C_{1-4}$ alkyl, $C_{1-4}$ alkoxy, halo$C_{1-4}$ alkoxy, -$NHC_{1-4}$ alkyl, $C_{2-4}$ alkenyl, $C_{2-4}$ alkynyl, -N($C_{1-4}$ alkyl)$_2$ or CN, $R_8$ is halogen, -$NHC_{1-4}$ alkyl, -N($C_{1-4}$ alkyl)$_2$, CN, $C_{1-4}$ alkoxy or halo$C_{1-4}$ alkoxy.

A specific technical solution 1 of the present invention provides a compound represented by formula **I,** and a stereoisomer, a deuterated product or a pharmaceutically acceptable salt thereof,

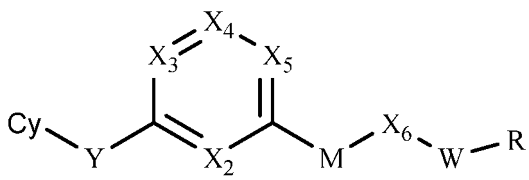

I

wherein, Cy is selected from P1, P2, P3, P4, P5, P6, P7 or P8;

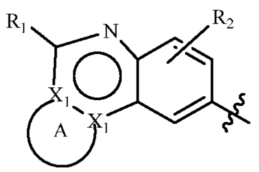
P1 ,
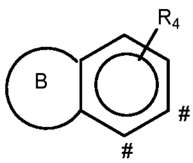
P2 ,
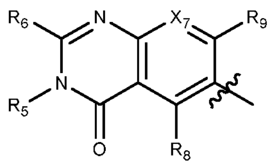
P3 ,
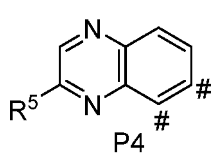
P4 ,

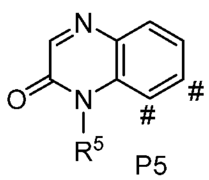
P5 ,
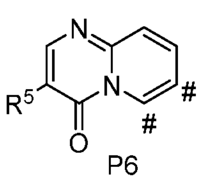
P6 ,
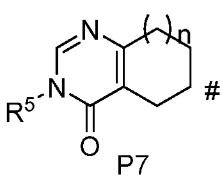
P7 ,
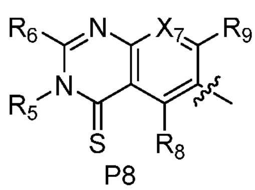
P8 ;

ring A is 5-membered or 6-membered heteroaryl containing 1 to 3 heteroatoms selected from N, S, and O, and the heteroaryl is optionally substituted with 1 or 2 groups selected from halogen, $C_{1-4}$ alkoxy, -$NHC_{1-4}$ alkyl, -N($C_{1-4}$ alkyl)$_2$, CN, $C_{1-4}$ alkyl, halo$C_{1-4}$ alkoxy and halo$C_{1-4}$ alkyl;

ring B is 5-membered heterocyclic ring containing 1 to 3 heteroatoms selected from N, S, and O, and the heterocyclic ring is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;

# means that one point is selected to connect to Y;

n = 0 or 1;

each $X_1$ is independently N or C;

$X_2$ is N or $CR_3$;

$X_3$ is N or $CR_{31}$;

$X_4$ is N or $CR_{32}$;

$X_5$ is N or $CR_{33}$;

$X_6$ is C(O), S(O) or S(O)$_2$;

$X_7$ is $CR_7$ or N;

$R_1$, $R_2$ and $R_4$ are independently H, halogen, OH, -$NHC_{1-4}$ alkyl, -N($C_{1-4}$ alkyl)$_2$, CN, $C_{1-4}$ alkyl, $C_{1-4}$ alkoxy, halo$C_{1-4}$ alkoxy, halo$C_{1-4}$ alkyl, $C_{2-4}$ alkenyl or $C_{2-4}$ alkynyl;

$R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -$NHC_{1-4}$ alkyl, -N($C_{1-4}$ alkyl)$_2$, CN or $C_{3-6}$ cycloalkyl, alternatively, $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, -$NHC_{1-4}$ alkyl, -N($C_{1-4}$ alkyl)$_2$, $C_{1-4}$ alkoxy, halo$C_{1-4}$ alkoxy, halo$C_{1-4}$ alkyl, CN, $C_{1-4}$ alkyl or =O;

$R_6$ is H, halogen, $C_{1-4}$ alkyl or halo$C_{1-4}$ alkyl;

alternatively, $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -$NHC_{1-4}$ alkyl, -N($C_{1-4}$ alkyl)$_2$, CN and $C_{1-4}$ alkyl;

$R_7$, $R_8$, and $R_9$ are independently H, halogen, -$NHC_{1-4}$ alkyl, -N($C_{1-4}$ alkyl)$_2$, CN, $C_{1-4}$ alkyl, $C_{2-4}$ alkenyl, $C_{2-4}$ alkynyl, halo$C_{1-4}$ alkyl, $C_{1-4}$ alkoxy or halo$C_{1-4}$ alkoxy;

Y is $C_{1-2}$ alkylene, O or $NR_y$;

$R_y$ is H or $C_{1-4}$ alkyl;

M is $C_{1-2}$ alkylene, O or $NR_m$;

W is a bond, O or $NR_w$;

$R_m$ and $R_w$ are independently H or $C_{1-4}$ alkyl;

R is selected from $C_{1-4}$ alkyl, $C_{3-8}$ cycloalkyl, 4-membered to 10-membered heterocyclic ring containing 1 to 3 heteroatoms selected from N, S, and O, or -O-$C_{3-6}$ cycloalkyl, and the alkyl, cycloalkyl or heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl)$_2$ and halo$C_{1-4}$ alkyl;

when Cy is P2, R is 4-membered to 10-membered saturated heterocycloalkyl containing 1 to 3 heteroatoms selected from N, S, and O, and is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl)$_2$ and halo$C_{1-4}$ alkyl.

A specific technical solution 2 of the present invention provides a compound represented by formula I, and a stereoisomer, a deuterated product or a pharmaceutically acceptable salt thereof,

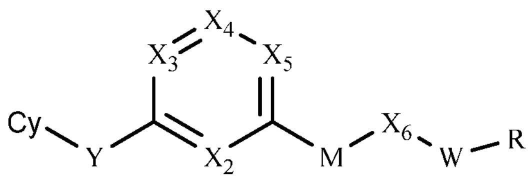

I

wherein, Cy is selected from P1, P2, P3, P4 or P5;

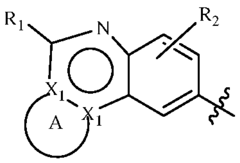
P1 ,
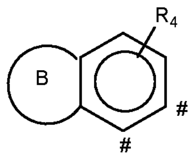
P2 ,
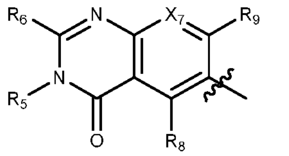
P3 ,
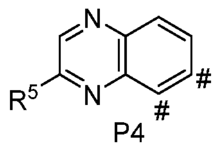
P4 ,

and

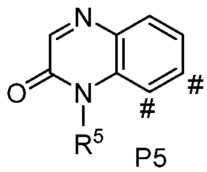
P5 ;

ring A is 5-membered or 6-membered heteroaryl containing 1 to 3 heteroatoms selected from N, S, and O, and the heteroaryl is optionally substituted with 1 or 2 groups selected from halogen, $C_{1-4}$ alkoxy, $-NHC_{1-4}$ alkyl, $-N(C_{1-4}$ alkyl$)_2$, CN, $C_{1-4}$ alkyl, halo$C_{1-4}$ alkoxy and halo$C_{1-4}$ alkyl;
ring B is 5-membered heterocyclic ring containing 1 to 3 heteroatoms selected from N, S, and O, and the heterocyclic ring is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;
# means that one point is selected to connect to Y;
each $X_1$ is independently N or C;
$X_2$ is N or $CR_3$;
$X_3$ is N or $CR_{31}$;
$X_4$ is N or $CR_{32}$;
$X_5$ is N or $CR_{33}$;
$X_6$ is C(O), S(O) or $S(O)_2$;
$X_7$ is $CR_7$ or N;
$R_1$, $R_2$ and $R_4$ are independently H, halogen, OH, $-NHC_{1-4}$ alkyl, $-N(C_{1-4}$ alkyl$)_2$, CN, $C_{1-4}$ alkyl, $C_{1-4}$ alkoxy, halo$C_{1-4}$ alkoxy, halo$C_{1-4}$ alkyl, $C_{2-4}$ alkenyl or $C_{2-4}$ alkynyl;
$R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, $-NHC_{1-4}$ alkyl, $-N(C_{1-4}$ alkyl$)_2$ or CN. Alternatively, $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;
$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, $-NHC_{1-4}$ alkyl, $-N(C_{1-4}$ alkyl$)_2$, $C_{1-4}$ alkoxy, halo$C_{1-4}$ alkoxy, halo$C_{1-4}$ alkyl, CN and $C_{1-4}$ alkyl;
$R_6$ is H, halogen, $C_{1-4}$ alkyl or halo$C_{1-4}$ alkyl;
alternatively, $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, $-NHC_{1-4}$ alkyl, $-N(C_{1-4}$ alkyl$)_2$, CN and $C_{1-4}$ alkyl;
$R_7$, $R_8$, and $R_9$ are independently H, halogen, $-NHC_{1-4}$ alkyl, $-N(C_{1-4}$ alkyl$)_2$, CN, $C_{1-4}$ alkyl, $C_{2-4}$ alkenyl, $C_{2-4}$ alkynyl, halo$C_{1-4}$ alkyl, $C_{1-4}$ alkoxy or halo$C_{1-4}$ alkoxy;
Y is $C_{1-2}$ alkylene, O or $NR_y$;
$R_y$ is H or $C_{1-4}$ alkyl;
M is $C_{1-2}$ alkylene, O or $NR_m$;
W is a bond, O or $NR_w$;
$R_m$ and $R_w$ are independently H or $C_{1-4}$ alkyl;
R is selected from $C_{1-4}$ alkyl, $C_{3-8}$ cycloalkyl or 4-membered to 10-membered heterocyclic ring containing 1 to 3 heteroatoms selected from N, S, and O, and the alkyl, cycloalkyl and heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and

halo$C_{1-4}$ alkyl;

when Cy is P2, R is 4-membered to 10-membered saturated heterocycloalkyl containing 1 to 3 heteroatoms selected from N, S, and O, and is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl.

In certain embodiments, in the compound of formula I, Cy is selected from P1, P2 or P3;

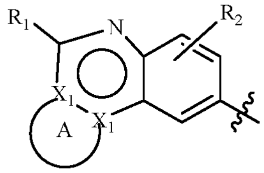
P1 ,
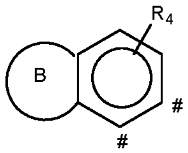
P2 ,
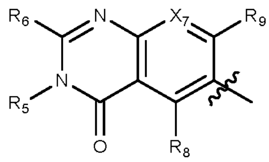
P3 ;

Ring A is 5-membered or 6-membered heteroaryl containing 1 to 3 heteroatoms selected from N, S, and O, and the heteroaryl is optionally substituted with 1 or 2 groups selected from halogen, $C_{1-4}$ alkoxy, -NH$C_{1-4}$ alkyl, -N($C_{1-4}$ alkyl)$_2$, CN, $C_{1-4}$ alkyl, halo$C_{1-4}$ alkoxy and halo$C_{1-4}$ alkyl;

ring B is 5-membered heterocyclic ring containing 1 to 3 heteroatoms selected from N, S, and O, and the heterocyclic ring is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;

# means that one point is selected to connect to Y;

each $X_1$ is independently N or C;

$X_2$ is N or $CR_3$;

$X_3$ is N or $CR_{31}$;

$X_4$ is N or $CR_{32}$;

$X_5$ is N or $CR_{33}$;

$X_6$ is C(O), S(O) or S(O)$_2$;

$X_7$ is $CR_7$ or N;

$R_1$, $R_2$ and $R_4$ are independently H, halogen, OH, -NH$C_{1-4}$ alkyl, -N($C_{1-4}$ alkyl)$_2$, CN, $C_{1-4}$ alkyl, $C_{1-4}$ alkoxy, halo$C_{1-4}$ alkoxy, halo$C_{1-4}$ alkyl, $C_{2-4}$ alkenyl or $C_{2-4}$ alkynyl;

$R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -NH$C_{1-4}$ alkyl, -N($C_{1-4}$ alkyl)$_2$ or CN. Alternatively, $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, -NH$C_{1-4}$ alkyl, -N($C_{1-4}$ alkyl)$_2$, $C_{1-4}$ alkoxy, halo$C_{1-4}$ alkoxy, halo$C_{1-4}$ alkyl, CN and $C_{1-4}$ alkyl;

$R_6$ is H, halogen, $C_{1-4}$ alkyl or halo$C_{1-4}$ alkyl;

alternatively, $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -NH$C_{1-4}$ alkyl, -N($C_{1-4}$ alkyl)$_2$, CN and $C_{1-4}$ alkyl;

$R_7$, $R_8$, and $R_9$ are independently H, halogen, -NH$C_{1-4}$ alkyl, -N($C_{1-4}$ alkyl)$_2$, CN, $C_{1-4}$ alkyl, $C_{2-4}$ alkenyl, $C_{2-4}$ alkynyl, halo$C_{1-4}$ alkyl, $C_{1-4}$ alkoxy or halo$C_{1-4}$ alkoxy;

Y is $C_{1-2}$ alkylene, O or $NR_y$;

$R_y$ is H or $C_{1-4}$ alkyl;

M is $C_{1-2}$ alkylene, O or $NR_m$;

W is a bond, O or $NR_w$;

$R_m$ and $R_w$ are independently H or $C_{1-4}$ alkyl;

R is selected from $C_{1-4}$ alkyl, $C_{3-8}$ cycloalkyl or 4-membered to 10-membered heterocyclic ring containing 1 to 3 heteroatoms selected from N, S, and O, and the alkyl, cycloalkyl and heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl;

when Cy is P2, R is 4-membered to 10-membered saturated heterocycloalkyl containing 1 to 3 heteroatoms selected from N, S, and O, and is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl.

A specific technical solution 3 of the present invention is the compound, and the stereoisomer, the deuterated product or the pharmaceutically acceptable salt thereof described in solution 1 or 2,

wherein, ring A is 5-membered heteroaryl, and the heteroaryl is optionally substituted with 1 or 2 groups selected from halogen, $C_{1-4}$ alkoxy, -NH$C_{1-4}$ alkyl, - N($C_{1-4}$ alkyl)$_2$, CN, $C_{1-4}$ alkyl, halo$C_{1-4}$ alkoxy and halo$C_{1-4}$ alkyl;
when Cy is P3, the compound meets at least one of the following conditions:

(1) $R_5$ is substituted or unsubstituted $C_{3-6}$ cycloalkyl, substituted or unsubstituted 4-membered to 7-membered heterocycloalkyl containing 1-3 heteroatoms selected from N, S, and O, substituted or unsubstituted $C_{3-6}$ cycloalkyloxy, substituted or unsubstituted $C_{1-4}$ alkoxy or substituted or unsubstituted halo$C_{1-4}$ alkoxy, R is $C_{3-8}$ cycloalkyl, 4-membered to 10-membered heterocyclic ring containing 1 to 3 heteroatoms selected from N, S, and O, or -O-$C_{3-6}$ cycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl;
(2) $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -NH$C_{1-4}$ alkyl, -N($C_{1-4}$ alkyl)$_2$, CN and $C_{1-4}$ alkyl;
(3) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN, W is O;
(4) $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, $C_{1-4}$ haloalkoxy and CN;
(5) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN, W is $NR_w$, R is $C_{3-8}$ cycloalkyl or 4-membered to 10-membered heterocyclic ring containing 1 to 3 heteroatoms selected from N, S, and O, and is optionally substituted with 1 to 3 groups selected from halogen, $C_{1-4}$ alkyl, $C_{1-4}$ alkoxy, $C_{1-4}$ haloalkoxy, OH, $NH_2$, - NH$C_{1-4}$ alkyl, -N($C_{1-4}$ alkyl)$_2$ and halo$C_{1-4}$ alkyl;
(6) $X_2$ is N or $CR_3$, $X_6$ is SO or $SO_2$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN, R is $C_{3-4}$ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, OH, $NH_2$, -NH$C_{1-4}$ alkyl, -N($C_{1-4}$ alkyl)$_2$ and halo$C_{1-4}$ alkyl;
(7) $X_6$ is CO, $X_2$ is N or $CR_3$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN;
(8) at least one of $X_2$, $X_3$, $X_4$, and $X_5$ is N, when $X_2$ is $CR_3$, $R_3$ is H, $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN;
(9) $X_2$ is N or $CR_3$, $R_3$ is H, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -NH$C_{1-4}$ alkyl, -N($C_{1-4}$ alkyl)$_2$ or CN, $R_8$ is halogen, - NH$C_{1-4}$ alkyl, -N($C_{1-4}$ alkyl)$_2$, CN, $C_{1-4}$ alkoxy or halo$C_{1-4}$ alkoxy.

A specific technical solution 4 of the present invention is the compound, and the stereoisomer, the deuterated product or the pharmaceutically acceptable salt thereof described in solution 1 or 2, wherein, when Cy is P3, the compound meets one of the following conditions:

(1) $R_5$ is substituted or unsubstituted $C_{3-6}$ cycloalkyl, substituted or unsubstituted 4-membered to 7-membered heterocycloalkyl containing 1-3 heteroatoms selected from N, S, and O, substituted or unsubstituted $C_{1-4}$ alkoxy or substituted or unsubstituted halo$C_{1-4}$ alkoxy, R is $C_{3-8}$ cycloalkyl or 4-membered to 10-membered heterocyclic ring containing 1 to 3 heteroatoms selected from N, S, and O, and is optionally substituted with 1 to 3 groups selected from halogen, $C_{1-4}$ alkyl, $C_{1-4}$ alkoxy, OH, $NH_2$, -NH$C_{1-4}$ alkyl, -N($C_{1-4}$ alkyl)$_2$ and halo$C_{1-4}$ alkyl;
(2) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ or CN, W is O;
(3) $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;
(4) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ or CN, W is $NR_w$, R is $C_{3-8}$ cycloalkyl or 4-membered to 10-membered heterocyclic ring containing 1 to 3 heteroatoms

selected from N, S, and O, and is optionally substituted with 1 to 3 groups selected from halogen, $C_{1-4}$ alkyl, $C_{1-4}$ alkoxy, OH, $NH_2$, $-NHC_{1-4}$ alkyl, $-N(C_{1-4}$ alkyl$)_2$ and halo$C_{1-4}$ alkyl;

(5) $X_2$ is N or $CR_3$, $X_6$ is SO or $SO_2$, W is a bond, $R_3$ is $C_{1-4}$ alkyl, halo$C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ or CN, R is $C_{3-4}$ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, OH, $NH_2$, $-NHC_{1-4}$ alkyl, $-N(C_{1-4}$ alkyl$)_2$ and halo$C_{1-4}$ alkyl;

(6) $X_6$ is CO, $X_2$ is N or $CR_3$, W is a bond, $R_3$ is $C_{1-4}$ alkyl, halo$C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ or CN;

(7) at least one of $X_2$, $X_3$, $X_4$, and $X_5$ is N, when $X_2$ is $CR_3$, $R_3$ is H, $C_{1-4}$ alkyl, halo$C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ or CN;

(8) $X_2$ is N or $CR_3$, $R_3$ is H, $C_{1-4}$ alkyl, halo$C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ or CN, $R_8$ is halogen, OH, $NH_2$, $- NHC_{1-4}$ alkyl, $-N(C_{1-4}$ alkyl$)_2$, CN, $C_{1-4}$ alkoxy or halo$C_{1-4}$ alkoxy.

A specific technical solution 5 of the present invention is the compound, and the stereoisomer, the deuterated product or the pharmaceutically acceptable salt thereof described in solution 3,

wherein, $R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, $-NHC_{1-4}$ alkyl, $-N(C_{1-4}$ alkyl$)_2$, CN or $C_{3-6}$ cycloalkyl, alternatively, $R_{31}$ and $R_{32}$, or $R_{32}$ and $R_{33}$ together with the atoms to which they are attached form $C_{3-6}$ cycloalkyl or 5-membered or 6-membered heterocycloalkyl containing 1 to 3 heteroatoms selected from N, S, and O, and the cycloalkyl or the heterocycloalkyl is optionally substituted with 1 to 3 groups selected from halogen, $C_{1-4}$ alkyl, halo$C_{1-4}$ alkyl, $C_{1-4}$ alkoxy, OH, $NH_2$ and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, $-NHC_{1-4}$ alkyl, $-N(C_{1-4}$ alkyl$)_2$, $C_{1-4}$ alkoxy, halo$C_{1-4}$ alkoxy, halo$C_{1-4}$ alkyl, CN and $C_{1-4}$ alkyl or =O;

$R_6$ is H, halogen, $C_{1-4}$ alkyl or halo$C_{1-4}$ alkyl;

alternatively, $R_5$ and $R_6$ together with the atoms to which they are attached form 5-membered or 6-membered heterocycloalkyl containing 1 to 3 heteroatoms selected from N, S, and O, and the heterocycloalkyl is optionally substituted with 1 to 3 groups selected from halogen, OH, $NH_2$, $-NHC_{1-4}$ alkyl, $-N(C_{1-4}$ alkyl$)_2$, CN and $C_{1-4}$ alkyl;

R is selected from $C_{1-4}$ alkyl, $C_{3-8}$ cycloalkyl or 4-membered to 10-membered heterocycloalkyl containing 1 to 3 heteroatoms selected from N, S, and O, or $-O-C_{3-6}$ cycloalkyl, and the alkyl, the cycloalkyl and the heterocycloalkyl is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl.

A specific technical solution 6 of the present invention is the compound, and the stereoisomer, the deuterated product or the pharmaceutically acceptable salt thereof described in solution 5, the compound has the structure of the following formula I-1,

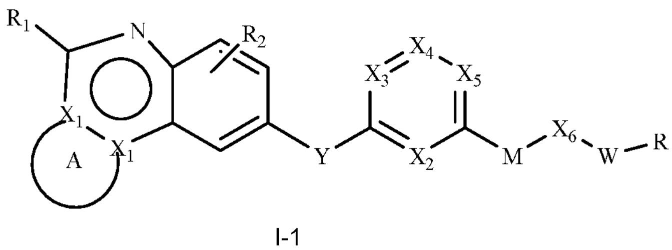

I-1

wherein, R is selected from $C_{1-4}$ alkyl, $C_{3-8}$ cycloalkyl or 4-membered to 7-membered monocyclic heterocycloalkyl containing 1 to 3 heteroatoms selected from N, S, and O, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, $-NHC_{1-4}$ alkyl, $-N(C_{1-4}$ alkyl$)_2$ and halo$C_{1-4}$ alkyl.

A specific technical solution 7 of the present invention is the compound, and the stereoisomer, the deuterated product or the pharmaceutically acceptable salt thereof described in solution 5, the compound has the structure of the

following formula 1-2,

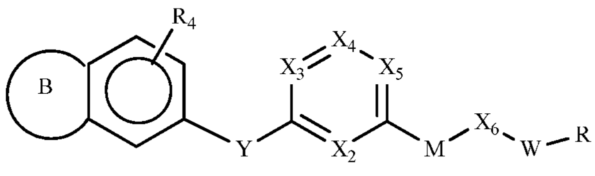

I-2

wherein, ring B is 5-membered heteroaryl or 5-membered heterocycloalkyl containing 1 to 3 heteroatoms selected from N, S, and O, and is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;

R is saturated 4-membered to 7-membered monocyclic heterocycloalkyl containing 1 to 3 heteroatoms selected from N, S, and O, saturated 5-membered to 10-membered fused heterocycloalkyl, saturated 5-membered to 10-membered bridged heterocycloalkyl or saturated 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl)$_2$ and halo$C_{1-4}$ alkyl.

A specific technical solution 8 of the present invention is the compound, and the stereoisomer, the deuterated product or the pharmaceutically acceptable salt thereof described in solution 5, the compound has the structure of the following formula I-3,

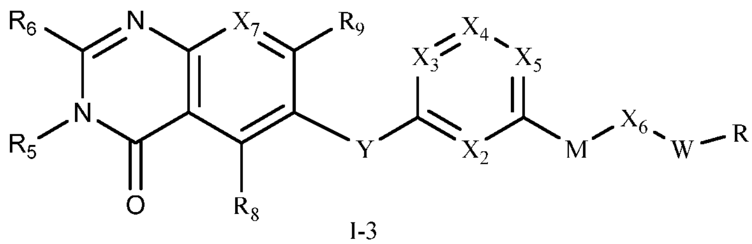

I-3

.

A specific technical solution 9 of the present invention is the compound, and the stereoisomer, the deuterated product or the pharmaceutically acceptable salt thereof described in solution 1 or 2, the compound has the structure of the following formula 1-4,

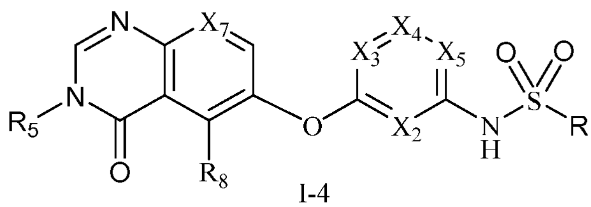

I-4

$X_2$ is N or $CR_3$;

$X_6$ is SO or $SO_2$;

$R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN;

R is 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and the 4-membered monocyclic heterocycloalkyl is substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl)$_2$ and halo$C_{1-4}$ alkyl, the 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl is optionally substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl)$_2$ and halo$C_{1-4}$ alkyl.

A specific technical solution 10 of the present invention is the compound, and the stereoisomer, the deuterated product or the pharmaceutically acceptable salt thereof described in solution 8, wherein

$R_6$ and $R_9$ are H; Y is O; M is NH;
$X_2$ is N or $CR_3$, $X_6$ is SO or $SO_2$, and W is a bond;
$R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl$)_2$ or CN;
R is 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl or - O-$C_{3-6}$ cycloalkyl, and the 4-membered monocyclic heterocycloalkyl is substituted with 1 to 3 groups selected from halogen, $C_{1-4}$ alkyl, $C_{1-4}$ alkoxy, OH, $NH_2$, -NH$C_{1-4}$ alkyl, -N($C_{1-4}$ alkyl$)_2$ and halo$C_{1-4}$ alkyl, the 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl is optionally substituted with 1 to 3 groups selected from halogen, $C_{1-4}$ alkyl, $C_{1-4}$ alkoxy, OH, $NH_2$, -NH$C_{1-4}$ alkyl, -N($C_{1-4}$ alkyl$)_2$ and halo$C_{1-4}$ alkyl.

A specific technical solution 11 of the present invention is the compound, and the stereoisomer, the deuterated product or the pharmaceutically acceptable salt thereof described in solution 9, the compound has the structure of the following formula I-5,

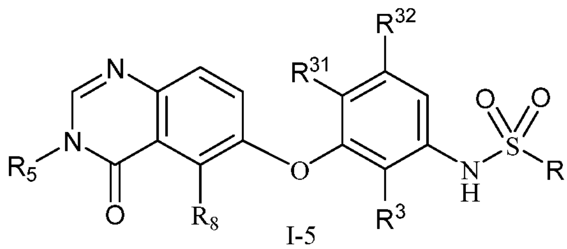

I-5

$R_3$ is $C_{1-2}$ alkyl, halo$C_{1-2}$ alkyl, $C_{1-2}$ alkoxy, halo$C_{1-2}$ alkoxy, $C_{2-4}$ alkenyl, $C_{2-4}$ alkynyl, -NH$C_{1-2}$ alkyl, -N($C_{1-2}$ alkyl$)_2$ or CN;
each of $R_{31}$ and $R_{32}$ is independently H, F, Cl, $C_{1-2}$ alkyl, halo$C_{1-2}$ alkyl, $C_{2-4}$ alkenyl, $C_{2-4}$ alkynyl, $C_{1-2}$ alkoxy, halo$C_{1-2}$ alkoxy, -NH$C_{1-2}$ alkyl, -N($C_{1-2}$ alkyl$)_2$ or CN;
$R_5$ is $C_{1-2}$ alkyl, halo$C_{1-2}$ alkyl, $C_{3-4}$ cycloalkyl, 4-membered to 7-membered heterocycloalkyl containing 1-3 heteroatoms selected from N, S, and O, $C_{1-2}$ alkoxy, halo$C_{1-2}$ alkoxy or $C_{3-4}$ cycloalkyloxy;
$R_8$ is selected from H, F, Cl, CN, $C_{1-2}$ alkyl, $C_{2-4}$ alkenyl, $C_{2-4}$ alkynyl, halo$C_{1-2}$ alkyl, $C_{1-2}$ alkoxy or halo$C_{1-2}$ alkoxy;
R is 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and the 4-membered monocyclic heterocycloalkyl is substituted with 1 to 3 groups selected from F, Cl, $C_{1-2}$ alkyl, $C_{1-2}$ alkoxy, OH, $NH_2$, -NH$C_{1-2}$ alkyl, -N($C_{1-2}$ alkyl$)_2$ and halo$C_{1-2}$ alkyl, the 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl is optionally substituted with 1 to 3 groups selected from F, Cl, $C_{1-2}$ alkyl, $C_{1-2}$ alkoxy, OH, $NH_2$, -NH$C_{1-2}$ alkyl, -N($C_{1-2}$ alkyl$)_2$ and halo$C_{1-2}$ alkyl.

A specific technical solution 12 of the present invention is the compound, and the stereoisomer, the deuterated product or the pharmaceutically acceptable salt thereof described in solution 8, solution 9 or solution 10, wherein

$X_2$ is $CR_3$; $X_3$ is $CR_{31}$; $X_4$ is $CR_{32}$; $X_5$ is CH; $X_6$ is $SO_2$; $X_7$ is CH;
$R_3$ is $C_{1-2}$ alkyl, halo$C_{1-2}$ alkyl, $C_{1-2}$ alkoxy, halo$C_{1-2}$ alkoxy, $C_{2-4}$ alkenyl, $C_{2-4}$ alkynyl, -NH$C_{1-2}$ alkyl, -N($C_{1-2}$ alkyl$)_2$ or CN;
each of $R_{31}$ and $R_{32}$ is independently H, F, Cl, $C_{1-2}$ alkyl, halo$C_{1-2}$ alkyl, $C_{2-4}$ alkenyl, $C_{2-4}$ alkynyl, $C_{1-2}$ alkoxy, halo$C_{1-2}$ alkoxy, -NH$C_{1-2}$ alkyl, -N($C_{1-2}$ alkyl$)_2$ or CN or $C_{3-6}$ cycloalkyl;
$R_5$ is $C_{12}$ alkyl, CN or =O substituted $C_{1-2}$ alkyl, halo$C_{1-2}$ alkyl, $C_{3-4}$ cycloalkyl, 4-membered to 7-membered heterocycloalkyl containing 1-3 heteroatoms selected from N, S, and O, $C_{1-2}$ alkoxy, halo$C_{1-2}$ alkoxy or $C_{3-4}$ cycloalkyloxy;

$R_8$ is selected from H, F, Cl, CN, $C_{1-2}$ alkyl, $C_{2-4}$ alkenyl, $C_{2-4}$ alkynyl, halo$C_{1-2}$ alkyl, $C_{1-2}$ alkoxy or halo$C_{1-2}$ alkoxy;
R is 4-membered or 5-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl or - O-$C_{3-6}$ cycloalkyl, and the 4-membered monocyclic heterocycloalkyl is substituted with 1 to 3 groups selected from F, Cl, $C_{1-2}$ alkyl, $C_{1-2}$ alkoxy, OH, $NH_2$, -$NHC_{1-2}$ alkyl, -N($C_{1-2}$ alkyl)$_2$ and halo$C_{1-2}$ alkyl, the 6-membered to 8-membered monocyclic heterocycloalkyl, the 5-membered or 6-membered monocyclic heteroaryl, the 5-membered to 10-membered fused heterocycloalkyl, the 5-membered to 10-membered bridged heterocycloalkyl or the 6-membered to 10-membered spirocyclic heterocycloalkyl or the $C_{3-6}$ cycloalkyl is optionally substituted with 1 to 3 groups selected from F, Cl, $C_{1-2}$ alkyl, $C_{1-2}$ alkoxy, OH, $NH_2$, -$NHC_{1-2}$ alkyl, -N($C_{1-2}$ alkyl)$_2$ and halo$C_{1-2}$ alkyl.

A specific technical solution 13 of the present invention is the compound, and the stereoisomer, the deuterated product or the pharmaceutically acceptable salt thereof described in solution 12, wherein

$R_3$ is CN;
each of $R_{31}$ and $R_{32}$ is independently H, F or Cl or $C_{3-6}$ cycloalkyl;
$R_5$ is selected from $C_{1-2}$ alkyl, CN or =O substituted $C_{1-2}$ alkyl, halo$C_{1-2}$ alkyl or $C_{1-2}$ alkoxy;
$R_8$ is H;
R is 6-membered to 10-membered spirocyclic heterocycloalkyl containing 1 to 3 heteroatoms selected from N, S, and O or 4-membered or 5-membered monocyclic heterocycloalkyl containing 1 to 3 heteroatoms selected from N, S, and O, or -O-$C_{3-6}$ cycloalkyl, and the 6-membered to 10-membered spirocyclic heterocycloalkyl, the 4-membered or 5-membered monocyclic heterocycloalkyl or the $C_{3-6}$ cycloalkyl is optionally substituted with 1 to 3 groups selected from F, Cl, $C_{1-2}$ alkyl or $C_{1-2}$ alkoxy.

A specific technical solution 14 of the present invention is the compound, and the stereoisomer, the deuterated product or the pharmaceutically acceptable salt thereof described in solution 8, solution 9 or solution 10, wherein

$X_2$ is $CR_3$; $X_3$ is $CR_{31}$; $X_4$ is $CR_{32}$; $X_5$ is CH; $X_6$ is $SO_2$; $X_7$ is CH;
$R_3$ is $C_{1-2}$ alkyl, halo$C_{1-2}$ alkyl, $C_{1-2}$ alkoxy, halo$C_{1-2}$ alkoxy, $C_{2-4}$ alkenyl, $C_{2-4}$ alkynyl, -$NHC_{1-2}$ alkyl, -N($C_{1-2}$ alkyl)$_2$ or CN;
each of $R_{31}$ and $R_{32}$ is independently H, F, Cl, $C_{1-2}$ alkyl, halo$C_{1-2}$ alkyl, $C_{2-4}$ alkenyl, $C_{2-4}$ alkynyl, $C_{1-2}$ alkoxy, halo$C_{1-2}$ alkoxy, -$NHC_{1-2}$ alkyl, -N($C_{1-2}$ alkyl)$_2$ or CN;
$R_5$ is $C_{1-2}$ alkyl, halo$C_{1-2}$ alkyl, $C_{3-4}$ cycloalkyl, 4-membered to 7-membered heterocycloalkyl containing 1-3 heteroatoms selected from N, S, and O, $C_{1-2}$ alkoxy, halo$C_{1-2}$ alkoxy or $C_{3-4}$ cycloalkyloxy;
$R_8$ is selected from H, F, Cl, CN, $C_{1-2}$ alkyl, $C_{2-4}$ alkenyl, $C_{2-4}$ alkynyl, halo$C_{1-2}$ alkyl, $C_{1-2}$ alkoxy or halo$C_{1-2}$ alkoxy;
R is 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and the 4-membered monocyclic heterocycloalkyl is substituted with 1 to 3 groups selected from F, Cl, $C_{1-2}$ alkyl, $C_{1-2}$ alkoxy, OH, $NH_2$, -$NHC_{1-2}$ alkyl, -N($C_{1-2}$ alkyl)$_2$ and halo$C_{1-2}$ alkyl, the 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl is optionally substituted with 1 to 3 groups selected from F, Cl, $C_{1-2}$ alkyl, $C_{1-2}$ alkoxy, OH, $NH_2$, -$NHC_{1-2}$ alkyl, -N($C_{1-2}$ alkyl)$_2$ and halo$C_{1-2}$ alkyl.

A specific technical solution 15 of the present invention is the compound, and the stereoisomer, the deuterated product or the pharmaceutically acceptable salt thereof described in the present invention, the compound has the structure of the following formula I-6,

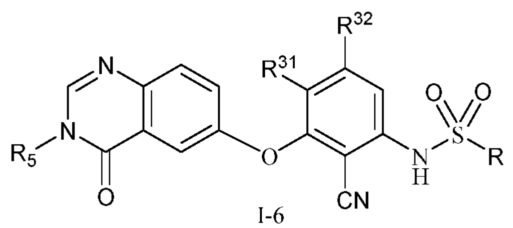

I-6

The compound, or the stereoisomer, the deuterated product or the pharmaceutically acceptable salt thereof of the present invention, wherein,
R is selected from 5-membered to 10-membered fused heterocycloalkyl, 5-membered to 10-membered bridged heterocycloalkyl or 6-membered to 10-membered spirocyclic heterocycloalkyl, and the 5-membered to 10-membered fused heterocycloalkyl, 5-membered to 10-membered bridged heterocycloalkyl or 6-membered to 10-membered spirocyclic heterocycloalkyl is optionally substituted with 1 to 3 groups selected from F, Cl, $C_{1-2}$ alkyl, $C_{1-2}$ alkoxy, OH, $NH_2$, $-NHC_{1-2}$ alkyl, $-N(C_{1-2}\ alkyl)_2$ and halo$C_{1-2}$ alkyl.
A specific technical solution 16 of the present invention is the compound, and the stereoisomer, the deuterated product or the pharmaceutically acceptable salt thereof described in solution 11, solution 12 or solution 14 in the present invention, wherein,

$R_3$ is CN;
each of $R_{31}$ and $R_{32}$ is independently H, F or Cl;
$R_5$ is selected from $C_{1-2}$ alkyl or $C_{1-2}$ alkoxy or is selected from halo$C_{1-2}$ alkyl;
$R_8$ is H;
R is 6-membered to 10-membered spirocyclic heterocycloalkyl containing 1 to 3 heteroatoms selected from N, S, and O or is 4-membered or 5-membered monocyclic heterocycloalkyl containing 1 to 3 heteroatoms selected from N, S, and O, and the 6-membered to 10-membered spirocyclic heterocycloalkyl is optionally substituted with 1 to 3 groups selected from F, Cl, $C_{1-2}$ alkyl or $C_{1-2}$ alkoxy, or the 4-membered or 5-membered monocyclic heterocycloalkyl is optionally substituted with 1 to 3 groups selected from F, Cl, $C_{1-2}$ alkyl or $C_{1-2}$ alkoxy.

The compound, or the stereoisomer, the deuterated product or the pharmaceutically acceptable salt thereof of the present invention, wherein,
R is selected from

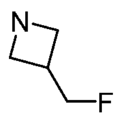 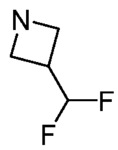 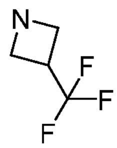

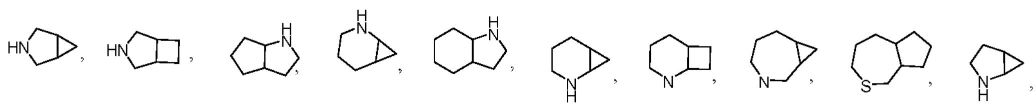 , , , , , , , , , ,

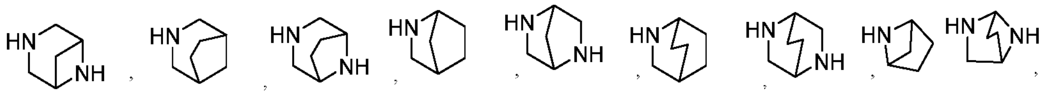 , , , , , , , , ,

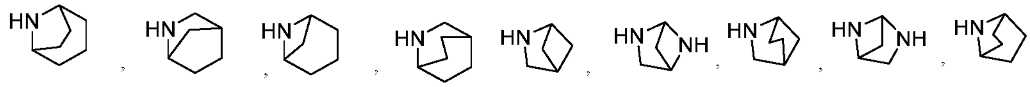 , , , , , , ,

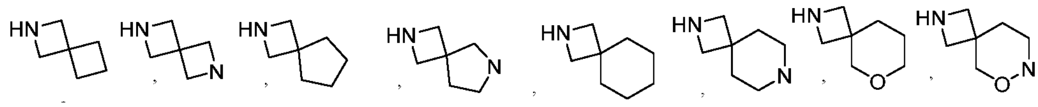 , , , , , , ,

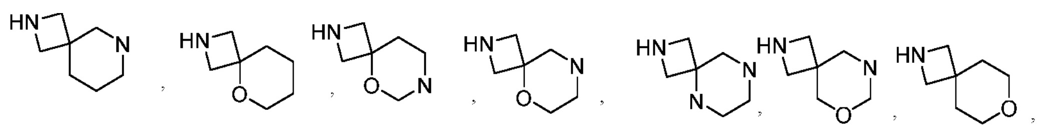 , , , , , , ,

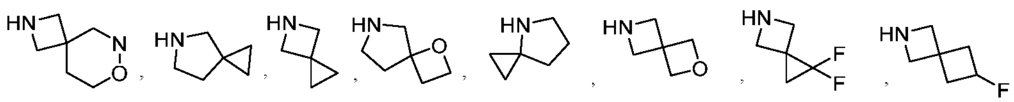 , , , , , , ,

or

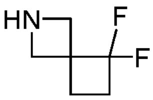, 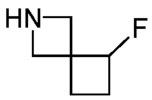 or 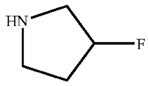, 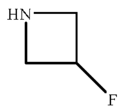, 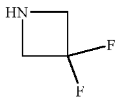, 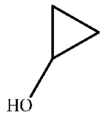, 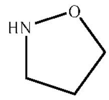,

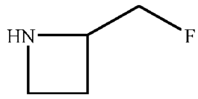.

Further, the compound, or the stereoisomer, the deuterated product or the pharmaceutically acceptable salt thereof of the present invention, wherein,
R is selected from

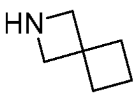, 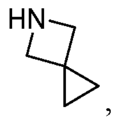, 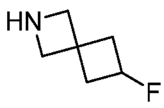, 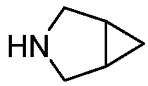, 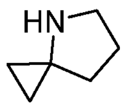,

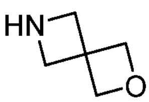, 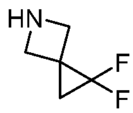, 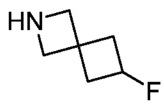, 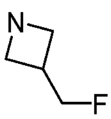, 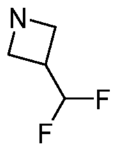, 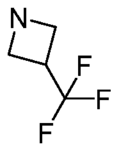, 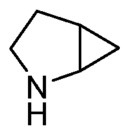,

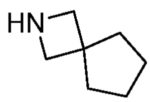 or 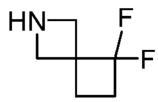, 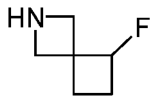.

A specific technical solution of the present invention is the compound, and the stereoisomer, the deuterated product or the pharmaceutically acceptable salt thereof described in the present invention, the compound is selected from one of the structures in Table 1,

Table 1:

| | | | |
|---|---|---|---|
| 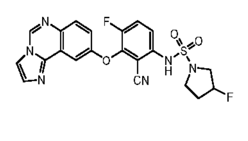 | 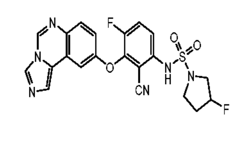 | 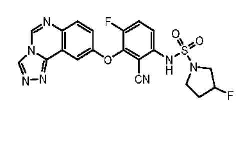 | 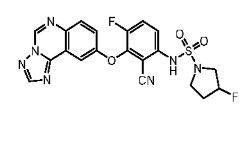 |
| 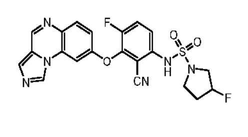 | 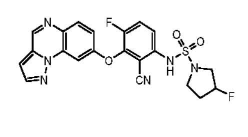 | 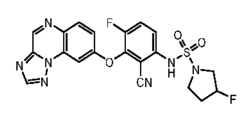 | 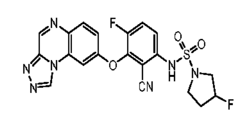 |
| 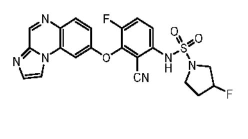 | 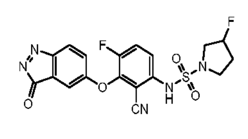 | 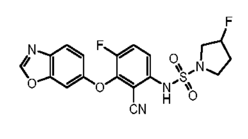 | 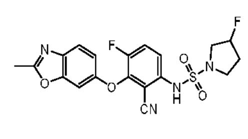 |

| | | | |
|---|---|---|---|
| 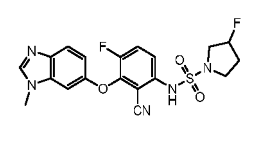 | 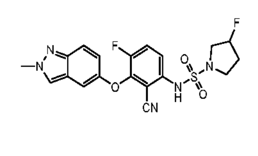 | 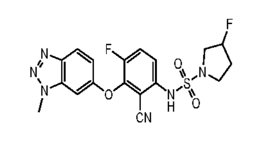 | 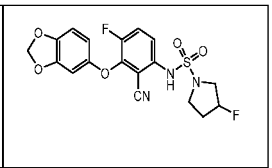 |
| 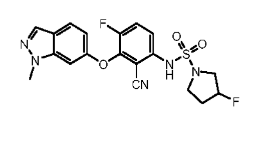 | 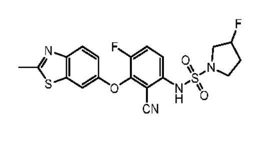 | 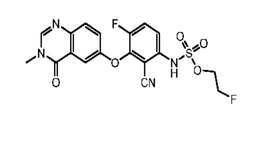 | 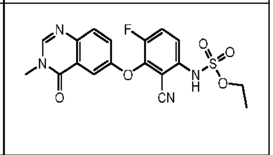 |
| 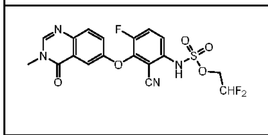 | 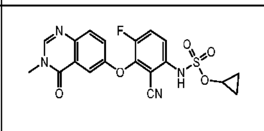 | 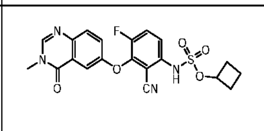 | 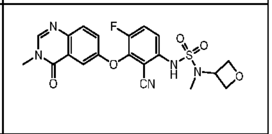 |
| 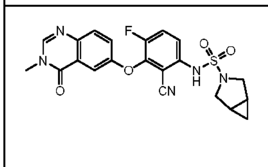 | 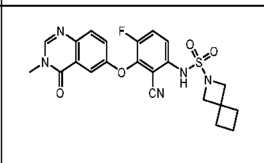 | 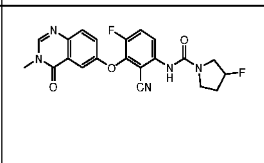 | 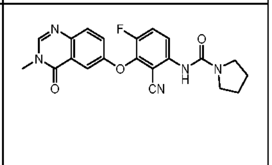 |
| 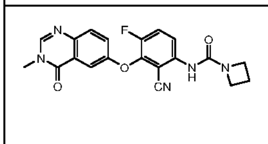 | 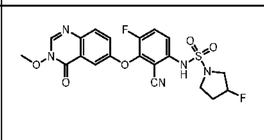 | 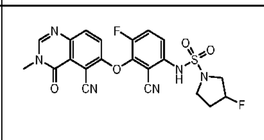 | 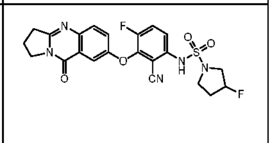 |
| 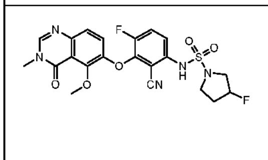 | 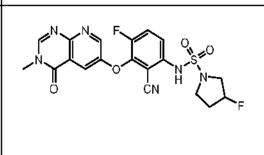 | 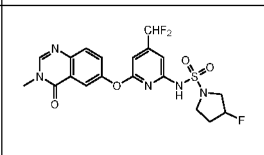 | 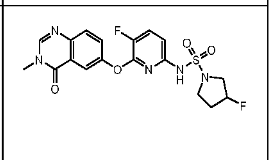 |
| 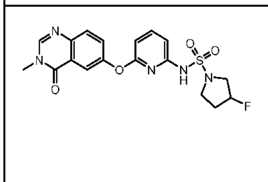 | 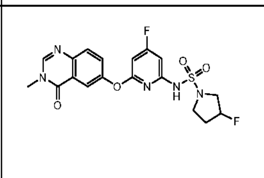 | 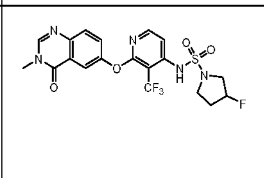 | 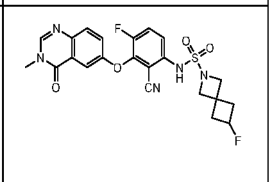 |
| 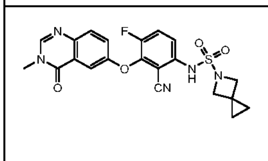 | 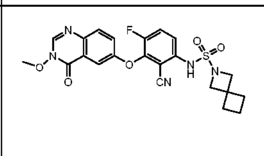 | 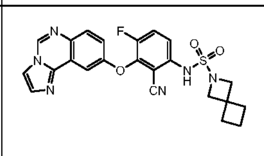 | 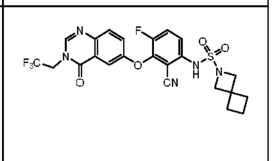 |
| 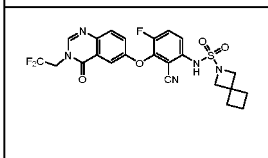 | 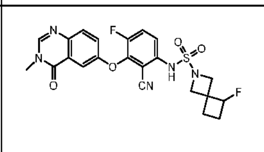 | 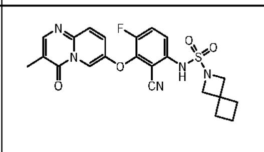 | 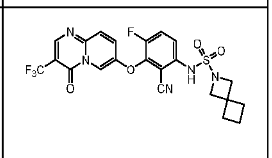 |
| 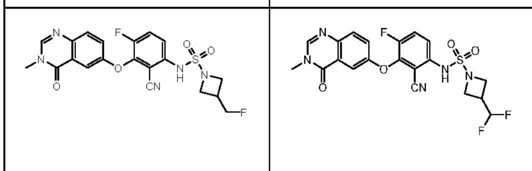 | | 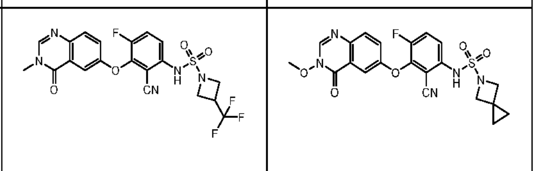 | |
| 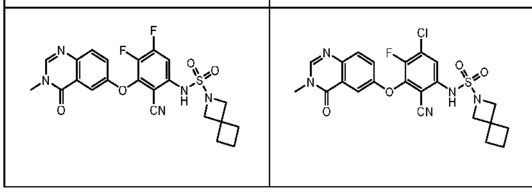 | | 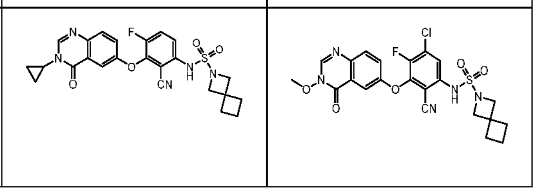 | |

| | | | |
|---|---|---|---|
| 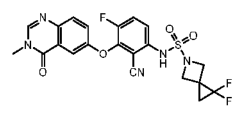 | 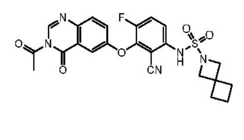 | 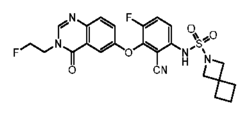 | 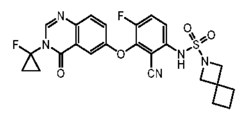 |
| 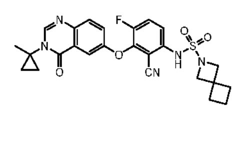 | 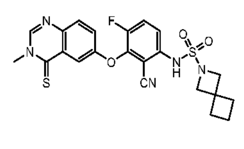 | 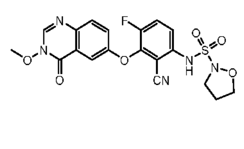 | 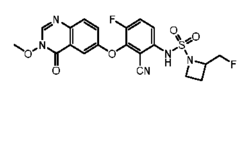 |
| 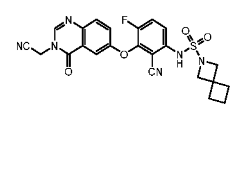 | 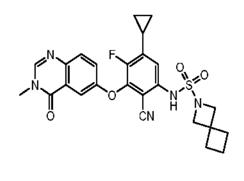 | 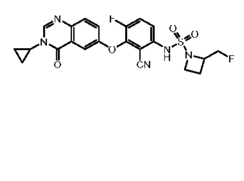 | 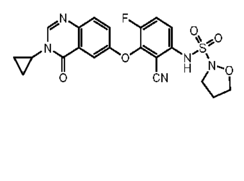 |
| 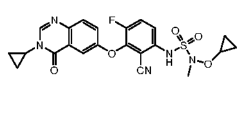 | 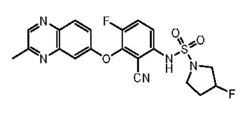 | 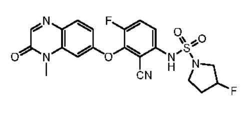 | 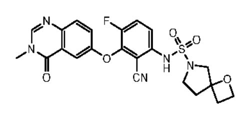 |
| 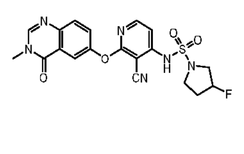 | 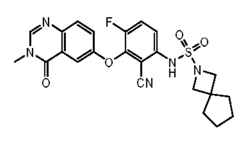 | 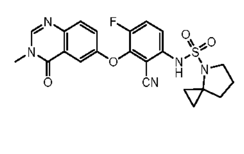 | 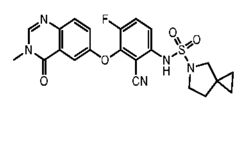 |
| 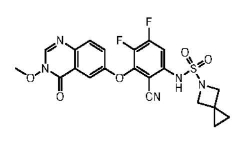 | 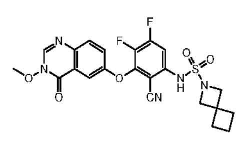 | 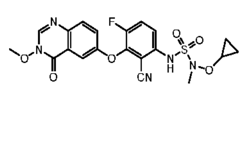 | 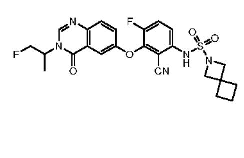 |
| 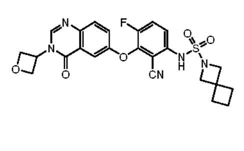 | 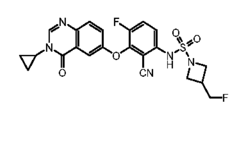 | 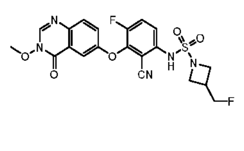 | 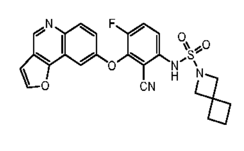 |
| 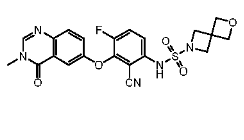 | 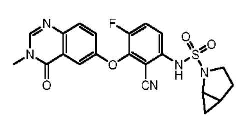 | 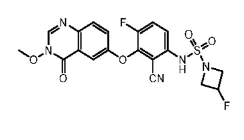 | 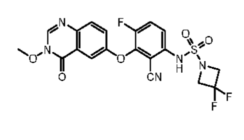 |
| 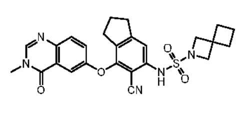 | 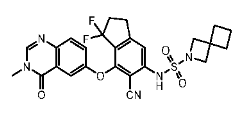 | 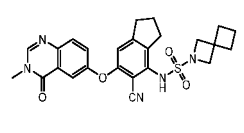 | 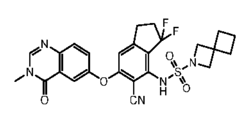 |
| 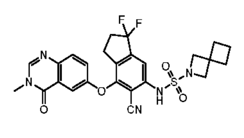 | 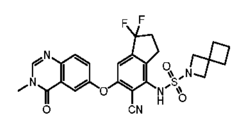 | 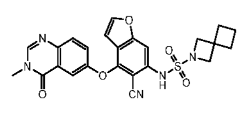 | 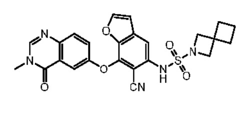 |
| | | | |

| | | | |
|---|---|---|---|
| 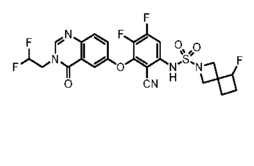 | 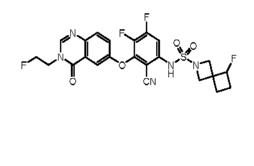 | 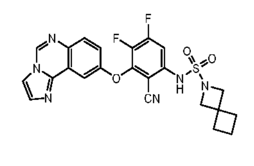 | 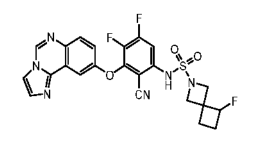 |
| 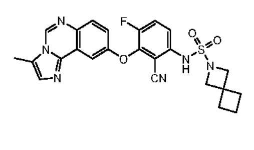 | 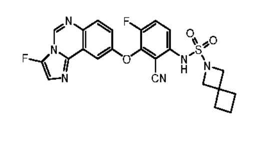 | 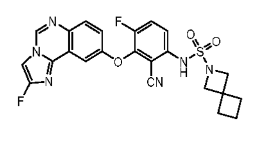 | 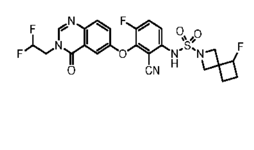 |
| 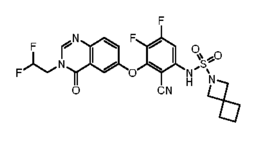 | 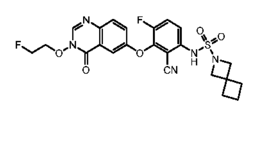 | 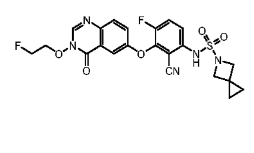 | 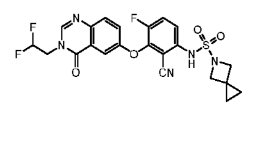 |
| 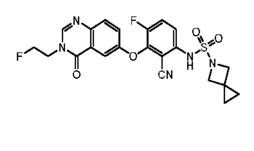 | 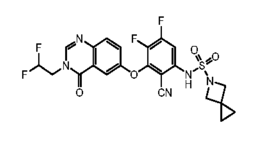 | 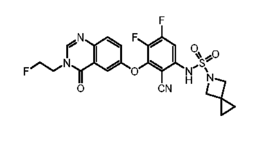 | 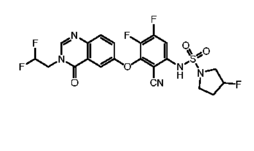 |
| 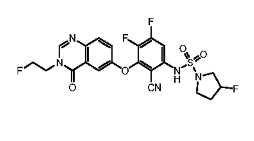 | 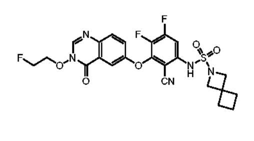 | 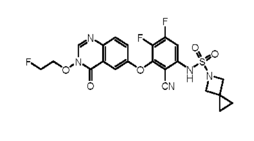 | 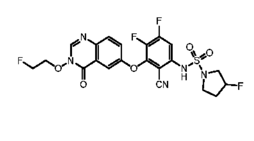 |

.

A specific technical solution of the present invention is the compound, and the stereoisomer, the deuterated product or the pharmaceutically acceptable salt thereof described in the present invention, the compound is selected from one of the structures in Table 2,

Table 2:

| | | | |
|---|---|---|---|
| 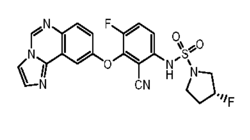 | 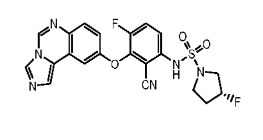 | 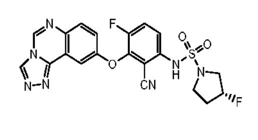 | 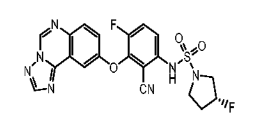 |
| 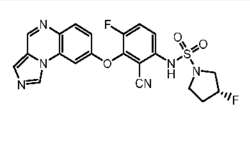 | 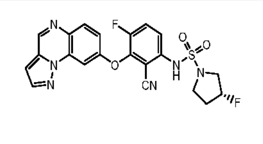 | 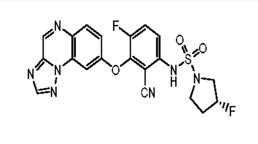 | 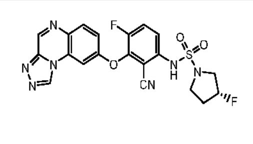 |
| 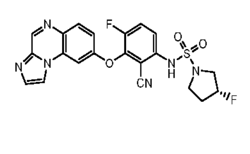 | 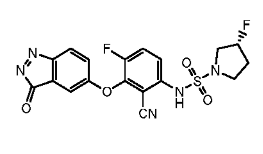 | 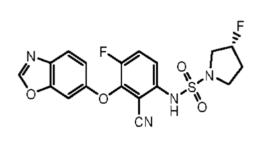 | 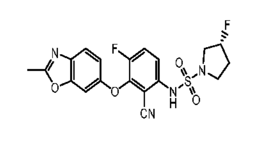 |
| 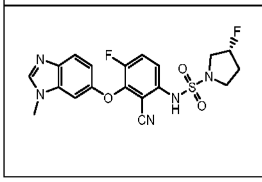 | 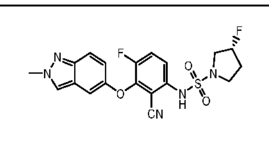 | 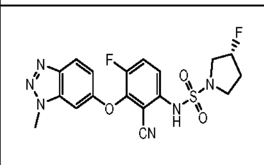 | 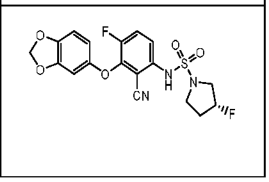 |

| | | | |
|---|---|---|---|
| 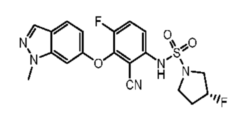 | 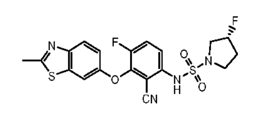 | 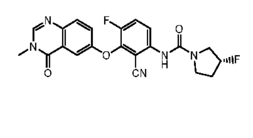 | 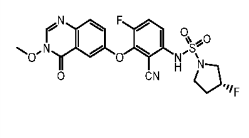 |
| 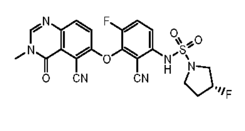 | 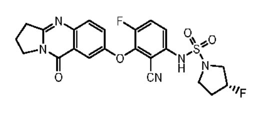 | 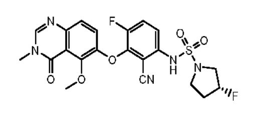 | 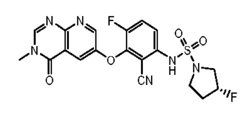 |
| 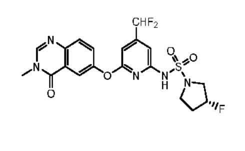 | 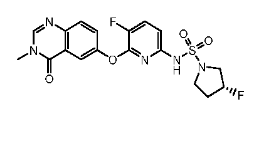 | 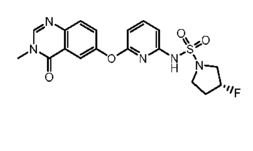 | 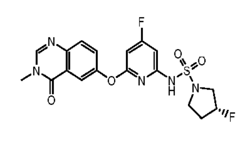 |
| 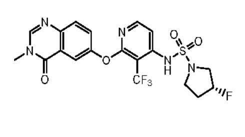 | 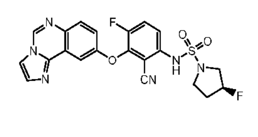 | 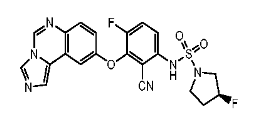 | 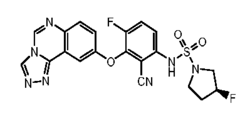 |
| 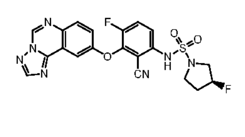 | 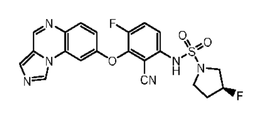 | 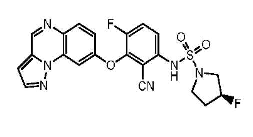 | 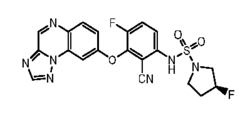 |
| 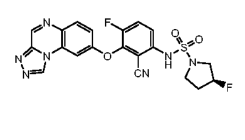 | 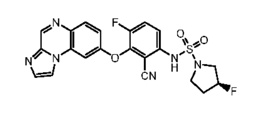 | 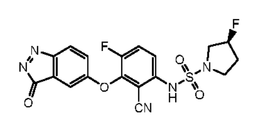 | 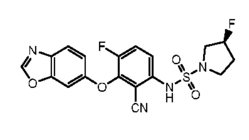 |
| 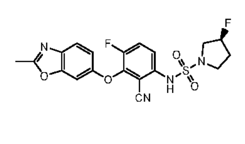 | 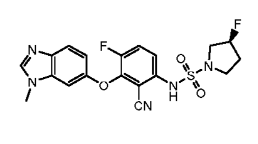 | 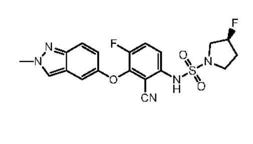 | 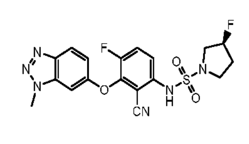 |
| 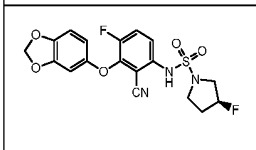 | 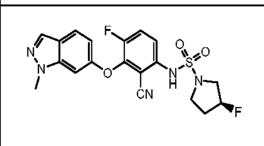 | 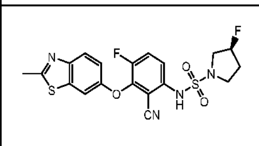 | 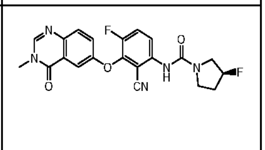 |
| 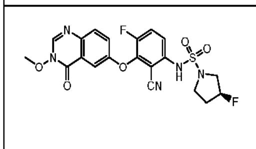 | 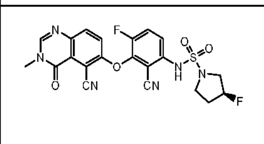 | 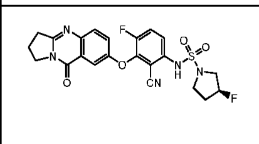 | 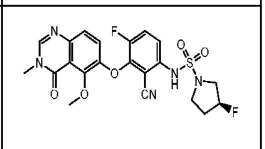 |
| 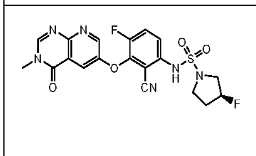 | 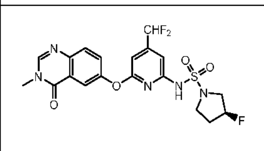 | 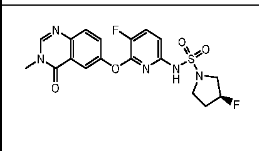 | 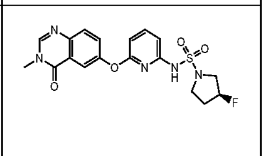 |
| | | | |
| | | | |

| | | | |
|---|---|---|---|
| 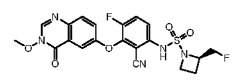 | 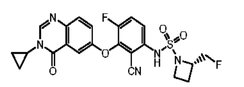 | 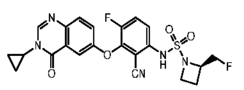 | 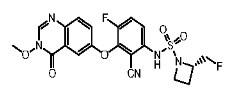 |
| 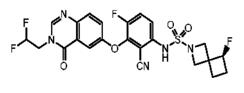 | 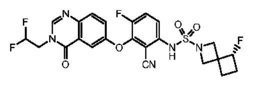 | 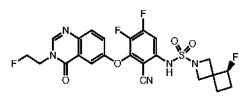 | 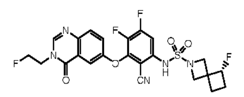 |
| 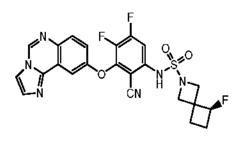 | 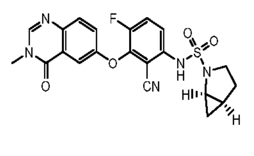 | 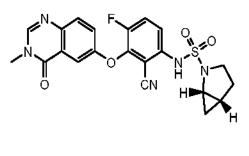 | 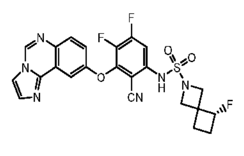 |
| 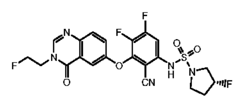 | 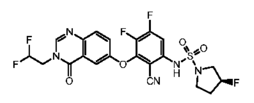 | 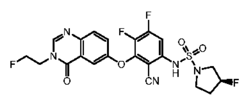 | 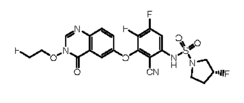 |
| 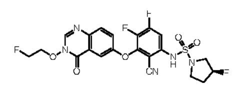 | 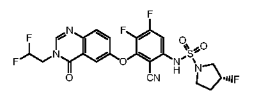 | | |

The present invention also provides a composition or pharmaceutical preparation, which comprises the compound, and the stereoisomer, the deuterated product or the pharmaceutically acceptable salt thereof described in any one of the above solutions, and a pharmaceutically acceptable carrier and/or excipient. The pharmaceutical composition can be in a unit preparation form (the unit preparation is also referred to as "preparation specification").

Further, the composition or pharmaceutical preparation of the present invention comprises 1 mg-1500 mg of the compound, and the stereoisomer, the deuterated product or the pharmaceutically acceptable salt thereof described in any one of the above solutions, and a pharmaceutically acceptable carrier and/or excipient.

The present invention further provides the use of the compound, and the stereoisomer, the deuterated product or the pharmaceutically acceptable salt thereof described in any one of the above solutions in the preparation of a drug for the treatment/prevention of a BRAF-mediated disease.

Further, the BRAF-mediated disease is a tumor, and a further preferred tumor is a brain tumor.

The present invention also provides a method for treating diseases in a mammals, which method comprises administering to a subject a therapeutically effective amount of the compound, and the stereoisomer, the deuterated product or the pharmaceutically acceptable salt thereof described in any one of the above solutions.

The disease is preferably a tumor.

Preferably, the therapeutically effective amount is 1 mg-1500 mg.

In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated.

In some embodiments, the outcome is the reduction and/or remission of signs, symptoms or causes of the disease, or any other desired change in the biological system. For example, an "effective amount" in terms of the therapeutic use is an amount comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700

mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;

In some embodiments, the pharmaceutical composition or preparation of the present invention comprises the above therapeutically effective amount of the compound, and the stereoisomer, the deuterated product or the pharmaceutically acceptable salt thereof of the present invention.

The present invention relates to a pharmaceutical composition or pharmaceutical preparation, which comprises a therapeutically effective amount of the compound, and the stereoisomer, the deuterated product or the pharmaceutically acceptable salt thereof of the present invention and a carrier and/or excipient.

The pharmaceutical composition can be in a unit preparation form (the amount of the active drug in the unit preparation is also referred to as the "preparation specification"). In some embodiments, the pharmaceutical composition comprises, but is not limited to, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, 1500 mg of the compound, and the stereoisomer, the deuterated product or the pharmaceutically acceptable salt thereof of the present invention.

A method for treating a disease in a mammal, the method comprises administering to a subject a therapeutically effective amount of the compound, and the stereoisomer, the deuterated product or the pharmaceutically acceptable salt thereof of the present invention, and a pharmaceutically acceptable carrier and/or excipient.

The therapeutically effective dose is preferably 1 mg-1500 mg.

The disease is preferably a tumor, especially a brain tumor.

A method for treating a disease in a mammal, the method comprises administering the compound, and the stereoisomer, the deuterated product or the pharmaceutically acceptable salt thereof of the present invention, and the pharmaceutically acceptable carrier and/or excipient to a subject at a daily dose of 1 mg/day-1500 mg/day, the daily dose may be a single dose or divided doses.

In some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400mg/day. In some embodiments, the daily dose includes, but is not limited to, 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500mg/day.

The present invention relates to a kit, which can comprise a composition in the form of a single dose or multiple doses and comprises the compound, and the stereoisomer, the deuterated product or the pharmaceutically acceptable salt thereof of the present invention. The amount of the compound, and the stereoisomer, the deuterated product or the pharmaceutically acceptable salt thereof of the present invention is the same as that described in the above-mentioned pharmaceutical composition.

In the present invention, the amount of the compound or the stereoisomer or pharmaceutically acceptable salt thereof according to the present invention is calculated in the form of a free base in each case.

The term "preparation specification" refers to the weight of the active drug contained in each vial, tablet or other unit preparation.

**Synthetic route**

The preparation method of BRAF modulator is introduced in patent documents such as WO 2021116050 A1, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalog are optionally prepared by custom chemical synthesis plants, wherein many of standard chemical supply plants (such as those listed above) provide custom synthesis services.

**Term**

Unless otherwise specified, the terms of the present invention have the following meanings.

The carbon, hydrogen, oxygen, sulfur, nitrogen and halogen involved in the groups and compounds of the present invention all include isotopes thereof, and are optionally further replaced by one or more of the corresponding isotopes thereof, wherein the isotopes of carbon include $^{12}C$, $^{13}C$ and $^{14}C$; the isotopes of hydrogen include protium (H), deuterium (D, also known as heavy hydrogen) and tritium (T, also known as superheavy hydrogen); the isotopes of oxygen include $^{16}O$, $^{17}O$ and $^{18}O$; the isotopes of sulfur include $^{32}S$, $^{33}S$, $^{34}S$ and $^{36}S$; the isotopes of nitrogen include $^{14}N$ and $^{15}N$; the isotope of fluorine includes $^{19}F$; the isotopes of chlorine include $^{35}Cl$ and $^{37}Cl$; and the isotopes of bromine include $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to $-CF_3$, $- CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may

further be optionally substituted with a substituent.

The term "alkenylene" refers to a straight or branched divalent unsaturated hydrocarbon group comprising at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched divalent unsaturated hydrocarbon group comprising a carbon-carbon triple bond (C≡C) and generally comprises 2-6 carbon atoms, and further comprises 2-4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

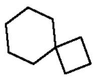, 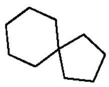, 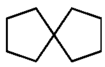, 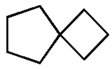, 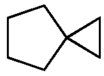, 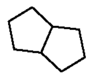,

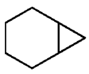, 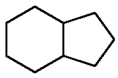, 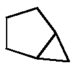, 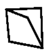, 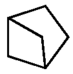, 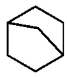, 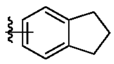,

etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 13 carbon atoms, and further contains 6 to 9 carbon atoms, and it is further phenyl. Non-limiting examples of aryl include phenyl, naphthyl, anthryl and phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes

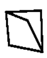, 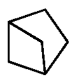, 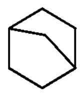,

*etc.,* a bicyclic fused ring includes

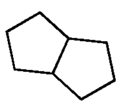, 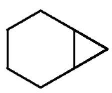, 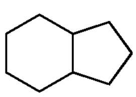, 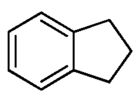, 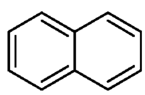, 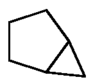,

*etc.,* and a bicyclic spiro ring includes

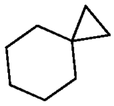, 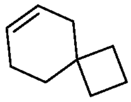, 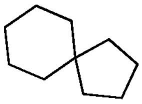, 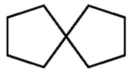, 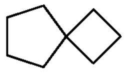,

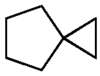,

*etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl,

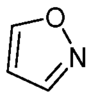, 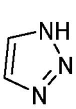, 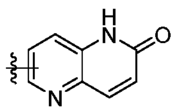, 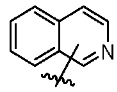,

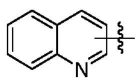, 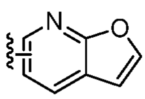, 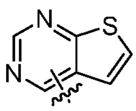, 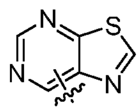, 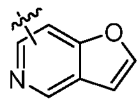,

etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, aziridinyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

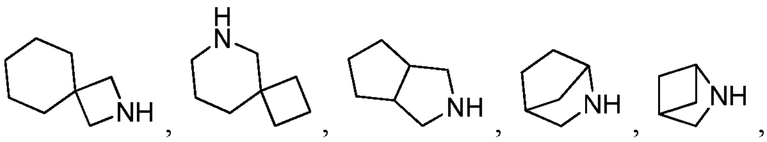

, , , , ,

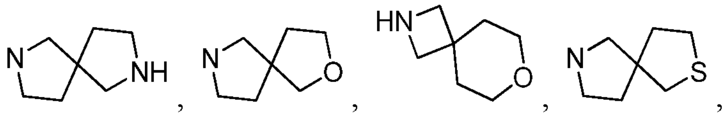

, , , ,

etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

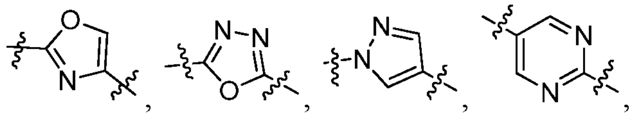

, , , ,

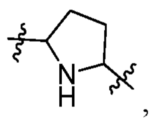

,

*etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include:

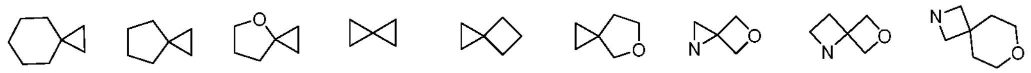

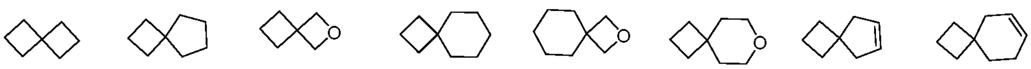

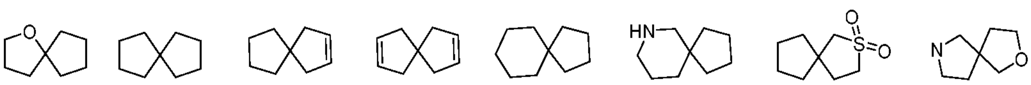

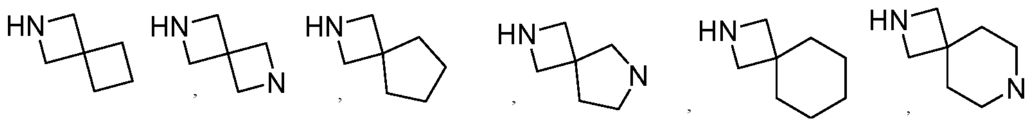

, , , , ,

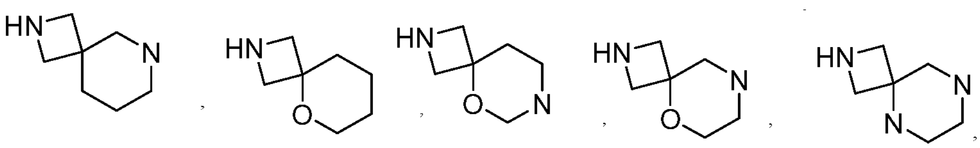

, , , , ,

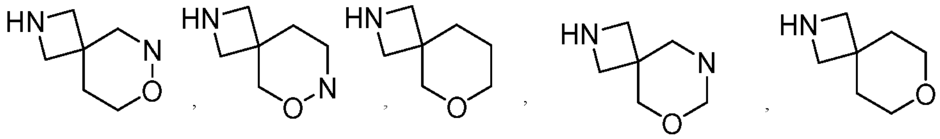,

and the spiro ring may be optionally substituted with a substituent.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of the fused ring include purine, quinoline, isoquinoline, benzopyran, benzofuran, benzothiophene, and

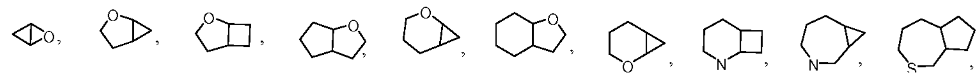

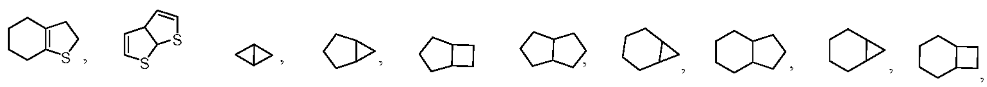

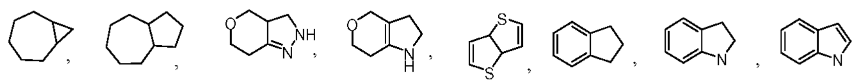;

the fused ring may be optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

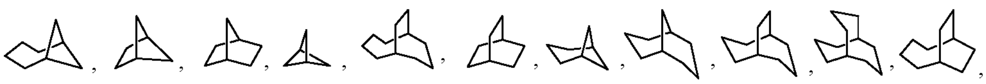

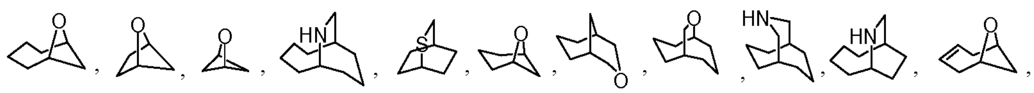

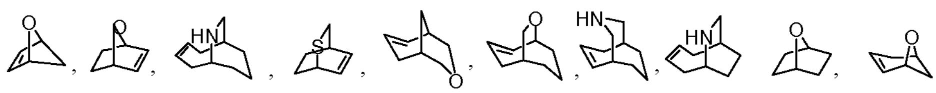

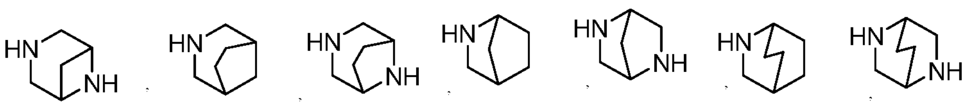

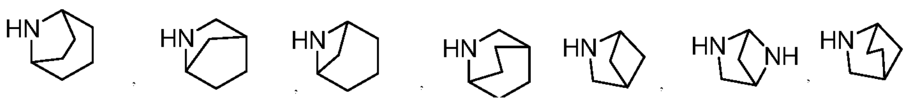

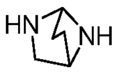 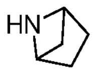 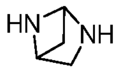 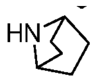

Unless otherwise specified, the term "substitution" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}$ alkyl), -OC(=O)($C_{1-6}$ alkyl), $-OCO_2(C_{1-6}$ alkyl), - $C(=O)NH_2$, -C(=O)N($C_{1-6}$ alkyl$)_2$, -OC(=O)NH($C_{1-6}$ alkyl), -NHC(=O)($C_{1-6}$ alkyl), -N($C_{1-6}$ alkyl)C(=O)($C_{1-6}$ alkyl), $-NHCO_2(C_{1-6}$ alkyl), -NHC(=O)N($C_{1-6}$ alkyl$)_2$, - HC(=O)NH($C_{1-6}$ alkyl), $-NHC(=O)NH_2$, $-NHSO_2(C_{1-6}$ alkyl), $-SO_2N(C_{1-6}$ alkyl$)_2$, - $SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or cocrystals thereof as described herein and other components including physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrating agent, an absorbent, a preservative, a surfactant, a colorant, a flavoring agent, and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerin, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

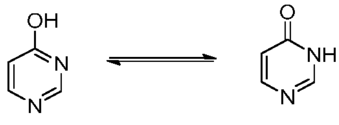

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and

co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

Detailed Description of Embodiments

The technical solutions of the present invention will be described in detail below in conjunction with examples, but the protection scope of the present invention includes but is not limited thereto.

**Detection method**

The structure of the compound is determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) NMR instrument, and the solvent for determination is deuterated dimethyl sulfoxide (DMSO-d6), deuterated chloroform ($CDCl_3$), deuterated methanol ($CD_3OD$), and the internal standard is tetramethylsilane (TMS);

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

**Example 1**

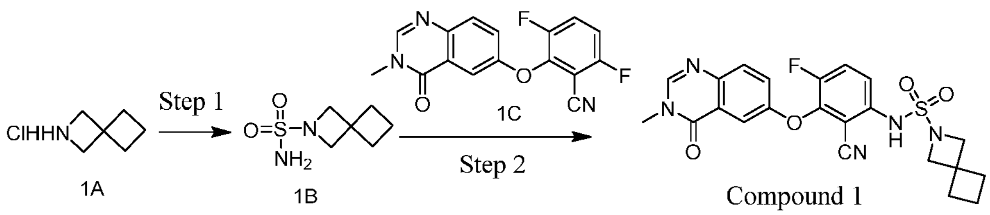


1A 1B 1C Compound 1

Step 1: 1A (800 mg, 5.99 mmol), triethylamine (1.82 g, 17.97 mmol) and dichloromethane (10 mL) were added in sequence into a 50 mL reaction flask. After the addition, the obtained mixture was stirred at 0°C for 20 min, a solution of sulfamoyl chloride (692 mg, 5.99 mmol) in dichloromethane (7 mL) was added dropwise slowly and the obtained mixture was reacted under stirring at room temperature for 1 h. The reaction liquid was diluted with dichloromethane (50 mL), washed with water (30 mL × 1) and saturated brine (30 mL × 1) in sequence, the organic layer was dried over anhydrous sodium sulfate and filtered, and the filtrate was concentrated under reduced pressure, and the residue was separated and purified by column chromatography (DCM/MeOH = 15/1) to give 1B (180 mg, yield 17%).

Step 2: 1C (250 mg, 0.8 mmol, the preparation method refers to WO 2021116050 A1), 1B (160 mg, 0.9 mmol), cesium carbonate (310 mg, 0.96 mmol) and N,N-dimethyl formamide (10 mL) were added in sequence into a 50 mL reaction flask. After the addition, the obtained mixture was reacted under stirring at 80°C for 18 h. Ethyl acetate (50 mL) was added to the reaction liquid, and then washing was performed with water (40 mL × 2). The organic layer was dried with anhydrous sodium sulfate and filtered, and the filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 1 (86 mg, yield 23%).

LCMS m/z = 470.5 $[M+H]^+$;
$^1H$ NMR (400 MHz, DMSO-$d_6$) δ 10.36 (s, 1H), 8.36 (s, 1H), 7.90 - 7.84 (m, 1H), 7.78 (d, 1H), 7.68 (dd, 1H), 7.53 (dd, 1H), 7.39 (d, 1H), 3.84 (s, 4H), 3.48 (s, 3H), 2.10 (t, 4H), 1.78 - 1.69 (m, 2H).

**Example 2**

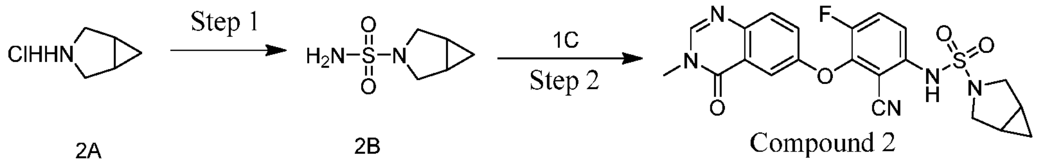


2A 2B Compound 2

Step 1: 2A (520 mg, 4.34 mmol), triethylamine (2.19 g, 21.64 mmol) and dichloromethane (10 mL) were added in sequence into a 50 mL reaction flask. After the addition, the obtained mixture was stirred at 0°C for 20 min, a solution of sulfamoyl chloride (500 mg, 4.34 mmol) in dichloromethane (7 mL) was added dropwise slowly and the obtained mixture was reacted under stirring at room temperature for 1 h. The reaction liquid was diluted with dichloromethane (50 mL), washed with water (50 mL × 1) and saturated brine (50 mL × 1) in sequence, the organic layer was dried over anhydrous sodium sulfate and filtered, and the filtrate was concentrated under reduced pressure, and the residue was separated and purified by column chromatography (DCM/MeOH = 15/1) to give 2B (450 mg, yield 32%).

Step 2: 1C (250 mg, 0.8 mmol), 2B (250 mg, 1.54 mmol), cesium carbonate (520 mg, 1.60 mmol) and N,N-dimethyl formamide (10 mL) were added in sequence into a 50 mL reaction flask. After the addition, the obtained mixture was reacted under stirring at 100°C for 16 h. Ethyl acetate (50 mL) was added to the reaction liquid, and then washing was performed with water (40 mL × 2). The organic layer was dried with anhydrous sodium sulfate and filtered, and the filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 2 (60 mg, yield 16%).

LCMS m/z = 456.2 $[M+H]^+$;
$^1$H NMR (400 MHz, DMSO-$d_6$) δ 10.26 (s, 1H), 8.35 (s, 1H), 7.89-7.84 (m, 1H), 7.78 (d, 1H), 7.67 (dd, 1H), 7.50 (dd, 1H), 7.39 (d, 1H), 3.47 (s, 3H), 3.37-3.34 (m, 4H), 1.59-1.56 (m, 2H), 0.65-0.59 (m, 1H), 0.19-0.16 (m, 1H).

**Example 3**

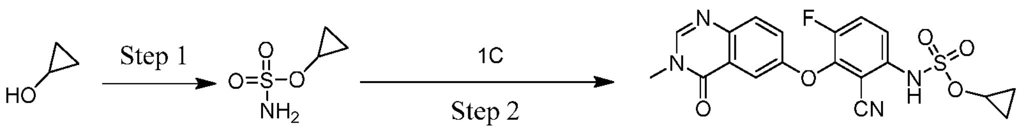


3A 3B

Compound 3

Step 1: Sulfamoyl chloride (1.0g, 8.68 mmol), triethylamine (2.63 g, 26.04) and dichloromethane (10 mL) were added in sequence into a 50 mL reaction flask. After the addition, the obtained mixture was stirred at 0°C for 20 min. Cyclopropanol (1.11g, 17.36 mmol) was slowly added dropwise. The obtained mixture was reacted under stirring at room temperature for 1 h. The reaction liquid was concentrated under reduced pressure to obtain 3B, which was used directly in the next step without purification.

Step 2: 1C (500 mg, 1.6 mmol), 3B (1.0 g crude product), cesium carbonate (620 mg, 1.92 mmol) and N,N-dimethylformamide (15 mL) were added in sequence into a 50 mL reaction flask. After the addition, the obtained mixture was reacted under stirring at 100°C for 18 h. Ethyl acetate (50 mL) was added to the reaction liquid. Then washing was performed with water (40 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 18 min) to obtain compound 3 (100 mg, yield 14.5%).

LCMS m/z = 431.1 $[M+H]^+$;

$^1H$ NMR (400 MHz, $CD_3OD$) δ 8.26 (s, 1H), 7.77 (d, 1H), 7.67 - 7.54 (m, 3H), 7.48 (dd, 1H), 4.24 - 4.13 (m, 1H), 3.58 (s, 3H), 0.94 - 0.83 (m, 2H), 0.79 - 0.68 (m, 2H).

**Example 4**

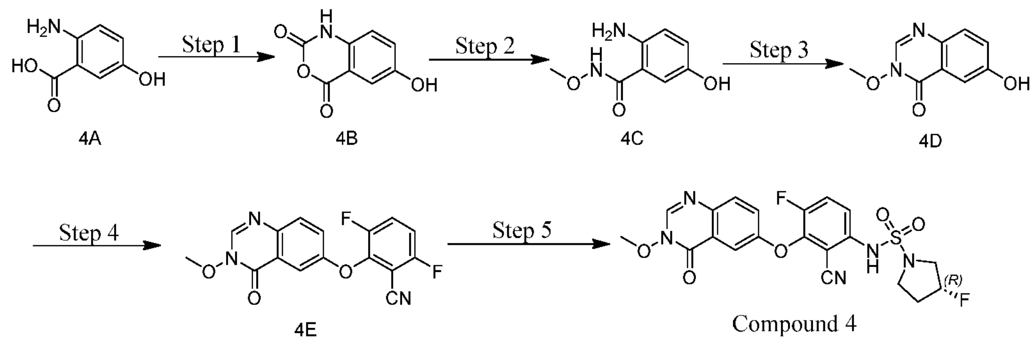


4A 4B 4C 4D 4E Compound 4

Step 1: 4A (4.0 g, 26.08 mmol) was dissolved in dry tetrahydrofuran (80 mL) in a 250 mL three-necked flask under nitrogen protection. Triphosgene (2.94 g, 9.91 mmol) was slowly added under an ice bath. After the addition, the obtained mixture was reacted under stirring at 70°C for 4 h. After the reaction was completed, the reaction liquid was concentrated under reduced pressure. The residue was purified by slurrying with petroleum ether. The solid was filtered and dried to obtain 4B (3.86 g, yield 83%).

$^1H$ NMR (400 MHz, DMSO-$d_6$) δ 11.49 (s, 1H), 9.84 (s, 1H), 7.24 - 7.18 (m, 2H), 7.03 (dd, 1H).

Step 2: 4B (3.85 g, 21.51 mmol) was dissolved in an aqueous solution of sodium hydroxide (1.03 g, 25.75 mmol) (60 mL) in a 250 mL single-neck flask. Methoxyamine hydrochloride (2.69 g, 32.27 mmol) was added. The obtained mixture was reacted under stirring at room temperature overnight. After the reaction was completed, the reaction liquid was concentrated under reduced pressure. The residue was dissolved in a mixed solvent of dichloromethane (200 mL) and methanol (10 mL). The obtained mixture was dried over anhydrous sodium sulfate, and filtered through diatomaceous earth pad. The filtrate was concentrated under reduced pressure. The residue was separated and purified by column chromatography (DCM/MeOH = 20/1) to obtain 4C (2.8 g, yield 71%).

LCMS m/z = 183.1 $[M+H]^+$;

Step 3: 4C (2.6 g,14.27 mmol) was dissolved in trimethyl orthoformate (54 mL) in a 50 mL single-neck flask. After the addition, the obtained mixture was reacted under stirring at 105°C for 4 h. After the reaction was completed, the reaction liquid was concentrated under reduced pressure. The residue was purified by slurrying with petroleum ether. The solid was filtered and dried to obtain 4D (2.4 g, yield 88%).

LCMS m/z = 193.1 $[M+H]^+$;

Step 4: 4D (2.4 g, 12.49 mmol) was dissolved in dry N,N-dimethylformamide (40 mL) in a 100 mL single-neck flask. Cesium carbonate (4.88 g, 14.99 mmol) was slowly added under an ice bath. After the addition, the obtained mixture was reacted under stirring at room temperature for 0.5 h. A solution of 2,3,6-trifluorobenzonitrile (2.32 g, 14.99 mmol) in N,N-dimethylformamide (10 mL) was slowly added dropwise under an ice bath. After the addition, the obtained mixture was reacted under stirring at room temperature for 1 h. After the reaction was completed, the reaction liquid was poured into ice water (250 mL). The obtained mixture was stirred for 30 min, and filtered. The obtained solid was dried and purified by slurrying with a mixed solvent of petroleum ether (100 mL) and ethyl acetate (10 mL). The solid was filtered and dried to obtain 4E (3.6 g, yield 88%).

LCMS m/z = 330.1 $[M+H]^+$;

$^1H$ NMR (400 MHz, DMSO-$d_6$) δ 8.64 (s, 1H), 7.98 (m, 1H), 7.83 (d, 1H), 7.75 (dd, 1H), 7.64 - 7.53 (m, 2H), 4.06 (s, 3H).

$^{19}F$ NMR (377 MHz, DMSO-$d_6$) δ -107.70 (s), -128.37 (s).

Step 5: 4E (0.33 g, 1.00 mmol) was dissolved in dry N,N-dimethylformamide (4 mL) in a 100 mL single-neck flask. Cesium carbonate (0.39 g, 1.20 mmol) was slowly added under an ice bath. After the addition, the obtained mixture was reacted under stirring at 50°C for 0.5 h. A solution of (3R)-3-fluoropyrrolidine-1-sulfonamide (0.20 g, 1.19 mmol) in N,N-dimethylformamide (1 mL) was slowly added dropwise under an ice bath. After the addition, the obtained mixture was reacted under stirring at 100°C for 6 h. After the reaction was completed, the reaction liquid was poured into ice water (50 mL). The obtained mixture was stirred for 30 min, and filtered. The obtained solid was is dried and then separated and purified by preparative liquid chromatography (instrument: waters 2767 preparative liquid chromatographic instrument;

chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 4 (32 mg, yield 7%).

LCMS m/z = 478.1 $[M+H]^+$;
$^1H$ NMR (400 MHz, DMSO-$d_6$) δ 10.33 (s, 1H), 8.63 (s, 1H), 7.91 - 7.81 (m, 2H), 7.71 (dd, 1H), 7.56 (d, 1H), 7.41 (d, 1H), 5.40 (m, 0.5H), 5.27 (m, 0.5H), 4.05 (s, 3H), 3.58 - 3.32 (m, 4H), 2.17 (m, 2H).

$^{19}F$ NMR (377 MHz, DMSO-$d_6$) δ -127.22 (s), -172.76 (s).

**Example 5**

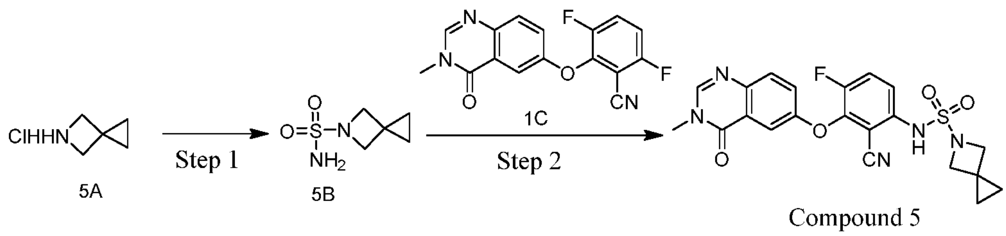


5A 5B 1C

Compound 5

Step 1: 5A (720 mg, 6.04 mmol), triethylamine (1.83 g, 18.12 mmol) and dichloromethane (10 mL) were added in sequence into a 50 mL reaction flask. After the addition, the obtained mixture was stirred at 0°C for 20 min. A solution of sulfamoyl chloride (700 mg, 6.04 mmol) in dichloromethane (7 mL) was slowly added dropwise. The obtained mixture was reacted under stirring at room temperature for 1-2 h. The reaction liquid was diluted with dichloromethane (100 mL), and washed with water (40 mL × 1) and saturated saline (40 mL × 1) in sequence. The organic layer was dried over anhydrous sodium sulfate and filtered. The filtrate was concentrated under reduced pressure. The residue was separated and purified by column chromatography (DCM/MeOH = 12/1) to obtain 5B (600 mg, yield 61%).

Step 2: 1C (0.72 g, 2.30 mmol), 5B (0.41 g, 2.53 mmol), cesium carbonate (0.90 g, 2.76 mmol) and N,N-dimethylformamide (20 mL) were added in sequence into a 100 mL reaction flask. After the addition, the obtained mixture was reacted under stirring at 80°C for 18 h. Ethyl acetate (100 mL) was added to the reaction liquid. Then washing was performed with water (40 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 5 (12 mg, yield 1%).

LCMS m/z = 456.30 $[M+H]^+$;
$^1H$ NMR (400 MHz, DMSO-$d_6$) δ 8.33 (s, 1H), 7.76 (d, 1H), 7.63 (dd, 2H), 7.48 (dd, 1H), 7.38 (d, 1H), 7.08 (s, 1H), 3.83 (s, 4H), 3.47 (s, 3H), 0.56 (s, 4H).

$^{19}F$ NMR (377 MHz, DMSO-$d_6$) δ -134.36 (s).

**Example 6**

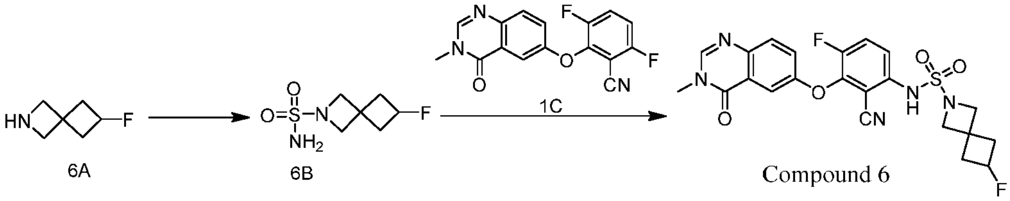

6A 6B 1C

Compound 6

Step 1: 6A (synthesis method was referred to WO 2017/1660, 2017, A1) (620 mg, 5.38 mmol), triethylamine

(1633.21 mg, 16.14 mmol) and dichloromethane (20 mL) were added in sequence into a 50 mL reaction flask, After the addition, the obtained mixture was stirred at 0°C for 20 min. A solution of sulfamoyl chloride (622 mg, 5.38 mmol) in dichloromethane (10 mL) was slowly added dropwise. The obtained mixture was reacted under stirring at room temperature for 1-2 h. The reaction liquid was diluted with dichloromethane (100 mL), and washed with water (40 mL × 1) and saturated saline (40 mL × 1) in sequence. The organic layer was dried over anhydrous sodium sulfate and filtered. The filtrate was concentrated under reduced pressure. The residue was separated and purified by column chromatography (DCM/MeOH = 20/1) to obtain 6B (500 mg, yield 48%).

Step 2: 1C (600 mg, 1.92 mmol), 6B (410 mg, 2.11 mmol), cesium carbonate (751 mg, 2.30 mmol) and N,N-dimethylformamide (20 mL) were added in sequence into a 100 mL reaction flask. After the addition, the obtained mixture was reacted under stirring at 80°C for 18 h. Ethyl acetate (100 mL) was added to the reaction liquid. Then washing was performed with water (40 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-80% acetonitrile; cycle time: 20 min) to obtain compound 6 (8 mg, yield 1%).

LCMS m/z = 488.20 $[M+H]^+$;
$^1$H NMR (400 MHz, DMSO-$d_6$) δ 10.42 (s, 1H), 8.34 (s, 1H), 7.77 (d, 2H), 7.66 (dd, 1H), 7.47 (dd, 1H), 7.38 (d, 1H), 5.01 (s, 0.5H), 4.87 (s, 0.5H), 3.81 (d, 4H), 3.47 (s, 3H), 2.56 (m, 2H), 2.37 - 2.18 (m, 2H).

**Example 7**

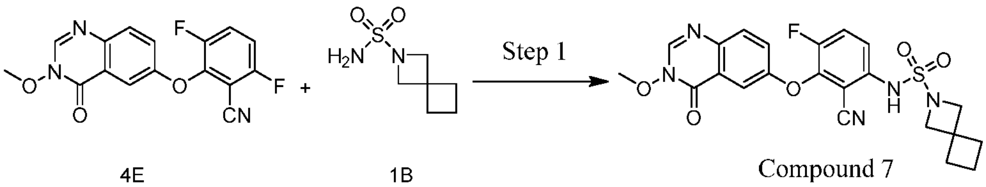


4E 1B Compound 7

Step 1: 1B (281 mg, 1.60 mmol) was dissolved in dry N,N-dimethylformamide (10 mL) in a 100 mL single-neck flask. Cesium carbonate (594 mg, 1.82 mmol) was slowly added under an ice bath. After the addition, the obtained mixture was reacted under stirring at 50°C for 0.5 h. A solution of 4E (500 mg, 1.52 mmol) in N,N-dimethylformamide (5 mL) was slowly added dropwise under an ice bath. After the addition, the obtained mixture was reacted under stirring at 100°C for 2 h. After the reaction was completed, the reaction liquid was poured into ice water (60 mL). The obtained mixture was stirred for 30 min, and filtered. The obtained solid was is dried and then separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 7 (58 mg, yield: 8%).

LCMS m/z = 486.1 $[M+H]^+$;
$^1$H NMR (400 MHz, DMSO-$d_6$) δ 10.38 (s, 1H), 8.63 (s, 1H), 7.93 - 7.85 (t, 1H), 7.84 (d, 1H), 7.72 (dd, 1H), 7.54 (dd, 1H), 7.41 (d, 1H), 4.04 (s, 3H), 3.84 (s, 4H), 2.10 (t, 4H), 1.81 - 1.66 (m, 2H).

$^{19}$F NMR (377 MHz, DMSO-$d_6$) δ -127.26 (s).

**Example 8**

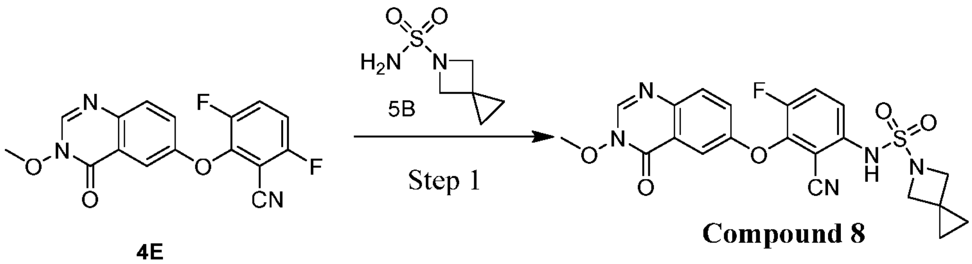


4E

Compound 8

Step 1: 5B (0.27 g, 1.52 mmol) was dissolved in dry N,N-dimethylformamide (10 mL) in a 100 mL single-neck flask. Cesium carbonate (0.74 g, 2.28 mmol) was slowly added under an ice bath. After the addition, the obtained mixture was reacted under stirring at 50°C for 0.5 h. A solution of 4E (0.5 mg, 1.52 mmol) in N,N-dimethylformamide (5 mL) was slowly added dropwise under an ice bath. After the addition, the obtained mixture was reacted under stirring at 100°C for 2 h. After the reaction was completed, the reaction liquid was filtered. The filtrate was concentrated to obtain an oily liquid, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-80% acetonitrile; cycle time: 15 min) to obtain compound 8 (0.3 g, yield: 42%).

LCMS m/z = 472.1 $[M+H]^+$;
$^1H$ NMR (400 MHz, DMSO-$d_6$) δ 10.48 (s, 1H), 8.63 (s, 1H), 7.93 - 7.86 (t, 1H), 7.84 (d, 1H), 7.72 (dd, 1H), 7.60 (dd, 1H), 7.43 (d, 1H), 4.05 (s, 3H), 3.98 (s, 4H), 0.62 (s, 4H).

$^{19}F$ NMR (377 MHz, DMSO-$d_6$) δ -127.15 (s).

**Example 9**

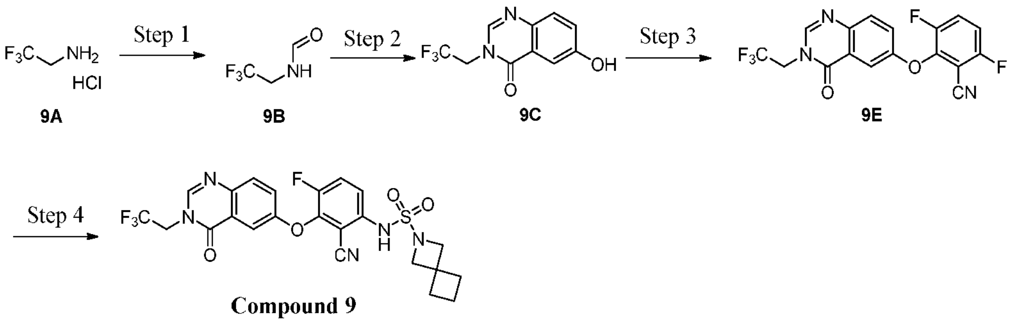


9A 9B 9C 9E

Compound 9

Step 1: 9A (25 g, 184.46 mmol) and sodium formate (15.55 g, 21.51 mmol) were added into a 250 mL single-neck flask. The obtained mixture was reacted at 130°C for 2 hrs. After the reaction was completed, the obtained mixture was cooled to room temperature and filtered. The filtrate was used to obtain 9B (22.0 g, yield 94%).

LCMS m/z = 128.2 $[M+H]^+$;
Step 2: 2-amino-5-hydroxybenzoic acid (1.0 g, 6.52 mmol) was added to 9B (6 mL, 36.2 mmol) in a 250 mL single-neck flask. The obtained mixture was reacted at 150°C for 21 hrs. After the reaction was completed, the obtained mixture was cooled to room temperature and filtered. The filter cake was washed twice with ethyl acetate (1 mL), and then the filter cake was concentrated to obtain 9C (1.3 g, yield 82%).

LCMS m/z = 245.2 $[M+H]^+$;
Step 3: 9C (0.60 g, 2.46 mmol) was dissolved in dry N,N-dimethylformamide (6 mL) in a 25 mL single-neck flask. Cesium carbonate (1.6 g, 4.91 mmol) was slowly added under an ice bath. After the addition, the obtained mixture was reacted under stirring at room temperature for 0.5 h. 2,3,6-trifluorobenzonitrile (0.41 g, 2.61 mmol) was slowly added dropwise under an ice bath. After the addition, the obtained mixture was reacted under stirring at room temperature overnight. After the reaction was completed, ethyl acetate (10 mL) was added to the reaction system to dilute the reaction. Then water (20 mL) was added to quench the reaction, and extraction was performed with ethyl acetate (20 mL x 2). The organic phase was washed with water (20 mL x 2). The organic phase was concentrated and then purified by column chromatography (petroleum ether/ethyl acetate = 4/1) to obtain 9E (0.75 g, yield 80%).

LCMS m/z = 382.0 $[M+H]^+$;
Step 4: 9E (0.50 g, 1.31 mmol) was dissolved in dry N,N-dimethylformamide (5 mL) in a 25 mL single-neck flask. Cesium carbonate (0.85 g, 2.61 mmol) and 1B (0.23 g, 1.31 mmol) was slowly added under an ice bath, After the addition, the obtained mixture was reacted under stirring at 100°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain 100 mg of solid, which was purified by column chromatography (petroleum ether/ethyl acetate = 5/1) to obtain compound 9 (22 mg, yield 3%).

LCMS m/z = 538.1 $[M+H]^+$;
$^1$H NMR (400 MHz, DMSO-$d_6$) δ 10.36 (s, 1H), 8.39 (s, 1H), 7.89-7.82 (dd, 2H), 7.75-7.72 (dd, 1H), 7.55-7.52 (dd, 1H), 7.44-7.43 (d, 1H), 4.98-4.91 (q, 2H), 3.83 (s, 4H), 2.11-2.07 (t, 4H), 1.77 - 1.70 (m, 2H).
$^{19}$F NMR (377 MHz, DMSO-$d_6$) δ -127.30 (s), -67.08 (s).

**Example 10**

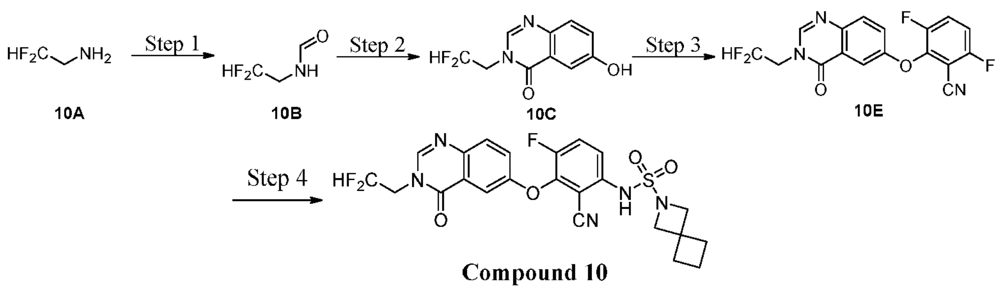

10A 10B 10C 10E

**Compound 10**

Step 1: 10A (20 g, 184.46 mmol) was added to ethyl formate (20 mL). The obtained mixture was reacted at 55°C overnight. After the reaction was completed, the reaction liquid was cooled to room temperature and concentrated to obtain residue 10B (3.1 g, yield 12%).

LCMS m/z = 100.2 $[M+H]^+$;
$^1$H NMR (400 MHz, $CDCl_3$) δ 8.23 (s, 1H), 6.32 (s, 1H), 6.01 - 5.67 (m, 1H), 3.71-3.60 (m, 2H).

Step 2: 2-amino-5-hydroxybenzoic acid (0.5 g, 3.31 mmol) was added to 10B (3.1 mL, 28.4 mmol) in a 25 mL single-neck flask. The obtained mixture was reacted at 150°C for 21 hrs. After the reaction was completed, the obtained mixture was cooled to room temperature and filtered. The filter cake was washed with ethyl acetate (0.5 mL × 2), and then the filter cake was concentrated to obtain 10C (0.70 g, yield 94%).

LCMS m/z = 227.2 $[M+H]^+$;
Step 3: 10C (0.70 g, 3.09 mmol) was dissolved in dry N,N-dimethylformamide (6 mL) in a 25 mL single-neck flask. Cesium carbonate (2.01 g, 6.17 mmol) was slowly added under an ice bath. After the addition, the obtained mixture was reacted under stirring at room temperature for 0.5 h. 2,3,6-trifluorobenzonitrile (0.51 g, 3.20 mmol) was slowly added dropwise under an ice bath. After the addition, the obtained mixture was reacted under stirring at room temperature overnight. After the reaction was completed, Ethyl acetate (5 mL) was added to the reaction system to dilute the reaction. Then water (20 mL) was added to quench the reaction, and extraction was performed with ethyl acetate (20 mL × 2). The organic phase was washed with water (20 mL × 2). The organic phase was concentrated and then purified by column chromatography (petroleum ether/ethyl acetate = 4/1) to obtain 10E (1.1 g, yield 98%).

LCMS m/z = 364.2 $[M+H]^+$;
Step 4: 10E (0.50 g, 1.38 mmol) was dissolved in dry N,N-dimethylformamide (5 mL) in a 25 mL single-neck flask. Cesium carbonate (0.90 g, 2.76 mmol) and 1B (0.24 g, 1.38 mmol) was slowly added under an ice bath, After the addition, the obtained mixture was reacted under stirring at 100°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 10 (150 mg, yield 21%).

LCMS m/z = 520.0 $[M+H]^+$;
$^1H$ NMR (400 MHz, DMSO-d6) δ 10.37 (s, 1H), 8.34 (s, 1H), 7.87-7.80 (m, 2H), 7.74-7.71 (dd, 1H), 7.54-7.50 (dd, 1H), 7.41-7.40 (d, 1H), 6.51 - 6.22 (m, 1H), 4.51-4.43 (m, 2H), 3.82 (s, 4H), 2.11-2.07 (t, 4H), 1.78 - 1.70 (m, 2H).
$^{19}F$ NMR (377 MHz, DMSO-*d6*) δ -127.22 (s), -120.46 (s).

**Example 11**

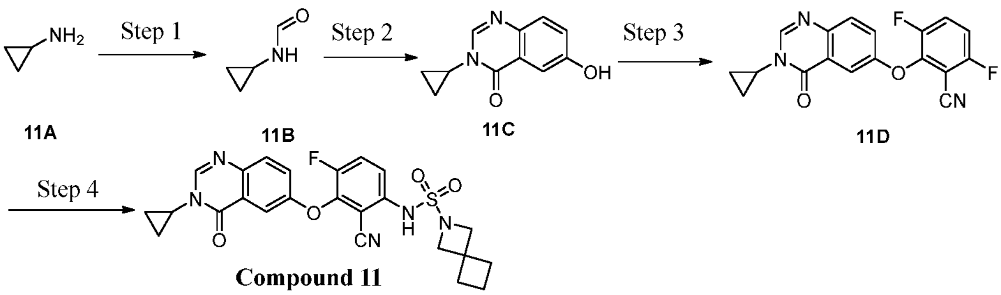


11A 11B 11C 11D

Compound 11

Step 1: 11A (5.0 g, 87.62 mmol) and ethyl formate (40 mL) were added in sequence into a 100 mL reaction flask. After the addition, the obtained mixture was stirred at 55°C for 12 h. After the reaction was completed, concentration was performed under reduced pressure to obtain 11B (7.0 g, yield 93%).

LCMS m/z = 86.20 $[M+H]^+$;
Step 2: 11B (7.0 g, 82.26 mmol) and 4A (1.5 g, 9.83 mmol) were added in sequence into a 100 mL reaction flask. After the addition, the obtained mixture was stirred at 145°C for 10 h. After the reaction was completed, filtration was performed. The obtained solid was purified by slurrying with ethyl acetate. The solid was filtered and dried to obtain 11C (1.5 g, yield 75%).

LCMS m/z = 203.10 $[M+H]^+$;
Step 3: 11C (1.5 g, 7.42 mmol) was dissolved in dry N,N-dimethylformamide (20 mL) in a 100 mL reaction flask. Cesium carbonate (3.63 g, 11.13 mmol) was added under an ice bath. After the addition, the obtained mixture was reacted at room temperature for 0.5 h. A solution of 2,3,6-trifluorobenzonitrile (1.28 g, 8.16 mmol) in N,N-dimethylformamide (15 mL) was slowly added dropwise under an ice bath. After the addition. the obtained mixture was reacted at room temperature for 2 h. The reaction liquid was diluted with ethyl acetate (50 mL), and washed with water (50 mL × 1) and saturated saline (50 mL × 1) in sequence. The organic layer was dried over anhydrous sodium sulfate, filtered, and concentrated under reduced pressure. The residue was separated and purified by column chromatography (PE/EA = 2/1) to obtain 11D (1.4 g, yield 56%).

LCMS m/z = 340.10 $[M+H]^+$;
Step 4: 11D (0.2 g, 0.59 mmol), 1B (0.2 g, 1.12 mmol), cesium carbonate (0.38 g, 1.18 mmol) and N,N-dimethylformamide (10 mL) were added in sequence into a 100 mL reaction flask. After the addition, the obtained mixture was reacted under stirring at 100°C for 12 h. Ethyl acetate (30 mL) was added to the reaction liquid. Then washing was performed with water (30 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 11 (150 mg, yield 52%).

LCMS m/z = 496.20 $[M+H]^+$;
$^1H$ NMR (400 MHz, DMSO-$d_6$) δ 10.37 (s, 1H), 8.27 (s, 1H), 7.91-7.85 (m, 1H), 7.76 (d, 1H), 7.67 (dd, 1H), 7.53 (dd, 1H), 7.37 (d, 1H), 3.84 (s, 4H), 3.28-3.14 (m, 1H), 2.13-2.06 (m, 4H), 1.79-1.70 (m, 2H), 1.06-0.90 (m, 4H).

**Example 12**

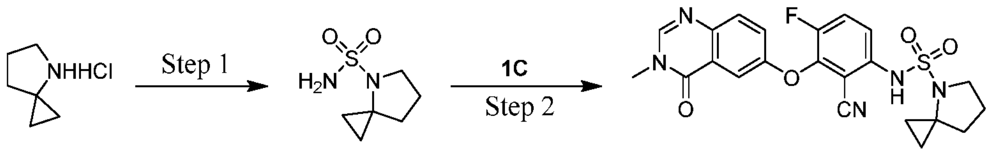


12A 12B Compound 12

Step 1: 12A (100 mg, 0.75 mmol), triethylamine (150 mg, 1.50 mmol) and dichloromethane (10 mL) were added in sequence into a 50 mL reaction flask. After the addition, the obtained mixture was stirred at 0°C for 20 min. A solution of sulfamoyl chloride (92 mg, 0.80 mmol) in dichloromethane (5 mL) was slowly added dropwise. The obtained mixture was reacted under stirring at room temperature for 2 h. The reaction liquid was diluted with dichloromethane (40 mL), and washed with water (30 mL × 1) and saturated saline (30 mL × 1) in sequence. The organic layer was dried over anhydrous sodium sulfate and filtered. The filtrate was concentrated under reduced pressure. The residue was separated and purified by column chromatography (DCM/MeOH = 15/1) to obtain 12B (80 mg, yield 55%).

Step 2: 1C (100 mg, 0.32 mmol), 12B (80 mg, 0.45 mmol), cesium carbonate (200 mg, 0.61 mmol) and N,N-dimethylformamide (10 mL) were added in sequence into a 50 mL reaction flask. After the addition, the obtained mixture was reacted under stirring at 100°C for 12 h. Ethyl acetate (40 mL) was added to the reaction liquid. Then washing was performed with water (40 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 12 (35 mg, yield 23%).

LCMS m/z = 470.50 $[M+H]^+$;
$^1$H NMR (400 MHz, DMSO-$d_6$) δ 10.23 (s, 1H), 8.35 (s, 1H), 7.92-7.84 (m, 1H), 7.78 (d, 1H), 7.68 (dd, 1H), 7.54 (dd, 1H), 7.36 (d, 1H), 3.56-3.50 (m, 2H), 3.47 (s, 3H), 1.94-1.84 (m, 2H), 1.80-1.72 (m, 2H), 1.08-0.98 (m, 2H), 0.57-0.51 (m, 2H).

**Example 13**

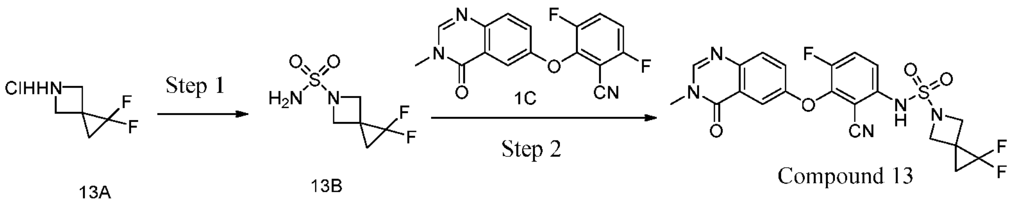


13A 13B Compound 13

Step 1: 13A (500 mg, 3.21 mmol), triethylamine (0.97 g, 9.59 mmol) and dichloromethane (10 mL) were added in sequence into a 50 mL reaction flask. After the addition, the obtained mixture was stirred at 0°C for 20 min. A solution of sulfamoyl chloride (740 mg, 6.40 mmol) in dichloromethane (7 mL) was slowly added dropwise. The obtained mixture was reacted under stirring at room temperature for 12 h. The reaction liquid was concentrated under reduced pressure to obtain 13B (500 mg, crude product).

Step 2: 1C (1.58 g, 5.04 mmol), 13B (500 mg, 2.52 mmol), cesium carbonate (2.46 g, 7.62 mmol) and N,N-dimethylformamide (15 mL) were added in sequence into a 50 mL reaction flask. After the addition, the obtained mixture was reacted under stirring at 100°C for 12 h. The reaction liquid was filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 13 (46.76 mg, yield 3.78%).

LCMS m/z = 492.4 $[M+H]^+$;
$^1$H NMR (400 MHz, $CDCl_3$) δ 8.30 (s, 1H), 7.83 (dd, 1H), 7.63-7.58 (m, 3H), 7.47 (t, 1H), 6.86 (s, 1H), 4.20-4.14 (m, 4H), 3.63 (s, 3H), 1.52 (t, 2H).

**Example 14**

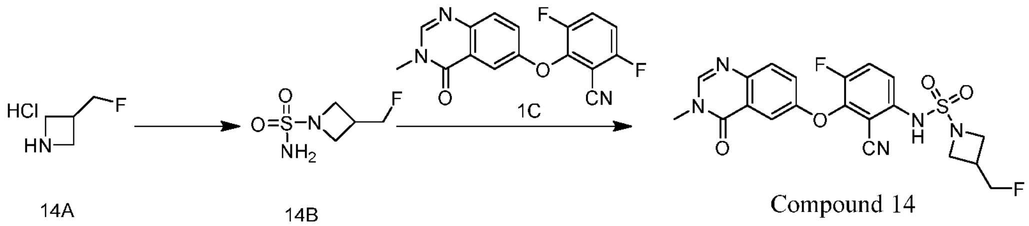


14A 14B Compound 14

Step 1: Sulfamoyl chloride (0.18g, 1.59 mmol), triethylamine (0.48 g, 4.74 mmol) and dichloromethane (5 mL) were added in sequence into a 50 mL reaction flask. After the addition, the obtained mixture was stirred at 0°C for 20 min. 14A (0.20 g, 1.59 mmol) was slowly added dropwise. The obtained mixture was reacted under stirring at room temperature for 1 h. The reaction liquid was concentrated under reduced pressure, and the crude product was used directly in the next step without purification.

Step 2: 1C (490 mg, 1.56 mmol), 14B (0.30 g crude product), cesium carbonate (1.52 mg, 4.67 mmol) and N,N-dimethylformamide (5 mL) were added in sequence into a 50 mL reaction flask. After the addition, the obtained mixture was reacted under stirring at 100°C for 18 h. Ethyl acetate (50 mL) was added to the reaction liquid. Then washing was performed with water (40 mL × 2). The organic layer was dried over anhydrous sodium sulfate and filtered. The filtrate was concentrated under reduced pressure. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: gradient elution using 5%-90% acetonitrile; cycle time: 20 min; retention time: 7 min) to obtain compound 14 (28 mg, yield 3.89%).

LCMS m/z = 462.1 $[M+H]^+$;
$^1$H NMR (400 MHz, $CD_3OD$) δ 8.22 (s, 1H), 7.75 - 7.70 (m, 1H), 7.57 (dd, 2H), 7.47 (dd, 1H), 7.33 - 7.25 (m, 1H), 4.59 (d, 1H), 4.47 (d, 1H), 3.88 (t, 2H), 3.64 (dd, 2H), 3.57 (s, 3H),2.88 - 2.76 (m, 1H).
$^{19}$F NMR (377 MHz, DMSO-$d_6$) δ -146.12 (s), -220.65 (s).

**Example 15**

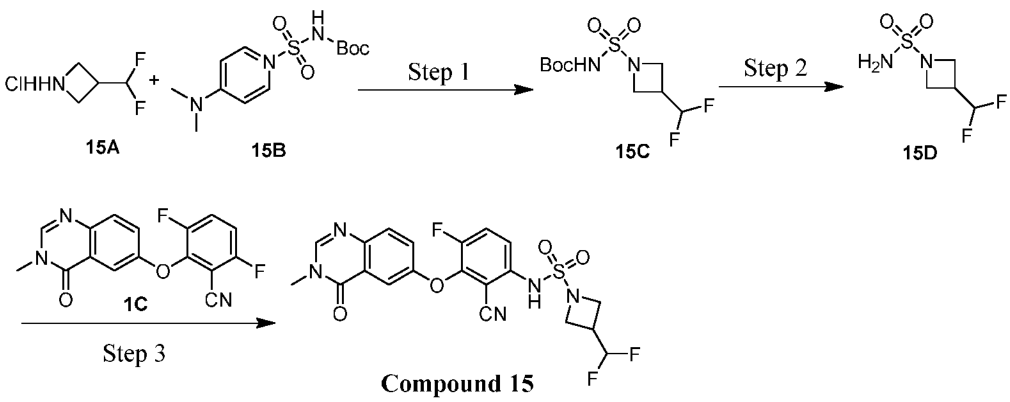


15A 15B 15C 15D 1C **Compound 15**

Step 1: 15A (0.25 g, 1.74 mmol) was dissolved in dry acetonitrile (15 mL) in a 100 mL single-neck flask. 15B (0.53 g, 1.74 mmol) was added. After the addition, the obtained mixture was reacted under stirring at 30°C for 1 h. After the reaction was completed, the reaction liquid was concentrated and the obtained solid was the crude compound 15C, which was directly used in the next reaction without purification.

Step 2: Compound 15C (crude product) and dichloromethane (12 ml) were added into a single-neck flask. Trifluoroacetic acid (3 ml) was added, The obtained mixture was reacted at room temperature for 2 h. Concentration was performed to obtain compound 15D, which was directly used in the next reaction without purification.

Step 3: 15D (crude product) was dissolved in dry N,N-dimethylformamide (10 mL) in a 100 mL single-neck flask. Cesium carbonate (2.27 g, 6.97 mmol) and 1C (300 mg, 0.96 mmol) were slowly added under an ice bath. After the

addition, the obtained mixture was reacted under stirring at 100°C for 6 h. After the reaction was completed, the reaction liquid was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: gradient elution using 5%-90% acetonitrile; cycle time: 20 min; retention time: 7 min) to obtain compound 15 (120 mg, total yield over three steps: 14.4%).

LCMS m/z = 480.4 $[M+H]^+$;
$^1H$ NMR (400 MHz, $CD_3OD$) δ 8.25 (s, 1H), 7.77 - 7.72 (m, 1H), 7.59 (dd, 2H), 7.46 (dd, 1H), 7.30 (t, 1H), 6.11 (td, 1H), 3.91 (t, 2H), 3.82 - 3.75 (m, 2H), 3.59 (s, 3H), 2.98 - 2.84 (m, 1H).
$^{19}F$ NMR (377 MHz, DMSO-$d_6$) δ -121.73 (s), -145.77 (s).

**Example 16**

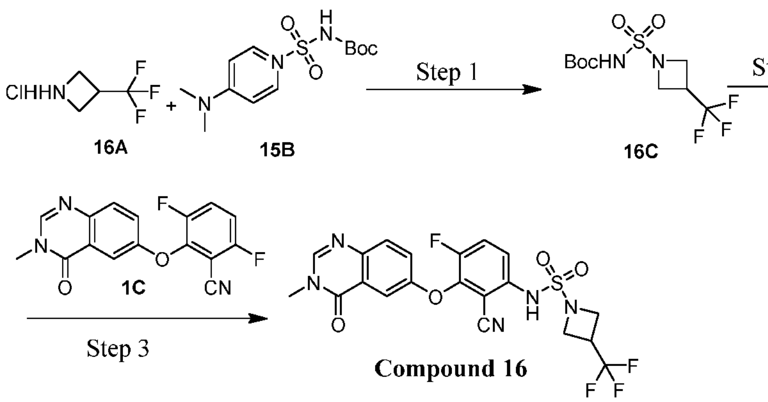


16A 15B 16C 16D 1C Compound 16

Step 1: 3-(trifluoromethyl)azetidine hydrochloride (16A) (0.25 g, 1.55 mmol) was dissolved in dry acetonitrile (15 mL) in a 100 mL single-neck flask. 15B (0.47 g, 1.55 mmol) was added. After the addition, the obtained mixture was reacted under stirring at 30°C for 1 h. After the reaction was completed, the reaction liquid was concentrated and the obtained solid was the crude compound 16C, which was directly used in the next reaction without purification.

Step 2: Compound 16C (crude product) and dichloromethane (12 ml) were added into a single-neck flask. Trifluoroacetic acid (3 ml) was added, The obtained mixture was reacted at room temperature for 2 h. Concentration was performed to obtain compound 16D, which was directly used in the next reaction without purification.

Step 3: 16D (crude product) was dissolved in dry N,N-dimethylformamide (10 mL) in a 100 mL single-neck flask. Cesium carbonate (2.02 g, 6.20 mmol) and 1C (300 mg, 0.96 mmol) were slowly added under an ice bath. After the addition, the obtained mixture was reacted under stirring at 100°C for 6 h. After the reaction was completed, the reaction liquid was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: gradient elution using 5%-90% acetonitrile; cycle time: 20 min; retention time: 8 min) to obtain compound 16 (100 mg, total yield over three steps: 12.9%).

LCMS m/z = 498.5 $[M+H]^+$;
$^1H$ NMR (400 MHz, $CD_3OD$) δ 8.23 (s, 1H), 7.75 - 7.70 (m, 1H), 7.57 (dd, 2H), 7.45 (dd, 1H), 7.34 - 7.26 (m, 1H), 3.96 (t, 2H), 3.89 - 3.82 (m, 2H), 3.57 (s, 3H), 3.33 - 3.24 (m, 1H).
$^{19}F$ NMR (377 MHz, DMSO-$d_6$) δ -70.81 (s), -145.19 (s).

**Example 17**

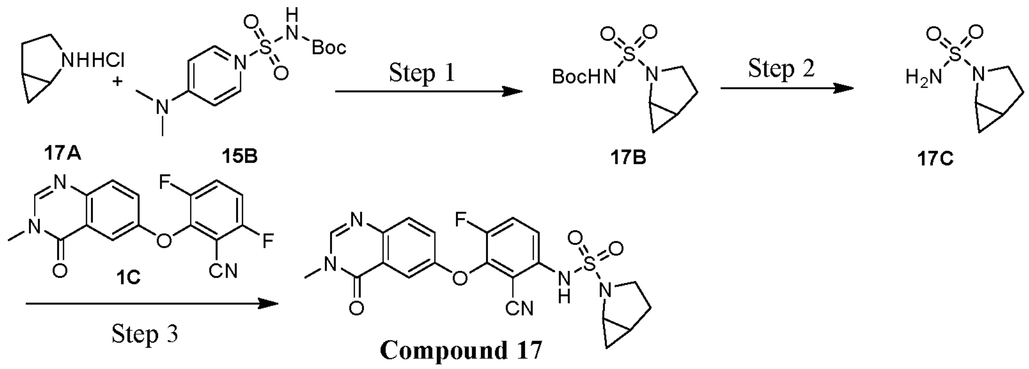


17A 15B 17B 17C 1C Compound 17

Step 1: 2-azabicyclo[3.1.0]hexane hydrochloride (17A) (0.47 g, 3.97 mmol) was dissolved in dry acetonitrile (25 mL) in a 100 mL single-neck flask. 15B (1.2 g, 3.97 mmol) was added. After the addition, the obtained mixture was reacted under stirring at 70°C for 4 h. After the reaction was completed, the reaction liquid was concentrated and the obtained solid was the crude compound 17B, which was directly used in the next reaction without purification.

Step 2: Compound 17B (680 mg, 2.59 mmol) and dichloromethane (12 ml) were added into a single-neck flask. Trifluoroacetic acid (3 ml) was added, The obtained mixture was reacted at room temperature for 2 h. After the obtained mixture was concentrated, the residue was separated by column chromatography (petroleum ether: ethyl acetate (v/v) = 1:0 - 0:1) to obtain compound 17C (420 mg, yield: 99%).

Step 3: 17C (217 mg, 1.34 mmol) was dissolved in dry N,N-dimethylformamide (10 mL) in a 100 mL single-neck flask. Cesium carbonate (655 mg, 2.01 mmol) was slowly added under an ice bath. After the addition, the obtained mixture was reacted under stirring at 50°C for 0.5 h. A solution of 1C (420 mg, 1.34 mmol) in N,N-dimethylformamide (5 mL) was slowly added dropwise under an ice bath. After the addition, the obtained mixture was reacted under stirring at 100°C for 2 h. After the reaction was completed, the reaction liquid was filtered. The filtrate was concentrated to obtain an oily liquid, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-80% acetonitrile; cycle time: 15 min) to obtain compound 17 (56 mg, yield: 9%).

LCMS m/z = 456.5 $[M+H]^+$;
$^1H$ NMR (400 MHz, DMSO-$d_6$) δ 10.39 (s, 1H), 8.36 (s, 1H), 7.86 (t, 1H), 7.78 (d, 1H), 7.68 (dd, 1H), 7.63 (dd, 1H), 7.38 (d, 1H), 3.48 (s, 3H), 3.39 (t, 1H), 3.18 (m, 1H), 2.93 (m, 1H), 2.05 (m, 1H), 1.98 (m, 1H), 1.61 (m, 1H), 0.78 (m, 1H), 0.52 (m, 1H).
$^{19}F$ NMR (377 MHz, DMSO-$d_6$) δ -128.22 (s).

**Example 18**

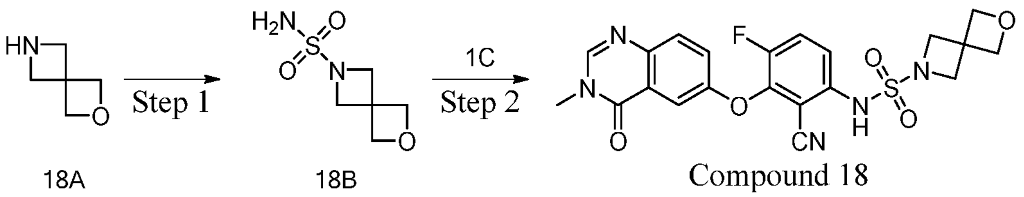


18A 18B Compound 18

Step 1: 18A (500 mg, 5.08 mmol), triethylamine (1.54 g, 15.24 mmol) and dichloromethane (10 mL) were added in sequence into a 50 mL reaction flask. After the addition, the obtained mixture was stirred at 0°C for 20 min. A solution of sulfamoyl chloride (1.17 g, 10.16 mmol) in dichloromethane (7 mL) was slowly added dropwise. The obtained mixture was reacted under stirring at room temperature for 12 h. The reaction liquid was concentrated under reduced pressure to obtain 18B (500 mg, crude product).

Step 2: 1C (1.76 g, 5.62 mmol), 18B (500 mg, 2.81 mmol), cesium carbonate (2.75 g, 8.37 mmol) and N,N-dimethylformamide (15 mL) were added in sequence into a 50 mL reaction flask. After the addition, the obtained mixture was reacted under stirring at 100°C for 12 h. The reaction liquid was filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm);

composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 18 (22.34 mg, yield 1.7%).

LCMS m/z = 472.5 $[M+H]^+$;
$^1$H NMR (400 MHz, $CDCl_3$) δ 8.31 (s, 1H), 7.84 (d, 1H), 7.62 (dd, 1H), 7.57 (d, 1H), 7.54 (dd, 1H), 7.45 (t, 1H), 6.95 (s, 1H), 4.78 (s, 4H), 4.18 (s, 4H), 3.63 (s, 3H).

**Example 19**

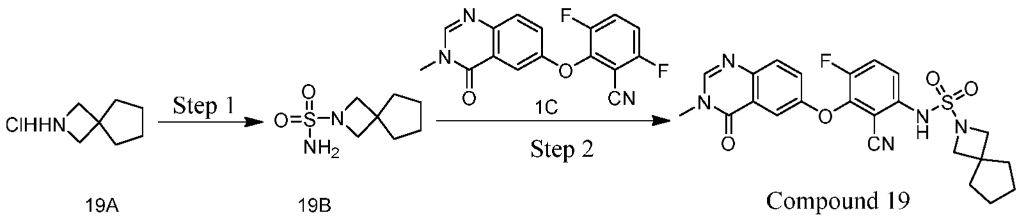


19A 19B Compound 19

Step 1: 19A (500 mg, 4.49 mmol), triethylamine (1.36 g, 13.47 mmol) and dichloromethane (10 mL) were added in sequence into a 50 mL reaction flask. After the addition, the obtained mixture was stirred at 0°C for 20 min. A solution of sulfamoyl chloride (520 mg, 4.49 mmol) in dichloromethane (7 mL) was slowly added dropwise. The obtained mixture was reacted under stirring at room temperature for 1-2 h. The reaction liquid was diluted with dichloromethane (100 mL), and washed with water (40 mL × 1) and saturated saline (40 mL × 1) in sequence. The organic layer was dried over anhydrous sodium sulfate and filtered. The filtrate was concentrated under reduced pressure. The residue was separated and purified by column chromatography (DCM/MeOH = 12/1) to obtain 19B (600 mg, yield 70%).

Step 2: 1C (0.9 g, 2.87 mmol), 19B (0.6 g, 3.16 mmol), cesium carbonate (1.12 g, 3.44 mmol) and N,N-dimethylformamide (20 mL) were added in sequence into a 100 mL reaction flask. After the addition, the obtained mixture was reacted under stirring at 80°C for 18 h. Ethyl acetate (100 mL) was added to the reaction liquid. Then washing was performed with water (40 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 19 (12 mg, yield 1%).

LCMS m/z = 484.60 $[M+H]^+$;
$^1$H NMR (400 MHz, DMSO-$d_6$) δ 10.36 (s, 1H), 8.36 (s, 1H), 7.88 (t, 1H), 7.78 (d, 1H), 7.68 (dd, 1H), 7.56 (dd, 1H), 7.39 (d, 1H), 3.72 (s, 4H), 3.48 (s, 3H), 1.71 (m, 4H), 1.50 (m, 4H).
$^{19}$F NMR (377 MHz, DMSO-$d_6$) δ -127.31 (s).

**Example 20 and example 21**

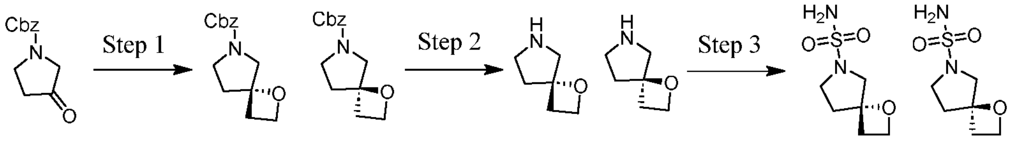


20A 20B 21B 20C 21C 20D 21D

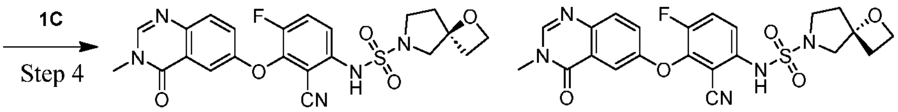


**Compound 20**

**Compound 21**

Step 1: A 500 mL reaction flask was taken, and trimethylsulfoxide iodide (100.45 g, 456.62 mmol) was dissolved in tert-butanol (350 mL) at room temperature. Then potassium tert-butoxide (45.0 g, 401.83 mmol) was added. The obtained mixture was reacted at 50°C for 1 h, and then cooled to room temperature. 20A (40.0 g, 182.65 mmol) was slowly added. After the addition, the obtained mixture was reacted at 50°C for 48 h. After the reaction was completed, saturated ammonium chloride solution (20 mL) was added to quench the reaction. Water (50 mL) was added. Extraction was performed with ethyl acetate (150 mL × 2). The combined organic phase was washed with saturated brine (50 mL × 1), dried over anhydrous sodium sulfate and filtered. After the filtrate was concentrated, the residue was separated and purified by silica gel column chromatography (petroleum ether: ethyl acetate (v/v) = 2:1) to obtain 10.0 g of a racemate. The racemate was separated by chiral preparative HPLC to obtain peak 1 (3.0 g, retention time 8.45 min, set as 20B) and peak 2 (3.0 g, retention time 11.62 min, set as 21B), respectively, preparative chromatography separation conditions: instrument SFC Prep 150 AP; chromatographic column: Daicel AD-H (19 mm × 250 mm); mobile phase system: A for $CO_2$ and B for MeOH; gradient: B 10%; 5. Flow rate: 45mL/min.

Step 2: A 100 mL reaction flask was taken, and compound 20B (3.0 g, 12.13 mmol) was dissolved in methanol (30 mL) at room temperature. Pd/C (0.3 g, Pd content 10%) was added. The obtained mixture was reacted under hydrogen atmosphere for 3 h. After the reaction was completed, filtration was performed, the filtrate was concentrated to obtain the crude title compound 20C (1.1 g, 80%), which was directly used in the next reaction without purification.

LCMS m/z = 114.2 $[M+H]^+$.

Step 3: A 50 mL reaction flask was taken, and compound 20C (1.1 g, 9.72 mmol) was dissolved in 1,4-dioxane (15 mL) at room temperature. Sulfonamide (0.78 g, 8.11 mmol) was added. The obtained mixture was reacted at 100°C for 12 h. After the reaction was completed, water (10 mL) was added, and ethyl acetate (15 mL×2) was used for extraction. The combined organic phase was washed with saturated brine (10 mL×1), dried over anhydrous sodium sulfate and filtered. The filtrate was concentrated. The residue was separated and purified by silica gel column chromatography (petroleum ether:ethyl acetate (v/v) = 1 : 1) to obtain the title compound 20D (1.3 g, 70%).

LCMS m/z = 193.1 $[M+H]^+$.

Step 4: 1C (407.4 mg, 1.30 mmol), 20D (500 mg, 2.60 mmol), cesium carbonate (813.8 mg, 2.60 mmol) and N,N-dimethylformamide (10 mL) were added in sequence into a 50 mL reaction flask at room temperature. After the addition, the obtained mixture was reacted under stirring at 100°C for 12 h. After the reaction was completed, water (10 mL) was added, and ethyl acetate (30 mL×2) was used for extraction. The combined organic phase was washed with saturated brine (10 mL×1), dried over anhydrous sodium sulfate and filtered. The filtrate was concentrated. The residue was prepared by preparative liquid chromatography, HPLC separation method: 1. Instruments: waters 2767 preparative liquid chromatographic instrument; chromatographic column: SunFire@ Prep C18 (19 mm × 250 mm). 2. The sample was filtered with a 0.45 μm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; d. elution time: 20 min. retention time 7.0 min, to obtain compound 20 (230 mg, 18%).

LCMS m/z = 486.1 $[M+H]^+$.

$^1H$ NMR (400 MHz, $CDCl_3$) δ 8.00 (s, 1H), 7.73 (d, 1H), 7.68 (s, 1H), 7.61 (d, 1H), 7.58 (dd, 1H), 7.52 (dd, 1H), 7.41 - 7.34 (m, 1H), 4.57 - 4.45 (m, 2H), 3.93 - 3.87 (m, 1H), 3.56 (s, 3H), 3.56 - 3.51 (m, 2H), 3.40 (d, 1H), 2.73 - 2.63 (m, 2H), 2.49 - 2.40 (m, 1H), 2.07 - 1.98 (m, 1H).

Compound 21 (200 mg) can be obtained by using 21B (3 g) as raw material through the same synthesis method as compound 20.

LCMS m/z = 486.1 $[M+H]^+$.

$^1H$ NMR (400 MHz, $CDCl_3$) δ 8.01 (s, 1H), 7.75 (s, 1H), 7.73 (d, 1H), 7.61 (d, 1H), 7.57 (dd, 1H), 7.51 (dd, 1H), 7.40

- 7.34 (m, 1H), 4.56 - 4.45 (m, 2H), 3.92 - 3.86 (m, 1H), 3.56 (s, 3H), 3.55 - 3.51 (m, 2H), 3.40 (d, 1H), 2.71 - 2.63 (m, 2H), 2.47 - 2.40 (m, 1H), 2.06 - 1.98 (m, 1H).

Example 22

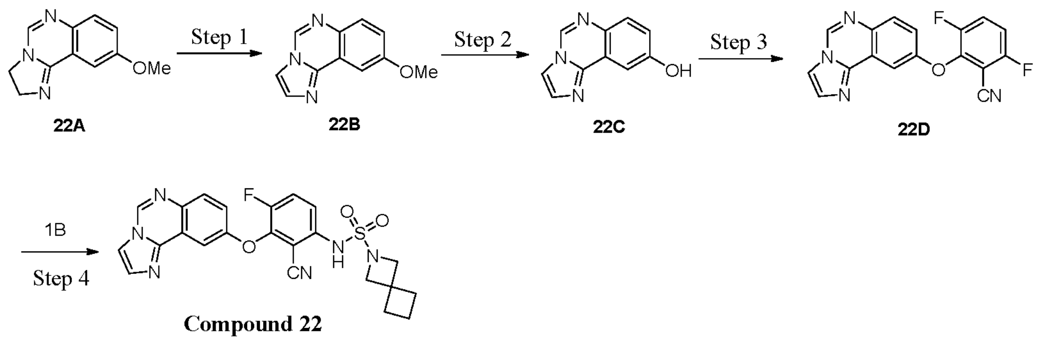


22A 22B 22C 22D

Compound 22

Step 1: 22A (0.8 g, 3.98 mmol) (synthesis of compound 22A was referred to patent WO2019/60611, 2019, A1) was dissolved in acetonitrile (20 mL) in a 100 mL reaction flask. Potassium permanganate (1.20 g, 7.61 mmol) and basic alumina (1.0 g, 9.79 mmol) were added in sequence. After the addition, the obtained mixture was reacted at room temperature for 5 h. After the reaction was completed, filtration was performed. The resulting filtrate was concentrated, The residue was separated and purified by column chromatography (PE/EA = 1/1) to obtain 22B (0.65 g, yield 82%).

LCMS m/z = 200.10 $[M+H]^+$;

Step 2: 22B (0.6 g, 3.01 mmol) was dissolved in N, N-dimethylformamide (10 mL) in a 100 mL reaction flask. Then sodium thioethylate (0.50 g, 5.94 mmol) was added. After the reaction liquid was subjected to nitrogen replacement three times, the reaction liquid was heated to 130°C and reacted for 10 h. After the reaction was completed, filtration was performed. The obtained filtrate was concentrated to obtain 22C (0.45 g, yield 80%).

LCMS m/z = 186.10 $[M+H]^+$;

Step 3: 22C (0.45 g, 2.43 mmol) was dissolved in N, N-dimethylformamide (10 mL) in a 100 mL reaction flask. Cesium carbonate (1.20 g, 3.75 mmol) was slowly added under an ice-water bath. After the addition, the obtained mixture was stirred at room temperature for 0.5 h. A solution of 2,3,6-trifluorobenzonitrile (0.38 g, 2.43 mmol) N,N-dimethylformamide (5 mL) was slowly added dropwise under an ice-water bath. After the addition, the obtained mixture was reacted at room temperature for 1 h. The reaction liquid was diluted with ethyl acetate (50 mL), and washed with water (50 mL × 1) and saturated saline (50 mL × 1) in sequence. The organic layer was dried over anhydrous sodium sulfate, filtered, and concentrated under reduced pressure. The residue was separated and purified by column chromatography (PE/EA = 2/1) to obtain 22D (0.55 g, yield 70%).

LCMS m/z = 323.30 $[M+H]^+$;

Step 4: 22D (0.25 g, 0.78 mmol), 1B (0.21 g, 1.17 mmol), cesium carbonate (0.51 g, 1.56 mmol) and N,N-dimethylformamide (15 mL) were added in sequence into a 100 mL reaction flask. After the addition, the obtained mixture was reacted under stirring at 100°C for 12 h. Ethyl acetate (30 mL) was added to the reaction liquid. Then washing was performed with water (30 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 10 min) to obtain compound 22 (150 mg, yield 40%).

LCMS m/z = 479.10 $[M+H]^+$;

$^1$H NMR (400 MHz, DMSO-$d_6$) δ 10.38 (s, 1H), 9.27 (s, 1H), 8.09 (d, 1H), 8.01 (d, 1H), 7.87 (t, 1H), 7.62 (d, 1H), 7.59 (d, 1H), 7.58-7.53 (m, 2H), 3.83 (s, 4H), 2.08 (t, 4H), 1.75-1.66 (m, 2H).

**Example 23**

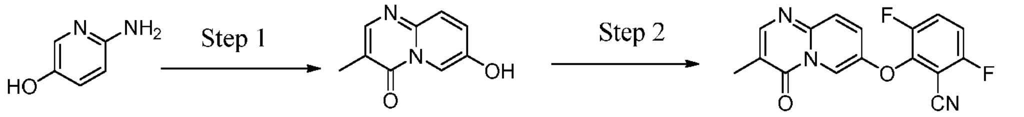


23A 23B 23C

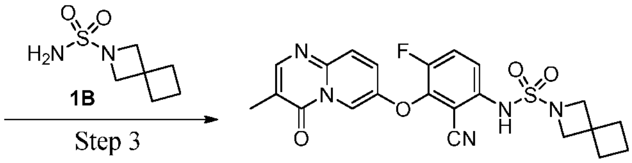


Compound **23**

Step 1: 23A (1.00 g, 9.11 mmol) and acetic acid (15 mL) were added into a single-neck flask. Then ethyl 3-ethoxy-2-methyl-2-acrylate (2.88 g, 18.22 mmol) was added. The obtained mixture was heated to reflux and reacted for 16 h. After the reaction was completed as monitored by LCMS, the acetic acid was spun off, and the remaining solid was slurried with ethyl acetate (20 mL). The solid was filtered and dried to obtain product 23B (1.2 g, 74.7%).

LC-MS (ESI): m/z = 177.1 $[M+H]^+$.

Step 2: 23B (0.20 g, 1.14 mmol) was dissolved in N,N-dimethylformamide (5 mL). Then cesium carbonate (0.74 g, 2.27 mmol) was added. 2,3,6-trifluorobenzonitrile (0.23 g, 1.48 mmol) was added under stirring at 0°C. After the addition, the temperature was slowly raised to room temperature for reaction for 2 h. After the reaction was completed as monitored by LCMS, the reaction liquid was poured into water (15 mL), and a large amount of solid was precipitated. The solid was filtered and dried to obtain the product 23C (0.12 g, 33.6%).

LC-MS (ESI): m/z = 314.1 $[M+H]^+$.

Step 3: 23C (120.0 mg, 0.38 mmol), 1B (87.0 mg, 0.49 mmol), cesium carbonate (250.0 mg, 0.76 mmol) and N,N-dimethylformamide (5 mL) were added in sequence into a single-neck flask. After the addition, the obtained mixture was reacted under stirring at 80°C for 18 h. After the reaction was completed as monitored by LCMS, ethyl acetate (50 mL) was added to the reaction liquid. Then washing was performed with water (40 mL × 2). The organic layer was dried over anhydrous sodium sulfate and filtered. The filtrate was concentrated under reduced pressure. The residue was separated and purified by column chromatography (DCM/MeOH = 10/1) to obtain compound 23 (50.0 mg, 28.0%).

$^1$H NMR ((400 MHz, DMSO-$d_6$) δ 10.41 (s, 1H), 8.38 (d, 1H), 8.30 (d, 1H), 8.00 (dd, 1H), 7.86 (t, 1H), 7.78 (d, 1H), 7.53 (dd, 1H), 3.82 (s, 4H), 2.12 (s, 3H), 2.09 (t, 4H), 1.77-1.69 (m, 2H).

LC-MS (ESI): m/z = 470.1 $[M+H]^+$.

**Example 24**

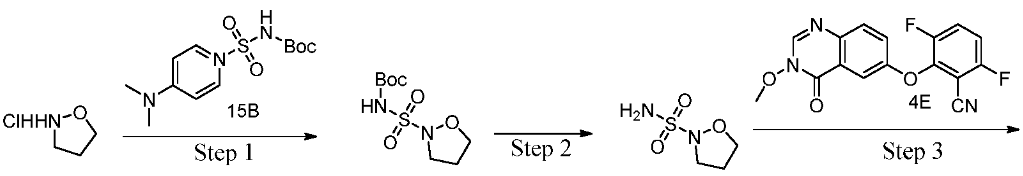


24A 24B 24C

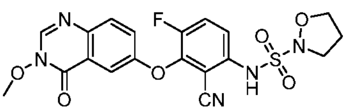

Compound 24

Step 1: 24A (1.2 g, 10.95 mmol), triethylamine (3.32 g, 32.85 mmol) and acetonitrile (20 mL) were added in sequence into a 50 mL reaction flask. After the addition, the obtained mixture was stirred for 30 min. 15B (4.97g, 16.43 mmol) was slowly added dropwise. The obtained mixture was reacted under stirring at 40°C for 3 h. The reaction liquid was concentrated under reduced pressure to obtain 24B (1 g, crude product).

Step 2: 24B (0.8 g, 3.17 mmol) and dichloromethane (6 mL) were added in sequence into a 50 mL reaction flask. Trifluoroacetic acid (2 mL) was slowly added dropwise. The obtained mixture was reacted under stirring at room

temperature for 1 h. The reaction liquid was concentrated under reduced pressure to obtain 24C (0.8 g, crude product).

Step 3: 24C (0.8 g, 5.26 mmol), cesium carbonate (2.57 g, 7.89 mmol) and N,N-dimethylformamide (15 mL) were added in sequence into a 50 mL reaction flask. After the addition, the obtained mixture was reacted under stirring at 50°C for 2 h. Then 4E (2.08 g, 6.31 mmol) was added. The obtained mixture was reacted under stirring at 80°C for 12 h. The reaction liquid was filtered, the filtrate was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 24 (91.8 mg, yield 3.79%).

LCMS m/z = 462.1 $[M+H]^+$;

$^1H$ NMR (400 MHz, $CDCl_3$) δ 8.26 (s, 1H), 7.80 (d, 1H), 7.65-7.61 (m, 2H), 7.58 (dd, 1H), 7.44 (t, 1H), 4.24 (t, 2H), 4.15 (s, 3H), 3.73 (t, 2H), 2.47-2.40 (m, 2H).

**Example 25**

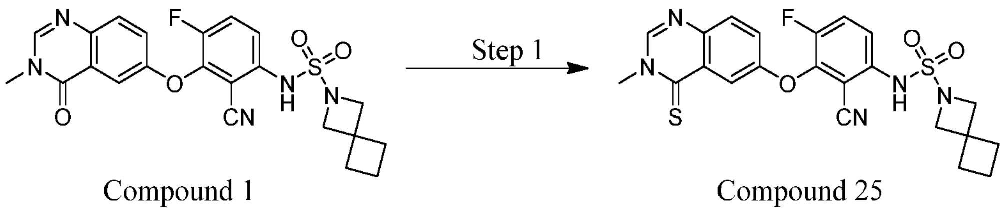


Compound 1 Compound 25

Step 1: Compound 1 (180 mg, 0.38 mmol), Lawesson's reagent (768 mg, 1.90 mmol) and dry tetrahydrofuran (9 mL) were added in sequence into a 25 mL reaction flask. After the addition, the obtained mixture was reacted under stirring at 80°C for 4 h. The reaction liquid was directly concentrated under reduced pressure. The residue was separated and purified by column chromatography (DCM/MeOH = 15/1) to obtain a crude product. Compound 25 (35 mg, yield 19%) was obtained through separation and purification by preparative liquid chromatography.

LCMS m/z = 486.1 $[M+H]^+$;

$^1H$ NMR (400 MHz, DMSO-$d_6$) δ 10.41 (s, 1H), 8.70 (s, 1H), 7.99 (d, 1H), 7.89 (dd, 2H), 7.77 (dd, 1H), 7.56 (dd, 1H), 3.87 (s, 3H), 3.83 (s, 4H), 2.09 (t, 4H), 1.78 - 1.65 (m, 2H).

$^{19}F$ NMR (377 MHz, DMSO-$d_6$) δ -127.24 (s).

**Example 26**

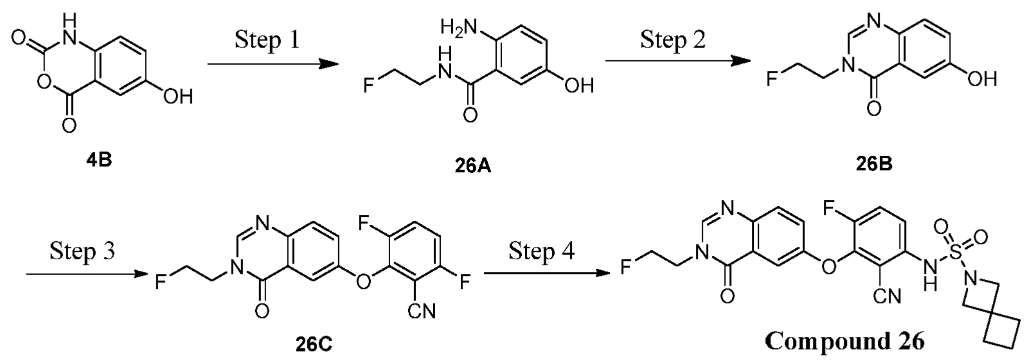


4B 26A 26B

26C Compound 26

Step 1: 2-fluoroethylamine hydrochloride (9.0 g, 90.53 mmol) was dissolved in water (80 mL) in a 250 mL reaction flask. An aqueous solution of sodium hydroxide (3.3 g, 82.54 mmol) was added under an ice-water bath. After the obtained mixture was stirred for 30 min, 4B (4.0 g, 22.29 mmol) was added and the obtained mixture was stirred at room temperature for 4 h. After the reaction was completed, concentration was performed under reduced pressure. The residue was dissolved in a mixed solvent of dichloromethane (100 mL) and methanol (50 mL). After filtration, the filtrate was

concentrated under reduced pressure. The residue was separated and purified by column chromatography (PE/EA = 1/1) to obtain 26A (3.8 g, yield 86%).

LCMS m/z = 199.10 $[M+H]^+$;

Step 2: 26A (1.5 g, 8.58 mmol) was dissolved in triethyl orthoformate (10 mL) in a 20 mL microwave tube. The reaction liquid was heated to 185°C and reacted for 2 h. After the reaction was completed, filtration was performed. The obtained filter cake was spin-dried to obtain 26B (1.6 g, yield 89%).

LCMS m/z = 209.10 $[M+H]^+$;

Step 3: 26B (1.5 g, 7.20 mmol) was dissolved in N, N-dimethylformamide (20 mL) in a 100 mL reaction flask. Cesium carbonate (3.52 g, 10.80 mmol) was slowly added under an ice-water bath. After the obtained mixture was stirred at room temperature for 30 min, a solution of 2,3,6-trifluorobenzonitrile (1.36 g, 8.64 mmol) in N,N-dimethylformamide (5 mL) was slowly added dropwise under an ice-water bath. After the addition, the obtained mixture was reacted at room temperature for 2 h. The reaction liquid was diluted with ethyl acetate (100 mL), and washed with water (100 mL × 1) and saturated saline (100 mL × 1) in sequence. The organic layer was dried over anhydrous sodium sulfate, filtered, and concentrated under reduced pressure. The residue was separated and purified by column chromatography (PE/EA = 2/1) to obtain 26C (1.35 g, yield 54%).

LCMS m/z = 346.10 $[M+H]^+$;

Step 4: 26C (0.4 g, 1.16 mmol), 1B (0.31 g, 1.74 mmol), cesium carbonate (0.76 g, 2.32 mmol) and N,N-dimethylformamide (15 mL) were added in sequence into a 100 mL reaction flask. After the addition, the obtained mixture was reacted at 100°C for 12 h. Ethyl acetate (30 mL) was added to the reaction liquid. Then washing was performed with water (30 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 10 min) to obtain compound 26 (320 mg, yield 55%).

LCMS m/z = 502.10 $[M+H]^+$;

$^1$H NMR (400 MHz, DMSO-$d_6$) δ 10.37 (s, 1H), 8.33 (s, 1H), 7.88 (t, 1H), 7.80 (d, 1H), 7.76-7.68 (m, 1H), 7.56-7.51 (m, 1H), 7.39 (d, 1H), 4.79-4.62 (m, 2H), 4.36-4.25 (m, 2H), 3.83 (s, 4H), 2.09 (t, 4H), 1.78-1.69 (m, 2H).

## Example 27

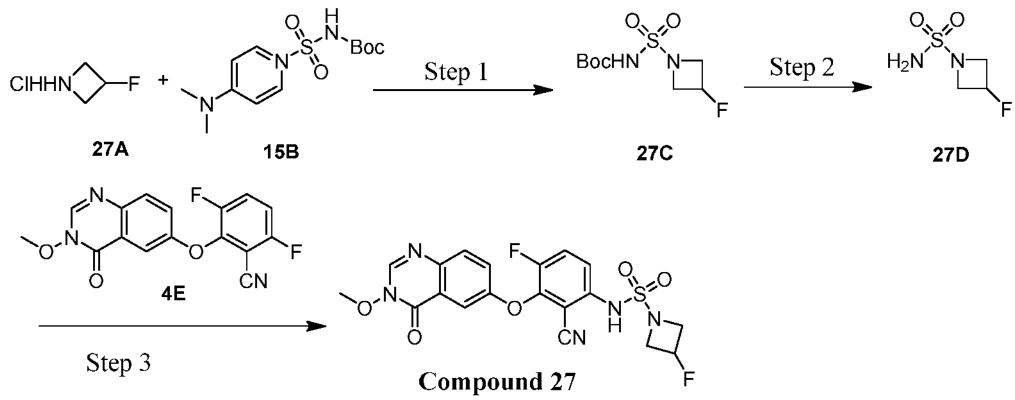


27A 15B 27C 27D 4E Compound 27

Step 1: 27A (1.0 g, 8.96 mmol) was dissolved in dry acetonitrile (15 mL) in a 100 mL single-neck flask. 15B (2.71 g, 8.96 mmol) and triethylamine (1.81 g, 17.96 mmol) were added. After the addition, the obtained mixture was reacted under stirring at 30°C for 1 h. After the reaction was completed, the reaction liquid was concentrated and the obtained solid was the compound 27C, which was directly used in the next reaction without purification.

Step 2: Compound 27C and dichloromethane (12 ml) were added into a single-neck flask. Trifluoroacetic acid (6 ml) was added, The obtained mixture was reacted at room temperature for 2 h. After concentration, the crude product was purified by silica gel column chromatography (petroleum ether:ethyl acetate = 3:1) to obtain compound 27D (800 mg, total yield over two steps: 57.9%).

Step 3: 27D (0.45 g, 2.92 mmol) was dissolved in dry N,N-dimethylformamide (6 mL) in a 100 mL single-neck flask. Cesium carbonate (0.95 g, 2.92 mmol) and 4E (800 mg, 2.43 mmol) were slowly added. After the addition, the obtained mixture was reacted under stirring at 80°C for 6 h. After the reaction was completed, the reaction liquid was

filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: gradient elution using 5%-90% acetonitrile; cycle time: 20 min; retention time: 7.5 min) to obtain compound 27 (85 mg, yield 7.5%).

LCMS m/z = 464.1 $[M+H]^+$;
$^1H$ NMR (400 MHz, DMSO-$d_6$) δ 10.63 (s, 1H), 7.96 - 7.78 (m, 2H), 7.73 (dd, 1H), 7.56 (dd, 1H), 7.44 (d, 1H), 5.47-5.28 (m, 1H), 4.25-4.16 (m, 2H), 4.05 (s, 3H), 4.04 - 3.95 (m, 2H).
$^{19}F$ NMR (377 MHz, DMSO-$d_6$) δ -126.63 (s), -177.31 (s).

**Example 28**

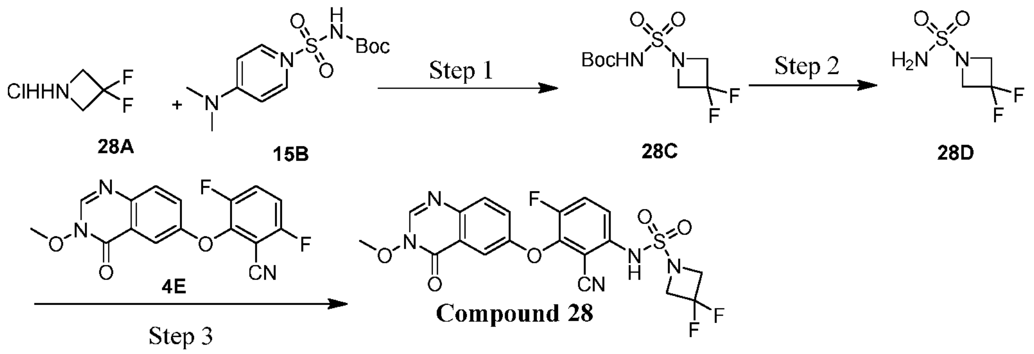


Step 1: 28A (1.0 g, 7.72 mmol) was dissolved in dry acetonitrile (15 mL) in a 100 mL single-neck flask. were added 15B (2.33 g, 7.72 mmol) and triethylamine (1.56 g, 15.44 mmol), After the addition, the obtained mixture was reacted under stirring at 30°C for 1 h. After the reaction was completed, the reaction liquid was concentrated and the obtained solid was the crude compound 28C, which was directly used in the next reaction without purification.

Step 2: Compound 28C (crude product) and dichloromethane (12 ml) were added into a single-neck flask. Trifluoroacetic acid (6 ml) was added, The obtained mixture was reacted at room temperature for 2 h. After concentration, the crude product was purified by silica gel column chromatography (petroleum ether:ethyl acetate = 3:1) to obtain compound 28D (670 mg, total yield over two steps: 56.30%).

Step 3: 28D (0.47 g, 2.74 mmol) was dissolved in dry N,N-dimethylformamide (6 mL) in a 100 mL single-neck flask. Cesium carbonate (0.89 g, 2.74 mmol) and 4E (750 mg, 2.28 mmol) were slowly added. After the addition, the obtained mixture was reacted under stirring at 80°C for 6 h. After the reaction was completed, the reaction liquid was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: gradient elution using 5%-90% acetonitrile; cycle time: 20 min; retention time: 8 min) to obtain compound 28 (180 mg, yield 16.4%).

LCMS m/z = 482.1 $[M+H]^+$;
$^1H$ NMR (400 MHz, DMSO-$d_6$) δ 8.64 (s, 1H), 7.91 (dd, 1H), 7.84 (d, 1H), 7.73 (dd, 1H), 7.58 (dd, 1H), 7.45 (d, 1H), 4.41 (t, 4H), 4.05 (s, 3H).
$^{19}F$ NMR (377 MHz, DMSO-$d_6$) δ -90.00 (s), -126.12. (s).

**Example 29**

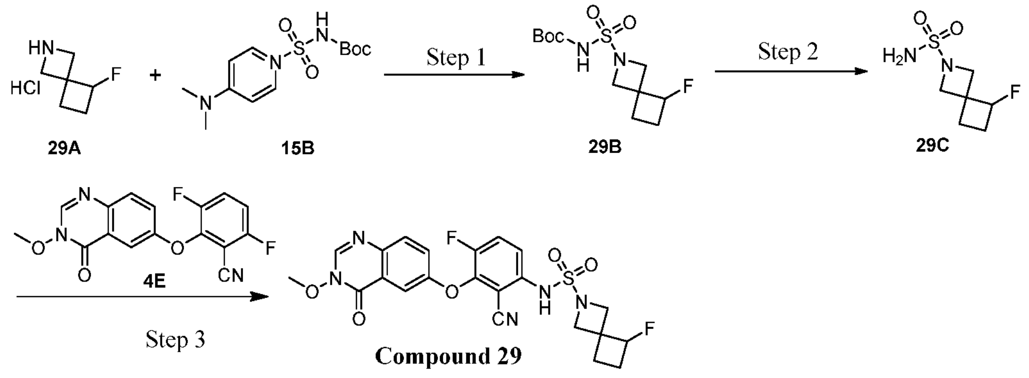


29A 15B 29B 29C 4E Compound 29

Step 1: Compound 29A (0.4 g, 2.64 mmol) was dissolved in acetonitrile (25 mL) in a 100 mL single-neck flask. Then 15B (1.10 g, 3.64 mmol) was added. After the addition, the obtained mixture was reacted at 40°C for 3 h. After the reaction was completed, the reaction liquid was concentrated to obtain compound 29B (1.5 g, crude product), which was directly used in the next reaction.

Step 2: Compound 29B (1.5 g, 5.10 mmol) was dissolved in dichloromethane (30 mL) in a 100 mL single-neck flask. Then trifluoroacetic acid (10 mL) was added. The obtained mixture was reacted at room temperature for 2 h. After the reaction was completed, concentration was performed, and the residue was purified by column chromatography (PE/EA = 1/1) to obtain compound 29C (420 mg, yield 43%).

LCMS m/z = 502.10 $[M+H]^+$;

Step 3: Compound 4E (0.5 g, 1.52 mmol) was dissolved in dry N,N-dimethylformamide (20 mL) in a 100 mL single-neck flask. After the addition of cesium carbonate (1.0 g, 3.07 mmol), the obtained mixture was reacted under stirring at 50°C for 0.5 h. Then a solution of 29C (0.42 g, 2.16 mmol) in N,N-dimethylformamide (5 mL) was added dropwise. After the addition, the reaction liquid was stirred at 100°C for 12 h. After the reaction was completed, filtration was performed. The filtrate was concentrated, and the obtained residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 10 min) to obtain compound 29 (80 mg, yield 10%).

LCMS m/z = 504.10 $[M+H]^+$;

$^1$H NMR (400 MHz, DMSO-$d_6$) δ 10.49 (s, 1H), 8.63 (s, 1H), 7.89 (t, 1H), 7.83 (d, 1H), 7.75-7.68 (m, 1H), 7.57-7.53 (m, 1H), 7.42 (d, 1H), 5.04-4.84 (m, 1H), 4.18 (d, 1H), 4.04 (s, 3H), 3.94 (d, 1H), 3.86-3.79 (m, 2H), 2.19-2.10 (m, 1H), 2.02-1.78 (m, 2H), 1.70-1.62 (m, 1H).

### Example 30

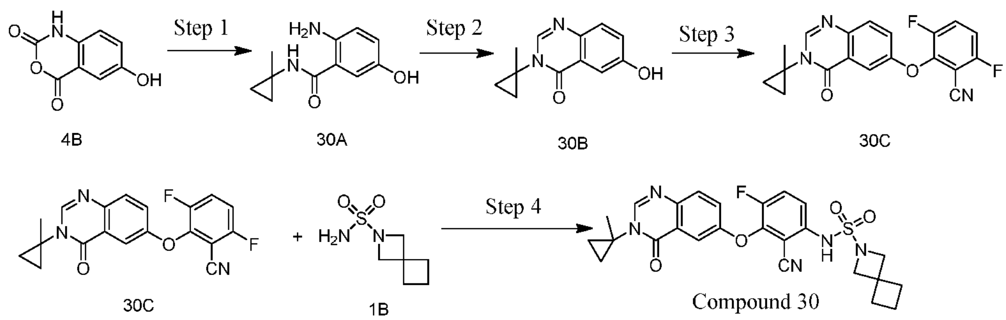


4B 30A 30B 30C 30C 1B Compound 30

Step 1: 4B (4 g, 22.29 mmol) was dissolved in an aqueous solution of sodium hydroxide (2.85 g, 71.33 mmol) (40 mL) in a 250 mL single-neck flask. 1-methylcyclopropylamine hydrochloride (8.39 g, 78.02 mmol) was added. The obtained mixture was reacted under stirring at room temperature overnight. After the reaction was completed, the reaction liquid was concentrated under reduced pressure. The residue was dissolved in a mixed solvent of dichloromethane (200 mL) and methanol (10 mL). The obtained mixture was dried over anhydrous sodium sulfate, and filtered through diatomaceous earth pad. The filtrate was concentrated under reduced pressure. The residue was separated and purified by column chromatography (DCM/MeOH = 20/1) to obtain 30A (4.0 g, yield 87%).

Step 2: 30A (4.0 g, 19.39 mmol) was dissolved in trimethyl orthoformate (32 mL) in a 50 mL single-neck flask. After the addition, the obtained mixture was reacted under stirring at 105°C for 4 h. After the reaction was completed, the reaction liquid was concentrated under reduced pressure. The residue was purified by slurrying with petroleum ether. The solid was filtered and dried to obtain 30B (2.4 g, yield 57%).

LCMS m/z = 217.1 $[M+H]^+$;

Step 3: 30B (2.0 g, 9.25 mmol) was dissolved in dry N,N-dimethylformamide (40 mL) in a 100 mL single-neck flask. Cesium carbonate (3.62 g, 11.1 mmol) was slowly added under an ice bath. After the addition, the obtained mixture was reacted under stirring at room temperature for 0.5 h. A solution of 2,3,6-trifluorobenzonitrile (1.45 g, 9.25 mmol) in N,N-dimethylformamide (10 mL) was slowly added dropwise under an ice bath. After the addition, the obtained mixture was reacted under stirring at room temperature for 1 h. After the reaction was completed, the reaction liquid was poured into ice water (250 mL). The obtained mixture was stirred for 30 min, and filtered. The obtained solid was dried and purified by slurrying with a mixed solvent of petroleum ether (100 mL) and ethyl acetate (10 mL). The solid was filtered and dried to obtain 30C (2.5 g, yield 76%).

LCMS m/z = 354.1 $[M+H]^+$;

Step 4: Compound 1B (240 mg, 1.36 mmol) was dissolved in dry N,N-dimethylformamide (8 mL) in a 100 mL single-neck flask. Cesium carbonate (552 mg, 1.69 mmol) was slowly added under an ice bath. After the addition, the obtained mixture was reacted under stirring at 50°C for 0.5 h. A solution of compound 30C (400 mg, 1.13 mmol) in N,N-dimethylformamide (2 mL) was slowly added dropwise under an ice bath. After the addition, the obtained mixture was reacted under stirring at 100°C for 6 h. After the reaction was completed, the reaction liquid was poured into ice water (50 mL). The obtained mixture was stirred for 30 min, and filtered. The obtained solid was is dried and then separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 30 (240 mg, yield 42%).

LCMS m/z = 510.2 $[M+H]^+$;

$^1H$ NMR (400 MHz, DMSO-$d_6$) δ 10.38 (s, 1H), 8.36 (s, 1H), 7.93 - 7.84 (m, 1H), 7.77 (d, 1H), 7.68 (dd, 1H), 7.54 (dd, 1H), 7.35 (d, 1H), 3.84 (s, 4H), 2.10 (t, 4H), 1.75 (dd, 2H), 1.45 (s, 3H), 1.06 (t, 2H), 0.94 (t, 2H).

$^{19}F$ NMR (377 MHz, DMSO-$d_6$) δ -127.33 (s).

**Example 31**

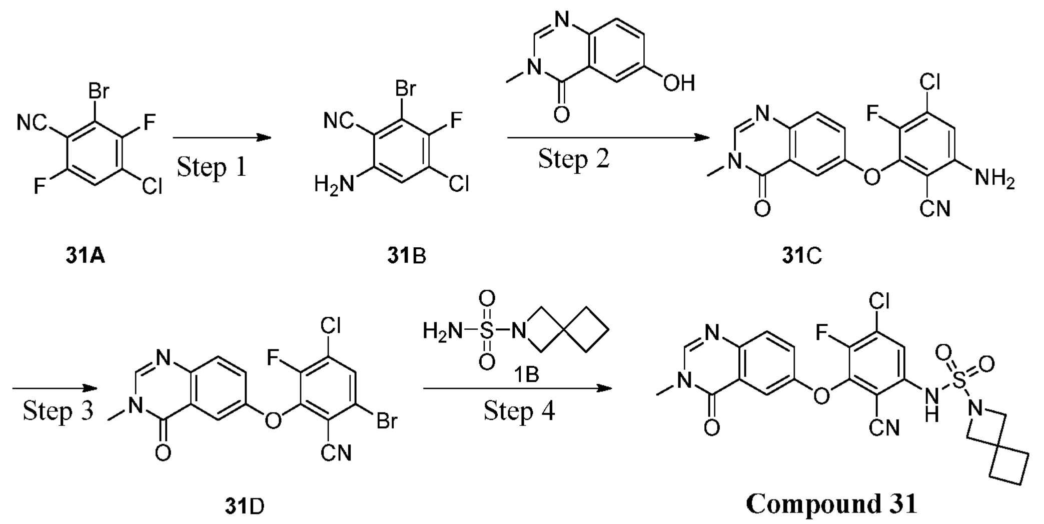


**31A** **31B** **31C** **31D** **Compound 31**

Step 1: 31A (10 g, 39.61 mmol), ammonia (20 mL) and 1,4-dioxane (100 mL) were added in sequence into a 250 mL reaction flask. The obtained mixture was reacted under stirring at 100°C for 24 h. The reaction liquid was extracted (ethyl acetate), washed, and concentrated to obtain a crude product. Column chromatography separation was performed (petroleum ether : ethyl acetate = 1 : 0-0 : 1) to obtain 31B (8.5 g, yield 86.02%).

Step 2: 31B (2 g, 8.02 mmol), 6-hydroxy-3-methylquinazolin-4(3H)-one (CAS: 19181-69-2, 2.31 g, 12.03 mmol), 18-crown-6 (2.12 g, 8.02 mmol), potassium carbonate (3.33 g, 24.06 mmol) and N-methylpyrrolidone (20 mL) were added

in sequence into a 50 mL reaction flask. After the addition, the obtained mixture was reacted under stirring at 110°C for 12 h. The reaction liquid was extracted (ethyl acetate), washed, and concentrated to obtain a crude product. Column chromatography separation was performed (petroleum ether : ethyl acetate = 1 : 0-0 : 1) to obtain 31C (500 mg, yield 17.28%).

Step 3: 31C (0.25 g, 0.73 mmol), copper bromide (0.49 g, 2.19 mmol), tert-butyl nitrite (0.23 g, 2.19 mmol) and acetonitrile (3 mL) were added into a 50 mL reaction flask. After the addition, the obtained mixture was reacted under stirring at room temperature for 1 h. The reaction liquid was extracted (ethyl acetate), washed, and concentrated to obtain a crude product. Column chromatography separation was performed (petroleum ether : ethyl acetate = 1 : 0-0 : 1) to obtain 31D (250 mg, yield 83.81%).

Step 4: 31D (250 mg, 0.61 mmol), 1B (0.22 g, 1.22 mmol), potassium carbonate (100 mg, 0.73 mmol), Xantphos (71 mg, 0.12 mmol), and $Pd_2(dba)_3$ (56 mg, 0.06 mmol) and 1,4-dioxane (5 mL) were added in sequence into a 50 mL reaction flask. The obtained mixture was reacted under stirring at 120°C for 5 h. The reaction liquid was filtered, and the filtrate was concentrated under reduced pressure. The residue was separated and purified by preparative liquid chromatography to obtain compound 31 (88.1 mg, yield 28.6%).

LCMS m/z = 504.8 $[M+H]^+$;

$^1H$ NMR (400 MHz, $CDCl_3$) δ 8.09 (s, 1H), 7.79 (d, 1H), 7.64-7.56 (m, 3H), 3.99 (s, 4H), 3.60 (s, 3H), 2.20 (t, 4H), 1.89-1.81 (m, 2H).

## Example 32

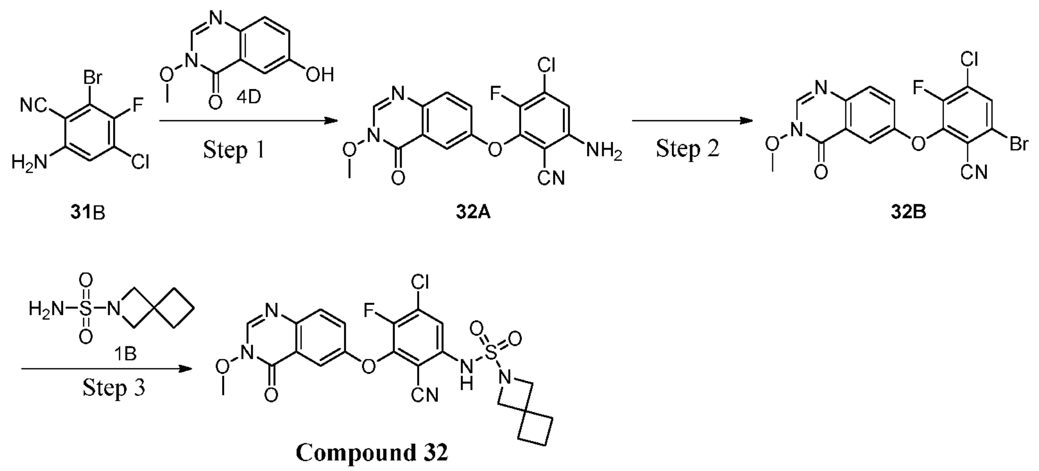


31B 32A 32B

Compound 32

Step 1: 31B (2 g, 8.02 mmol), 4D (2.31 g, 12.03 mmol), 18-crown-6 (2.12 g, 8.02 mmol), potassium carbonate (3.33 g, 24.06 mmol) and N-methylpyrrolidone (20 mL) were added in sequence into a 50 mL reaction flask. After the addition, the obtained mixture was reacted under stirring at 110°C for 12 h. The reaction liquid was extracted (ethyl acetate), washed, and concentrated to obtain a crude product. Column chromatography separation was performed (petroleum ether : ethyl acetate = 1 : 0-0 : 1) to obtain 32A (500 mg, yield 17.28%).

Step 2: 32A (0.13 g, 0.36 mmol), copper bromide (0.24 g, 1.08 mmol), tert-butyl nitrite (0.11 g, 1.08 mmol) and acetonitrile (3 mL) were added into a 50 mL reaction flask. After the addition, the obtained mixture was reacted under stirring at room temperature for 2 h. The reaction liquid was extracted (ethyl acetate), washed, and concentrated to obtain a crude product. Column chromatography separation was performed (petroleum ether : ethyl acetate = 1 : 0-0 : 1) to obtain 32B (90 mg, yield 58.88%).

Step 3: 32B (90 mg, 0.21 mmol), 1B (74 g, 0.42 mmol), potassium carbonate (35 mg, 0.25 mmol), Xantphos (24 mg, 0.04 mmol), and $Pd_2(dba)_3$ (19 mg, 0.02 mmol) and 1,4-dioxane (3 mL) were added in sequence into a 50 mL reaction flask. The obtained mixture was reacted under stirring at 120°C for 5 h. The reaction liquid was filtered, the filtrate was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 32 (18.28 mg, yield 16.74%).

LCMS m/z = 520.9 [M+H]+;

$^1$H NMR (400 MHz, $CDCl_3$) δ 8.33 (s, 1H), 7.84 (d, 1H), 7.66-7.59 (m, 3H), 4.17 (s, 3H), 3.99 (s, 4H), 2.20 (t, 4H), 1.89-1.81 (m, 2H).

**Example 33**

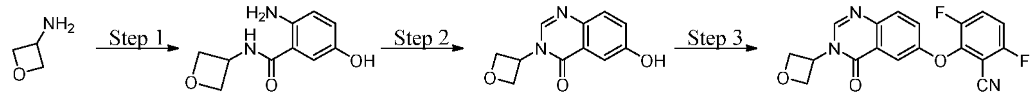


33A 33B 33C 33D

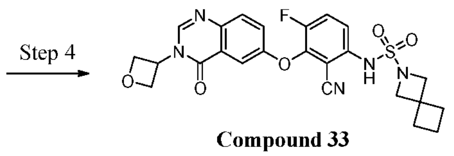


Compound 33

Step 1: 33A (2.92 g, 39.98 mmol) and water (40 mL) were added into a 250 mL single-neck flask. 4B (2.10 g, 11.76 mmol) was added at 0-5°C, The obtained mixture was reacted at room temperature for 4 hrs. After the reaction was completed, the reaction was concentrated and purified by column chromatography (dichloromethane/methanol=10/1) to obtain 33B (2.2 g, yield 89%).

LCMS m/z = 209.1 [M+H]+;

Step 2: 33B (2.0 g, 9.61 mmol) was added to trimethyl orthoformate (20 mL) in a 100 mL single-neck flask. The obtained mixture was reacted at 105°C overnight. After the reaction was completed, the reaction was cooled to room temperature, concentrated and purified by column chromatography (petroleum ether/ethyl acetate = 1/2) to obtain 33C (870 mg, yield 41%).

LCMS m/z = 219.1 [M+H]+;

Step 3: 33C (0.87 g, 3.99 mmol) was dissolved in dry N,N-dimethylformamide (10 mL) in a 25 mL single-neck flask. Cesium carbonate (1.56 g, 4.79 mmol) was slowly added under an ice bath. After the addition, the obtained mixture was reacted under stirring at room temperature for 0.5 h. 2,3,6-trifluorobenzonitrile (0.66 g, 4.19 mmol) was slowly added dropwise under an ice bath. After the addition, the obtained mixture was reacted under stirring at room temperature overnight. After the reaction was completed, ethyl acetate (15 mL) was added to the reaction system to dilute the reaction. Then water (20 mL) was added to quench the reaction, and extraction was performed with ethyl acetate (20 mL × 2). The organic phase was washed with water (20 mL × 2). The organic phase was concentrated and then purified by column chromatography (petroleum ether/ethyl acetate = 1/1) to obtain 33D (0.71 g, yield 50%).

LCMS m/z =356.2 [M+H]+;

$^1$H NMR (400 MHz, DMSO-$d_6$) δ 8.39 (s, 1H), 8.00 - 7.94(m, 1H), 7.82- 7.80(d, 1H), 7.76-7.73 (dd,1H), 7.61-7.55 (m, 2H), 5.55-4.47 (p, 1H), 4.97-4.93 (t, 2H), 4.89-4.85 (t, 2H).

Step 4: 1B (0.22 g, 1.24 mmol) and cesium carbonate (0.44 g, 1.36 mmol) were added to dry N,N-dimethylformamide (10 mL) in a 25 mL single-neck flask. The obtained mixture was reacted at 50°C for 30 min. A solution of 33D (0.40 g, 1.13 mmol) in N,N-dimethylformamide (2 mL) was added dropwise at 50°C. After the addition, the obtained mixture was reacted under stirring at 80°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain 220 mg of solid, which was purified by column chromatography (petroleum ether/ethyl acetate = 1/1) to obtain compound 33 (160 mg, yield 27%).

LCMS m/z = 512.6 [M+H]+;

$^1$H NMR (400 MHz, DMSO-$d_6$) δ 10.37 (s, 1H), 8.38 (s, 1H), 7.89 - 7.80 (m, 2H), 7.72-7.69 (dd,1H), 7.55-7.52 (dd, 1H), 7.37-7.36 (d,1H), 5.53-5.45 (p, 1H), 4.96-4.92 (t, 2H), 4.88-4.84 (t, 2H), 3.83 (s, 4H), 2.12-2.08 (t, 4H), 1.78 - 1.70 (m, 2H).

$^{19}F$ NMR (377 MHz, DMSO-$d_6$) δ -127.30 (s).

**Example 34**

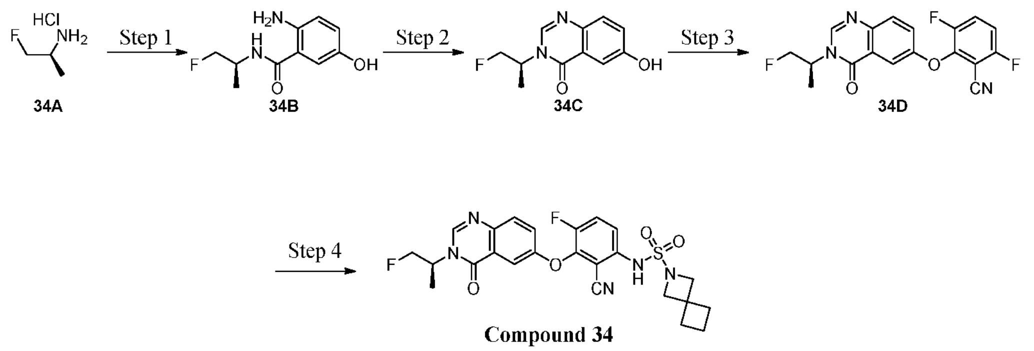


34A 34B 34C 34D

Compound 34

Step 1: 34A (2.00 g, 17.57 mmol) and water (20 mL) were added into a 250 mL single-neck flask. A solution of sodium hydroxide (0.62 g, 15.62 mmol) in water (20 mL) was slowly added dropwise at 0-5°C. The obtained mixture was reacted at 0-5°C for 10 min. Then 4B (0.87 g, 4.88 mmol) was added at 0-5°C and the obtained mixture was reacted at room temperature for 4 hrs. After the reaction was completed, the reaction was concentrated and then purified by column chromatography (dichloromethane/methanol = 10/1) to obtain 34B (0.83 g, yield 80%).

LCMS m/z = 213.1 [M+H]+;
Step 2: 34B (0.72 g, 3.39 mmol) was added to trimethyl orthoformate (10 mL) in a 100 mL single-neck flask. The obtained mixture was reacted at 105°C overnight. After the reaction was completed, the reaction was cooled to room temperature, concentrated and purified by column chromatography (petroleum ether/ethyl acetate = 1/2) to obtain 34C (280 mg, yield 37%).

LCMS m/z = 223.2 [M+H]+;
Step 3: 34C (0.28 g, 1.26 mmol) was dissolved in dry N,N-dimethylformamide (4 mL) in a 25 mL single-neck flask. Cesium carbonate (0.49 g, 1.51 mmol) was slowly added under an ice bath. After the addition, the obtained mixture was reacted under stirring at room temperature for 0.5 h. 2,3,6-trifluorobenzonitrile (0.22 g, 1.39 mmol) was slowly added dropwise under an ice bath. After the addition, the obtained mixture was reacted under stirring at room temperature overnight. After the reaction was completed, ethyl acetate (10 mL) was added to the reaction system to dilute the reaction. Then water (10 mL) was added to quench the reaction, and extraction was performed with ethyl acetate (10 mL × 2). The organic phase was washed with water (10 mL × 2). The organic phase was concentrated and then purified by column chromatography (petroleum ether/ethyl acetate = 3/1) to obtain 34D (0.25 g, yield 55%).

LCMS m/z = 360.1 [M+H]+;
$^1H$ NMR (400 MHz, DMSO-$d_6$) δ 8.44 (s, 1H), 8.01 - 7.95 (m, 1H), 7.81 - 7.73 (m, 2H), 7.62 - 7.56 (m, 2H), 5.17 - 5.04 (m, 1H), 4.91-4.86 (dd, 0.5H), 4.79 - 4.75 (m, 1H), 4.67-4.63 (dd, 0.5H), 1.48-1.45 (dd, 3H).

Step 4: 1B (0.13 g, 0.73 mmol) and cesium carbonate (0.27 g, 0.84 mmol) were added to dry N,N-dimethylformamide (6 mL) in a 25 mL single-neck flask. The obtained mixture was reacted at 50°C for 30 min. A solution of 34D (0.25 g, 0.70 mmol) in N,N-dimethylformamide (2 mL) was added dropwise at 50°C. After the addition, the obtained mixture was reacted under stirring at 80°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 34 (110 mg, yield 30%).

LCMS m/z = 516.2 [M+H]+;
$^1H$ NMR (400 MHz, DMSO-$d_6$) δ 10.37 (s, 1H), 8.42 (s, 1H), 7.90-7.85 (dd, 1H), 7.82-7.79 (d, 1H), 7.73-7.70 (dd, 1H), 7.56-7.52 (dd,1H), 7.38-7.37 (d, 1H), 5.13-5.04 (m, 1H), 4.90-4.85 (dd, 0.5H), 4.78 - 4.73 (m, 1H), 4.65-4.62 (dd, 0.5H), 3.84 (s, 4H), 2.11-2.07 (t, 4H), 1.77 - 1.70 (m, 2H), 1.45 - 1.44 (m, 3H).
$^{19}F$ NMR (377 MHz, DMSO-$d_6$) δ -72.80 (s), -127.34 (s).

**Example 35**

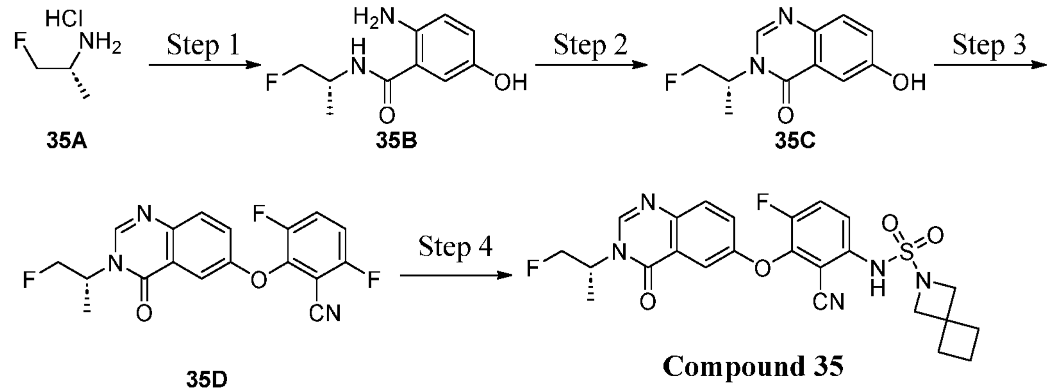


35A 35B 35C

35D Compound 35

Step 1: 35A (2.50 g, 22.01 mmol) and water (25 mL) were added into a 250 mL single-neck flask. A solution of sodium hydroxide (0.79 g, 19.78 mmol) in water (25 mL) was slowly added dropwise at 0-5°C. The obtained mixture was reacted at 0-5°C for 10 min. Then 4B (1.10 g, 6.18 mmol) was added at 0-5°C and the obtained mixture was reacted at room temperature for 4 hrs. After the reaction was completed, the reaction was concentrated and then purified by column chromatography (dichloromethane/methanol = 10/1) to obtain 35B (0.65 g, yield 49%).

LCMS m/z = 213.1 $[M+H]^+$;

Step 2: 35B (0.72 g, 3.39 mmol) was added to triethyl orthoformate (10 mL) in a 100 mL single-neck flask. The obtained mixture was reacted under microwave at 180°C for 2.5 hrs. After the reaction was completed, the reaction was cooled to room temperature, and concentrated to obtain crude product 35C, which was directly used in the next reaction.

LCMS m/z =223.2 $[M+H]^+$;

Step 3: Crude product 35C was dissolved in dry N,N-dimethylformamide (10 mL) in a 25 mL single-neck flask. Cesium carbonate (1.1 g, 3.41 mmol) was slowly added under an ice bath. After the addition, the obtained mixture was reacted under stirring at room temperature for 0.5 h. 2,3,6-trifluorobenzonitrile (0.49 g, 3.12 mmol) was slowly added dropwise under an ice bath. After the addition, the obtained mixture was reacted under stirring at room temperature overnight. After the reaction was completed, ethyl acetate (20 mL) was added to the reaction system to dilute the reaction. Then water (20 mL) was added to quench the reaction, and extraction was performed with ethyl acetate (20 mL × 2). The organic phase was washed with water (20 mL × 2). The organic phase was concentrated and then purified by column chromatography (petroleum ether/ethyl acetate = 3/1) to obtain 35D (0.16 g, yield 15%).

LCMS m/z = 360.1 $[M+H]^+$;

Step 4: 1B (83.27 mg, 0.47 mmol) and cesium carbonate (0.17 g, 0.54 mmol) were added to dry N,N-dimethylformamide (4 mL) in a 25 mL single-neck flask. The obtained mixture was reacted at 50°C for 30 min. A solution of 35D (0.16 g, 0.45 mmol) in N,N-dimethylformamide (1 mL) was added dropwise at 50°C. After the addition, the obtained mixture was reacted under stirring at 80°C for 3 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 35 (105 mg, yield 45%).

LCMS m/z = 516.6 $[M+H]^+$;
$^1$H NMR (400 MHz, DMSO-$d_6$) δ 10.37 (s, 1H), 8.42 (s, 1H), 7.90 - 7.79 (m, 2H), 7.73-7.70 (dd, 1H), 7.56-7.52 (dd, 1H), 7.38-7.37 (d, 1H), 5.13 - 5.04 (m, 1H), 4.89-4.85 (dd, 0.5H), 4.78 - 4.73 (m, 1H), 4.65-4.62 (dd, 0.5H), 3.83 (s, 4H), 2.11-2.07 (t, 4H), 1.77 - 1.70 (m, 2H), 1.46-1.44 (d, 3H).
$^{19}$F NMR (377 MHz, DMSO-$d_6$) δ -74.66 (s), -129.42 (s).

**Example 36 and example 37**

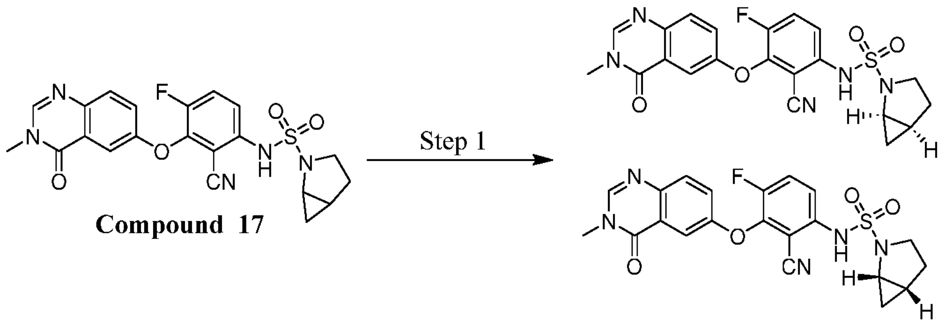


Compound 17

Compound 36 Compound 37

Compound 17 (300 mg) was subjected to chiral SFC resolution to obtain P1 (retention time: 0.588 min, set as compound 36) and P2 (retention time: 0.694 min, set as compound 37). Resolution method: instrument name: Waters 150 Prep-SFC F; chromatographic column: Chiralcel AD-Column; mobile phase: A for $CO_2$ and B for 0.1%$NH_3$•$H_2O$ in MEOH and ACN; gradient: isocratic elution, mobile phase B: 35%; flow rate: 120 mL/min. Sample preparation: the compound was dissolved in acetonitrile at a concentration of 10 mg/mL; injection: 2.0 mL/injection; treatment: after the separation, the obtained product was concentrated by a rotary evaporator at 35°C, and then the solvent was dried by a lyophilizer at -80°C to obtain compound 36 (86 mg, 29%) and compound 37 (74 mg, 25%).

Compound 36:

LCMS m/z = 456.1 $[M+H]^+$;
$^1$H NMR (400 MHz, DMSO-$d_6$) δ 10.40 (s, 1H), 8.35 (s, 1H), 7.84 (t, 1H), 7.78 (d, 1H), 7.68 (dd, 1H), 7.62 (dd, 1H), 7.37 (d, 1H), 3.47 (s, 3H), 3.40 - 3.34 (m, 1H), 3.17 (dd, 1H), 2.93 (dd, 1H), 2.10 - 2.01 (m, 1H), 1.96 (dd, 1H), 1.60 (dt, 1H), 0.81 - 0.75 (m, 1H), 0.50 (dd, 1H).
$^{19}$F NMR (377 MHz, DMSO-$d_6$) δ -128.22 (s).

Compound 37:

LCMS m/z = 456.1 $[M+H]^+$;
$^1$H NMR (400 MHz, DMSO-$d_6$) δ 10.42 (s, 1H), 8.36 (s, 1H), 7.85 (t, 1H), 7.78 (d, 1H), 7.68 (dd, 1H), 7.62 (dd, 1H), 7.38 (d, 1H), 3.47 (s, 3H), 3.37 (s, 1H), 3.17 (s, 1H), 2.93 (dd, 1H), 2.09 - 2.00 (m, 1H), 1.98 (d, 1H), 1.60 (s, 1H), 0.78 (s, 1H), 0.50 (d, 1H).
$^{19}$F NMR (377 MHz, DMSO-$d_6$) δ -128.22 (s).

**Example 38**

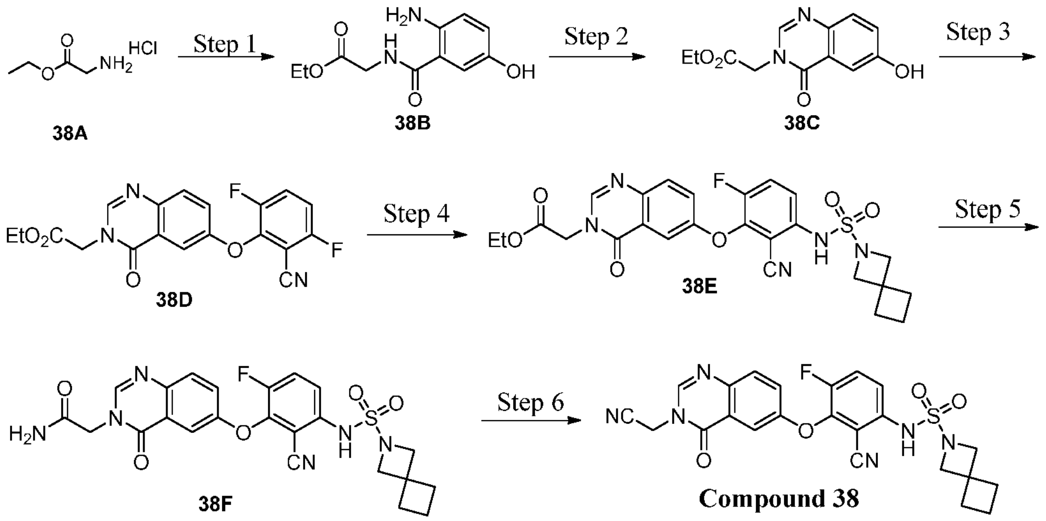


38A 38B 38C 38D 38E 38F Compound 38

Step 1: 38A (16.66 g, 119.37 mmol) and water (120 mL) were added into a 1000 mL single-neck flask. A solution of sodium hydroxide (4.29 g, 107.30 mmol) in water (120 mL) was slowly added dropwise at 0-5°C. The obtained mixture was reacted at 0-5°C for 10 min. Then 6-hydroxy-2H-benzo[d][1,3]oxazine-2,4(1H)-dione (6.00 g, 33.53 mmol) was added at 0-5°C, The obtained mixture was reacted at room temperature for 4 hrs. After the reaction was completed, the reaction was concentrated and then purified by column chromatography (dichloromethane/methanol = 10/1) to obtain 38B (3.0 g, yield 37.56%).

LCMS m/z = 239.1 $[M+H]^+$;
Step 2: 38B (2.7 g, 11.33 mmol) was added to dry N,N-dimethylformamide (30 mL) in a 100 mL single-neck flask. Triethyl orthoformate (30 mL) was added at room temperature. The obtained mixture was reacted at 150°C for 4 hrs. After the reaction was completed, the reaction was cooled to room temperature, and concentrated to obtain crude product 38C, which was directly used in the next reaction.

LCMS m/z = 249.1 $[M+H]^+$;
Step 3: 38C was dissolved in dry N,N-dimethylformamide (30 mL) in a 25 mL single-neck flask. Cesium carbonate (4.11 g, 13.54 mmol) was slowly added under an ice bath. After the addition, the obtained mixture was reacted under stirring at room temperature for 0.5 h. 2,3,6-trifluorobenzonitrile (1.95 g, 12.41 mmol) was slowly added dropwise under an ice bath. After the addition, the obtained mixture was reacted under stirring at room temperature overnight. After the reaction was completed, ethyl acetate (100 mL) was added to the reaction system to dilute the reaction. Then water (100 mL) was added to quench the reaction, and extraction was performed with ethyl acetate (100 mL × 2). The organic phase was washed with water (100 mL × 2), concentrated and then purified by column chromatography (petroleum ether/ethyl acetate = 3/1) to obtain 38D (2.4 g, yield 55%).

LCMS m/z = 386.2 $[M+H]^+$;
Step 4: 1B (1.32 g, 7.48 mmol) and cesium carbonate (3.04 g, 9.37 mmol) were added to dry N,N-dimethylformamide (20 mL) in a 25 mL single-neck flask. The obtained mixture was reacted at 50°C for 30 min. A solution of 38D (2.40 g, 6.23 mmol) in N,N-dimethylformamide (10 mL) was added dropwise at 50°C. After the addition, the obtained mixture was reacted under stirring at 80°C overnight. After the reaction was completed, the reaction was filtered, and the organic phase of the filtrate was concentrated and then purified by column chromatography (dichloromethane/petroleum ether = 10/1) to obtain compound 38E (2.1 g, yield 62%).

LCMS m/z = 542.4 $[M+H]^+$;
Step 5: 38E (2.00 g, 3.69 mmol) was added to methanol (5 mL) in a 25 mL single-neck flask. A solution of ammonia in methanol (7.0 M, 40mL) was added dropwise at 0-5°C. After the addition, the obtained mixture was reacted under stirring at room temperature overnight. After the reaction was completed, the reaction liquid was concentrated to obtain compound 38F (crude product), which was directly used in the next reaction.

LCMS m/z = 513.1 $[M+H]^+$;
Step 6: 38F (0.21 g, 0.41 mmol) was added to dry dichloromethane (6 mL) in a 25 mL single-neck flask. Burgess reagent (195.41 g, 0.82 mmol) was added at 0-5°C. After the addition, the obtained mixture was reacted under stirring at room temperature overnight. The reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 38 (35 mg, yield 17%).

LCMS m/z = 495.1 $[M+H]^+$;
$^1H$ NMR (400 MHz, DMSO-$d_6$) δ 10.36 (s, 1H), 8.40 (s, 1H), 7.90 - 7.82 (m, 2H), 7.75-7.73 (dd, 1H), 7.56-7.52 (dd, 1H), 7.44-7.43 (d, J = 2.9 Hz, 1H), 5.08 (s, 2H), 3.84 (s, 4H), 2.11-2.08 (t, 4H), 1.78 - 1.70 (m, 2H).
$^{19}F$ NMR (377 MHz, DMSO-$d_6$) δ -127.33 (s).

**Example 39**

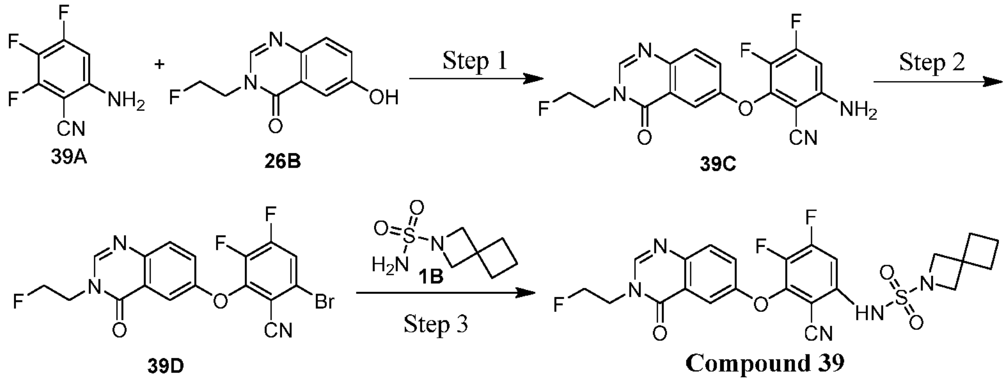


Step 1: 26B (627 mg, 3.01 mmol), 39A (701 mg, 3.01 mmol), and cesium carbonate (1.47 g, 4.51 mmol) were dissolved in dry N,N-dimethylformamide (25 mL) in a 100 mL single-neck flask. After the addition, the obtained mixture was reacted under stirring at room temperature for 2 h. After the reaction was completed, the reaction liquid was diluted with ethyl acetate (200 mL). The organic phase was washed twice with water (60 mL), washed twice with saturated saline (50 mL), dried over anhydrous sodium sulfate and filtered. The filtrate was concentrated and purified by column chromatography (PE : EA = 1 : 1) to obtain compound 39C (800 mg, yield: 74%).

Step 2: Compound 39C (800 mg, 2.22 mmol), copper bromide (992 mg, 4.44 mmol), and acetonitrile (40 ml) were added into a single-neck flask. Tert-butyl nitrite (458 mg, 4.44 mmol) was slowly added dropwise under an ice bath. After the addition, the obtained mixture was reacted at room temperature for 1 h. After the reaction was completed, the obtained mixture was filtered through diatomaceous earth pad. After the filtrate was concentrated, the residue was separated by column chromatography (petroleum ether: ethyl acetate (v/v) = 1 : 0 - 0 : 1) to obtain compound 39D (814 mg, yield: 86%).

Step 3: 39D (550 mg, 1.30 mmol), 39E (275 mg, 1.56 mmol), 2-di-tert-butylphosphino-2',4',6'-triisopropylbiphenyl (110 mg, 0.26 mmol), allylpalladium chloride (71 mg, 0.20 mmol), and potassium carbonate (629 mg, 4.55 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction liquid was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 30%-70% acetonitrile; cycle time: 15 min) to obtain compound 39 (210 mg, yield: 31%).

LCMS m/z = 520.50 $[M+H]^+$;
$^1H$ NMR (400 MHz, DMSO-$d_6$) δ 8.34 (s, 1H), 7.80 (d, 1H), 7.75 (dd, 1H), 7.59 (d, 1H), 7.53 (dd, 1H), 4.72 (dt, 2H), 4.32 (dt, 2H), 3.85 (s, 4H), 2.10 (t, 4H), 1.79 - 1.68 (m, 2H).
$^{19}F$ NMR (376 MHz, DMSO-$d_6$) δ -121.46 (s), -150.87 (s), -222.31 (s).

Example 40 and example 41:

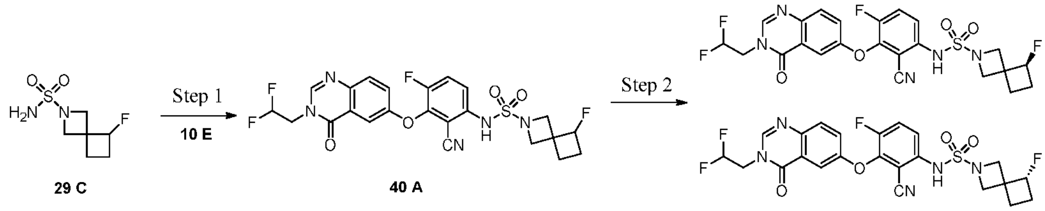


29 C

40 A

Compound 40 and Compound 41

Step 1: 29C (0.400 g, 2.06 mmol) and cesium carbonate (1.01 g, 3.09 mmol) were added to dry N,N-dimethylformamide (10 mL) in a 25 mL single-neck flask. The obtained mixture was reacted at 50°C for 30 min. A solution of 10E (0.748 g, 2.06 mmol) in N,N-dimethylformamide (2 mL) was added dropwise at 50°C. After the addition, the obtained mixture was reacted under stirring at 100°C overnight. After the reaction was completed, the reaction liquid was filtered. The filter cake use N,N-dimethylformamide (4 mL). The filtrate was concentrated and then dissolved in water (15 mL) and ethyl acetate (10 mL). Liquid separation was performed. After the aqueous phase was extracted once with ethyl acetate (10 mL), the pH was adjusted to about 7 with saturated ammonium chloride solution. Then the aqueous phase was extracted with dichloromethane (20 mL × 3). The organic phase was dried over anhydrous sodium sulfate and then concentrated to obtain 40A crude product. The crude product was directly used in the next reaction.

LCMS m/z = 538.5 $[M+H]^+$;

Step 2: The crude product 40A was subjected to chiral SFC resolution to obtain P1 (retention time: 14.73 min, set as compound 40) and P2 (retention time: 24.02 min, set as compound 41). Resolution method: instrument name: Waters 150 SFC; chromatographic column: AD; mobile phase: A for $CO_2$ and B for IPA+MeOH (0.05%$NH_3 \cdot H_2O$); flow rate: 42 mL/min; column pressure: 100 bar; column temperature: 25°C; absorption wavelength: 220 nm cycle time: 40 min). Sample preparation: the compound was dissolved in methanol at a concentration of 40 mg/mL; injection: 5 mL/injection; treatment: after the separation, the obtained product was concentrated by a rotary evaporator at 35°C, and then the solvent was dried by a lyophilizer at -80°C to obtain P1 and P2.

Compound 40: (P1: 120 mg, 11%), $^1$H NMR (400 MHz, DMSO-$d_6$) δ 10.45 (s, 1H), 8.33 (s, 1H), 7.83 - 7.80 (m, 2H), 7.72-7.69 (dd, 1H), 7.54-7.50 (dd, 1H), 7.43-7.42 (d, 1H), 6.51 - 6.22 (m, 1H), 5.02-4.99 (dt, 1H), 4.51-4.43 (td, 2H), 4.16-4.14 (d, 1H), 3.92 - 3.77 (m, 3H), 2.18-2.07 (m, 1H), 2.00 - 1.79 (m, 2H), 1.69-1.62 (m, 1H).

$^{19}$F NMR (376 MHz, DMSO-$d_6$) δ -120.44 (s), -171.59 (s), -216.41 (s).

Compound 41: (P2: 140mg, 13%), $^1$H NMR (400 MHz, DMSO-$d_6$) δ 10.45 (s, 1H), 8.34 (s, 1H), 7.88-7.80 (m, 2H), 7.73-7.70 (dd, 1H), 7.54-7.51 (dd, 1H), 7.43-7.42 (d, 1H), 6.51 - 6.22 (m, 1H), 5.03-4.99 (dt, 1H), 4.51-4.43 (td, 2H), 4.17-4.15 (d, 1H), 3.93 - 3.78 (m, 3H), 2.18 - 2.10 (m, 1H), 2.00 - 1.79 (m, 2H), 1.69-1.62 (m, 1H).

$^{19}$F NMR (376 MHz, DMSO-$d_6$) δ -120.44 (s), -127.17 (s), -171.59 (s).

**Example 42**

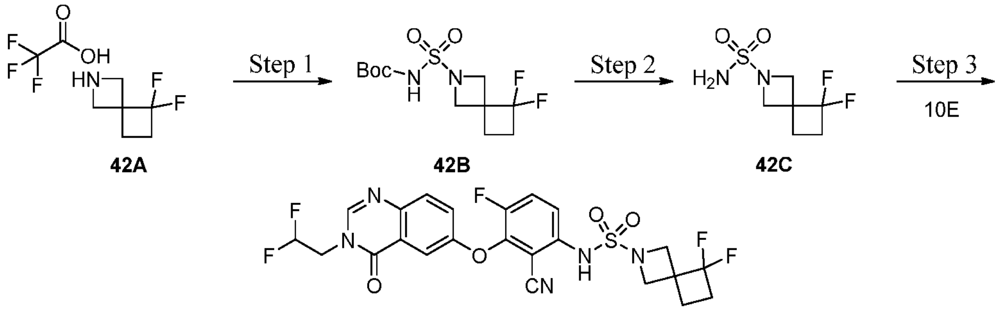


42A

42B

42C

Compound 42

Step 1: 42A (420 mg, 1.70 mmol) and acetonitrile (5 mL) were added into a 25 mL single-neck flask. Triethylamine (0.430 g, 4.25 mmol) and (tert-butoxycarbonyl)((4-(dimethylimino)pyridin-1(4H)yl)sulfonyl)amide (0.615 g, 2.04 mmol) were slowly added dropwise at 0-5°C. The obtained mixture was reacted at room temperature overnight. After the reaction was completed, the reaction was concentrated to obtain crude product 42B, which was directly used in the next reaction.

LCMS m/z = 257.1 $[M+H]^+$;

Step 2: The crude product 42B was dissolved in dichloromethane (10 mL) in a 25 mL single-neck flask. Trifluoroacetic acid (5 mL) was slowly added dropwise at 0-5°C. After the addition, the obtained mixture was reacted at room temperature

overnight. After the reaction was completed, the reaction was concentrated, and the organic phase was concentrated and then purified by column chromatography (petroleum ether/ethyl acetate = 2/1) to obtain 42C (0.250 g, yield 70%).

LCMS m/z = 213.2 $[M+H]^+$;

Step 3: 42C (0.250 g, 1.18 mmol) and cesium carbonate (0.577 g, 1.77 mmol) were added to dry N,N-dimethylformamide (5 mL) in a 25 mL single-neck flask. The obtained mixture was reacted at 50°C for 30 min. A solution of 10E (0.429 g, 1.18 mmol) in N,N-dimethylformamide (2 mL) was added dropwise at 50°C. After the addition, the obtained mixture was reacted under stirring at 80°C overnight. After the reaction was completed, the reaction liquid was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 42 (110 mg, yield 17%).

LCMS m/z = 556.0 $[M+H]^+$.

$^1$H NMR (400 MHz, DMSO-$d_6$) δ 10.57 (s, 1H), 8.34 (s, 1H), 7.92 - 7.87 (m, 1H), 7.83-7.80 (d, 1H), 7.73-7.70 (dd, 1H), 7.55-7.52 (dd, 1H), 7.44 (d, 1H), 6.51-6.22 (m, 1H), 4.52-4.43 (m, 2H), 4.08-4.06 (d, 2H), 3.91-3.88 (d, 2H), 2.47 - 2.42 (m, 2H), 2.04-2.00 (m , 2H).

$^{19}$F NMR (376 MHz, DMSO-$d_6$) δ -98.98 (s), -120.45 (s), -126.75 (s).

**Example 43**

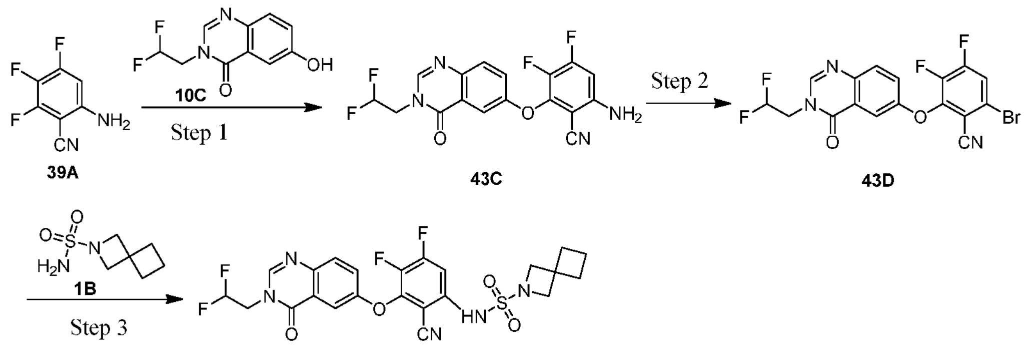


39A

43C

43D

Compound 43

Step 1: 10C (700 mg, 3.09 mmol), 39A (720 mg, 3.09 mmol), and cesium carbonate (1.53 g, 4.63 mmol) were dissolved in dry N,N-dimethylformamide (25 mL) in a 100 mL single-neck flask. After the addition, the obtained mixture was reacted under stirring at room temperature for 2 h. After the reaction was completed, the reaction liquid was diluted with ethyl acetate (200 mL). The organic phase was washed twice with water (60 mL), washed twice with saturated saline (50 mL), dried over anhydrous sodium sulfate and filtered. The filtrate was concentrated and purified by column chromatography (PE : EA = 1 : 1) to obtain compound 43C (830 mg, yield: 71%).

Step 2: Compound 43C (830 mg, 2.22 mmol), copper bromide (992 mg, 4.44 mmol), and acetonitrile (40 ml) were added into a single-neck flask. Tert-butyl nitrite (458 mg, 4.44 mmol) was slowly added dropwise under an ice bath. After the addition, the obtained mixture was reacted at room temperature for 1 h. After the reaction was completed, the obtained mixture was filtered through diatomaceous earth pad. After the filtrate was concentrated, the residue was separated by column chromatography (petroleum ether: ethyl acetate (v/v) = 1 : 0 - 0 : 1) to obtain compound 43D (800 mg, yield: 82%).

Step 3: 43D (800 mg, 1.81 mmol), 1B (400 mg, 2.26 mmol), 2-di-tert-butylphosphino-2',4',6'-triisopropylbiphenyl (154 mg, 0.36 mmol), allylpalladium chloride (99 mg, 0.27 mmol), and potassium carbonate (625 mg, 4.25 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction liquid was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 30%-70% acetonitrile; cycle time: 15 min) to obtain compound 43 (56 mg, yield: 6%).

LCMS m/z = 538.1 $[M+H]^+$;

$^1$H NMR (400 MHz, DMSO-$d_6$) δ 8.35 (s, 1H), 7.81 (d, 1H), 7.77 (dd, 1H), 7.59 (d, 1H), 7.52 (dd, 1H), 6.52 - 6.23 (m, 1H),

4.49 (td, 2H), 3.83 (s, 4H), 2.08 - 2.11 (m, 4H), 1.77 - 1.70 (m, 2H).

**Example 44**

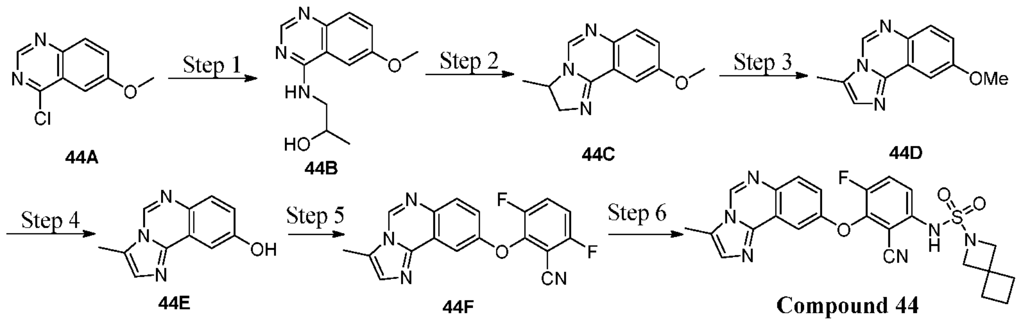


Step 1: 44A (4.5 g, 23.13 mmol) and isopropyl alcohol (50 mL) were added into a 250 mL single-neck flask. Then triethylamine (4.68 g, 46.26 mmol) and isopropanolamine (2.08 g, 27.76 mmol) were added. The obtained mixture was reacted at 80°C for 1 hr. After the reaction was completed, the reaction was concentrated, slurried with ethyl acetate (50 mL), and filtered, and the filter cake was washed with petroleum ether (50 mL) to obtain 44B (5.30 g, yield 98%).

LCMS m/z = 234.3 $[M+H]^+$;

Step 2: Phosphorus oxychloride (60 mL) was added into a 250 mL single-neck flask. 44B (5.3 g, 22.72 mmol) was slowly added in portions at 0-5°C. After the addition, the obtained mixture was reacted at 110°C for 5 hrs. After the reaction was completed, the reaction liquid was concentrated, then water (100 mL) was slowly added to quench the reaction at 0-5°C, and extraction was performed with ethyl acetate (100 mL $\times$ 2). The organic phase was concentrated and then purified by column chromatography (petroleum ether/ethyl acetate = 1/1) to obtain 44C (3.6 g, yield 73%).

LCMS m/z = 216.2 $[M+H]^+$;

Step 3: 4C (2.80 g, 13.01 mmol) and acetonitrile (30 mL) were added into a 100 mL single-neck flask. Then alumina (2.65 g, 25.99 mmol) and potassium permanganate (3.08 g, 19.49 mmol) were added. After the addition, the obtained mixture was reacted under stirring at room temperature overnight. After the reaction was completed, the reaction liquid was filtered, and the filtrate was concentrated and then purified by column chromatography (petroleum ether/ethyl acetate = 1/2) to obtain 44D (0.88 g, yield 31%).

LCMS m/z = 214.1 $[M+H]^+$;
$^1$H NMR (400 MHz, DMSO-d6) $\delta$ 9.01 (s, 1H), 7.86-7.84 (d, 1H), 7.74-7.73 (d, 1H), 7.35 (s, 1H), 7.32-7.29 (dd, 1H), 3.94 (s, 3H), 2.58 (s, 3H).

Step 4: 4D (0.88 g, 4.13 mmol) and N,N-dimethylformamide (10 mL) were added into a 50 mL single-neck flask. Then sodium thioethylate (1.04 g, 12.35 mmol) was added. After the addition, nitrogen protection was performed. The obtained mixture was reacted under stirring at 130°C overnight. After the reaction was completed, the reaction liquid was concentrated. Dilute hydrochloric acid was added to adjust the pH to acidic. After concentration, a residue was obtained, The residue was purified by column chromatography (dichloromethane/methanol = 20/1) to obtain 44E (0.68 g, yield 82%).

LCMS m/z =200.1 $[M+H]^+$;

Step 5: 44E (0.30 g, 1.51 mmol) was dissolved in dry N,N-dimethylformamide (8 mL) in a 25 mL single-neck flask. Cesium carbonate (0.74 g, 2.26 mmol) was slowly added under an ice bath. After the addition, the obtained mixture was reacted under stirring at room temperature for 0.5 h. 2,3,6-trifluorobenzonitrile (0.28 g, 1.81 mmol) was slowly added dropwise under an ice bath. After the addition, the obtained mixture was reacted under stirring at room temperature overnight. After the reaction was completed, the reaction liquid was filtered, and the filtrate was concentrated and then purified by column chromatography (petroleum ether/ethyl acetate = 1/1) to obtain 44F (0.45 g, yield 88%).

LCMS m/z =337.0 $[M+H]^+$;

Step 6: 1B (0.21 g, 1.16 mmol) and cesium carbonate (0.47 g, 1.46 mmol) were added to dry N,N-dimethylformamide (5 mL) in a 25 mL single-neck flask. The obtained mixture was reacted at 50°C for 30 min. A solution of 44F (0.36 g, 0.97 mmol) in N,N-dimethylformamide (1 mL) was added dropwise at 50°C. After the addition, the obtained mixture was reacted under stirring at 80°C for 4 hrs. After the reaction was completed, the reaction liquid was filtered. The filter cake was washed with N,N-dimethylformamide (2 mL). The filtrate was concentrated, and then ethyl acetate (15 mL) and water (15 mL) were added for dissolution. Liquid separation was performed, and the aqueous phase was adjusted to neutral pH with saturated ammonium chloride, and extracted with dichloromethane (20 mL $\times$ 3), The organic phases were combined,

dried over anhydrous sodium sulfate, and then purified by column chromatography (petroleum ether/ethyl acetate = 1/1) to obtain compound 44 (61 mg, yield 12%).

LCMS m/z = 493.5 [M+H]$^+$;
$^1$H NMR (400 MHz, DMSO-$d_6$) δ 10.44 (s, 1H), 9.19 (s, 1H), 8.00-7.98 (d, 1H), 7.93-7.88 (t, 1H), 7.81 (s, 1H), 7.59-7.55 (m, 2H), 7.52-7.51 (d, 1H), 3.84 (s, 4H), 2.36 (s, 3H), 2.10-2.06 (t, 4H), 1.73 - 1.65 (m, 2H).
$^{19}$F NMR (376 MHz, DMSO-$d_6$) δ -127.37 (s).

**Example 45**

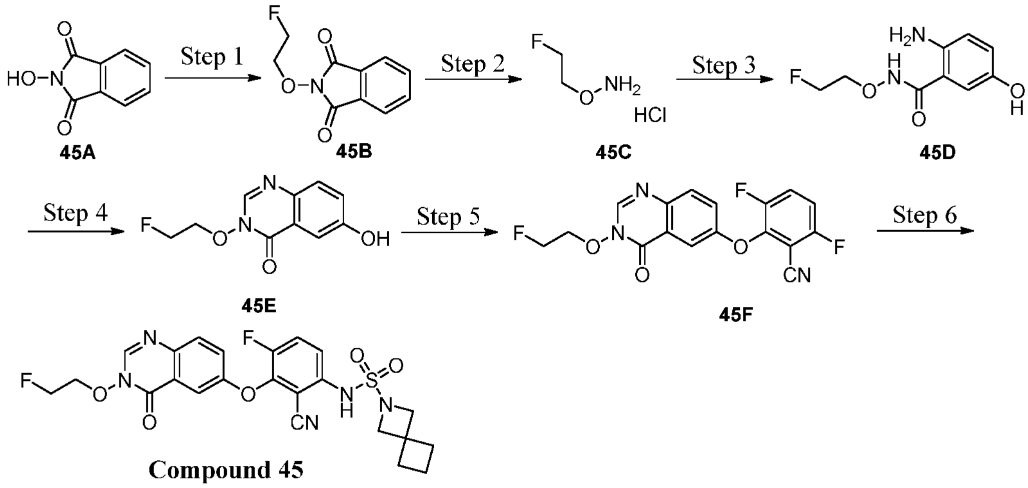


45A 45B 45C 45D 45E 45F

Compound 45

Step 1: 45A (10 g, 61.33 mmol) and N,N-dimethylformamide (100 mL) were added into a 250 mL single-neck flask. N,N-diisopropylethylamine (15.85 g, 122.64 mmol) and 1-fluoro-2-iodoethane (12.80 g, 73.63 mmol) were slowly added at 0-5°C. The obtained mixture was reacted at 80°C overnight. After the reaction was completed, the reaction liquid was added dropwise to water (500 mL), and a large amount of solids were precipitated. The obtained mixture was reacted under stirring for 30 min, and filtered. The filter cake was washed with water (100 mL) and petroleum ether (50 mL), and then concentrated to afford 45B (10.0 g, yield 78%).

LCMS m/z = 210.2 [M+H]$^+$;
Step 2: 45B (10 g, 47.81 mmol) was dissolved with dichloromethane (260 mL) and methanol (26 mL) in a 500 mL single-neck flask. Hydrazine hydrate (5.98 g, 95.62 mmol) was slowly added dropwise at room temperature. After the addition, the obtained mixture was reacted at room temperature 2.5 hrs. After the reaction was completed, the reaction liquid was filtered. The filtrate was washed with 5N ammonia (200 mL) and extracted with dichloromethane (200 mL × 2), The organic phase was dried over anhydrous sodium sulfate and then concentrated. The residue was dissolved in ethanol (50 mL). Then concentrated hydrochloric acid (8 mL) was added. The obtained mixture was stirred for 30 min and then concentrated. The resulting residue was slurried with ethyl acetate (30 mL). Filtration was performed to obtain 45C (1.90 g, yield 34%).

$^1$H NMR (400 MHz, DMSO-$d_6$) δ 11.25 (s, 3H), 4.74 - 4.72 (m, 1H), 4.62 - 4.60 (m, 1H), 4.36 - 4.34 (m, 1H), 4.29 - 4.27 (m, 1H).

Step 3: 45C (0.49 g, 3.23 mmol) and water (10 mL) were added into a 250 mL single-neck flask. A solution of sodium hydroxide (0.12 g, 3.04 mmol) in water (10 mL) was slowly added dropwise at 0-5°C. The obtained mixture was reacted at 0-5°C for 10 min. Then 6-hydroxy-2H-benzo[d][1,3]oxazine-2,4(1H)-dione (0.17 g, 0.95 mmol) was added at 0-5°C and the obtained mixture was reacted at room temperature overnight. After the reaction was completed, the reaction liquid was concentrated and purified by column chromatography (dichloromethane/methanol = 10/1) to obtain 45D (0.20 g, yield 98%).

LCMS m/z = 215.1 [M+H]$^+$;
Step 4: 45D (0.2 g, 0.93 mmol) was added to triethyl orthoformate (3 mL) and N,N-dimethylformamide (3 mL) in a 100 mL single-neck flask. The obtained mixture was reacted at 150°C for 4 hrs. After the reaction was completed, the reaction liquid was cooled to room temperature, and concentrated to obtain crude product 45E, which was directly used in the next reaction.

LCMS m/z = 225.1 [M+H]$^+$;

Step 5: 45E (0.31 g, 1.38 mmol) was dissolved in dry N,N-dimethylformamide (5 mL) in a 25 mL single-neck flask. Cesium carbonate (0.54 g, 1.66 mmol) was added under an ice bath. 2,3,6-trifluorobenzonitrile (0.24 g, 1.52 mmol) was slowly added dropwise. After the addition, the obtained mixture was reacted under stirring at room temperature overnight. After the reaction was completed, the reaction was filtered, and the filtrate was concentrated and then purified by column chromatography (petroleum ether/ethyl acetate = 2/3) to obtain 45F (0.36 g, yield 72%).

LCMS m/z =362.4 $[M+H]^+$;

Step 6: 1B (0.21 g, 1.20 mmol) and cesium carbonate (0.49 g, 1.5 mmol) were added to dry N,N-dimethylformamide (8 mL) in a 25 mL single-neck flask. The obtained mixture was reacted at 50°C for 30 min. A solution of 45F (0.36 g, 1.00 mmol) in N,N-dimethylformamide (2 mL) was added dropwise at 50°C. After the addition, the obtained mixture was reacted under stirring at 80°C overnight. After the reaction was completed, the reaction liquid was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 15 min) to obtain compound 45 (83.0 mg, yield 16%).

LCMS m/z = 518.2 $[M+H]^+$;
$^1$H NMR (400 MHz, DMSO-$d_6$) δ 10.38 (s, 1H), 8.58 (s, 1H), 7.91 - 7.83 (m, 2H), 7.74-7.71 (dd, 1H), 7.56-7.53 (dd, 1H), 7.42-7.41 (d,1H), 4.83 - 4.69 (m, 2H), 4.59 - 4.49 (m, 2H), 3.84 (s, 4H), 2.12-2.08 (t, 4H), 1.78 - 1.70 (m, 2H).
$^{19}$F NMR (376 MHz, DMSO-$d_6$) δ -127.24 (s), -220.60 (s).

## Example 46

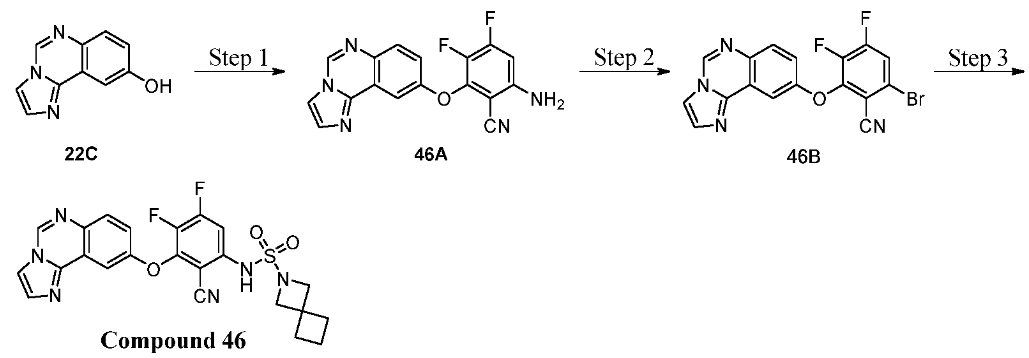

22C 46A 46B
Compound 46

Step 1: 22C (1.35 g, 7.29 mmol) was dissolved in dry N,N-dimethylformamide (30 mL) in a 25 mL single-neck flask. Cesium carbonate (4.75 g, 14.58 mmol) was slowly added under an ice bath. After the addition, the obtained mixture was reacted under stirring at room temperature for 0.5 h. 39A (1.51 g, 8.75 mmol) was slowly added dropwise under an ice bath. After the addition, the obtained mixture was reacted under stirring at 30°C overnight. After the reaction was completed, the reaction liquid was filtered, and the filtrate was concentrated and then purified by column chromatography (petroleum ether/ethyl acetate = 3/7) to obtain 46A (2.3 g, yield 93%).

LCMS m/z =338.3 $[M+H]^+$;

Step 2: 46A (2.30 g, 6.82 mmol) was dissolved in dry acetonitrile (50 mL) in a 25 mL single-neck flask. Tert-butyl nitrite (0.71 g, 6.89 mmol) and copper bromide (1.54 g, 6.90 mmol) were slowly added at 0-5°C. After the addition, the obtained mixture was reacted under stirring at room temperature overnight. After the reaction was completed, the reaction liquid was concentrated. After the concentration, saturated sodium bicarbonate (100 mL) was added to quench the reaction, and ethyl acetate (100 mL) was used for dissolution. Then filtration was performed. The filtrate was extracted with ethyl acetate (100 mL × 2). The organic phase was washed with water (100 mL) and saturated brine (100 mL), dried over anhydrous sodium sulfate, and then concentrated. The residue was purified by column chromatography (petroleum ether/ethyl acetate = 2/3) to obtain 46B (1.00 g, yield 36%).

LCMS m/z =401.0 $[M+H]^+$;

Step 3: 46B (0.80 g, 1.99 mmol), 1B (0.46 g, 2.59 mmol), 2-dicyclohexylphosphine-2',4',6'-triisopropylbiphenyl (0.10 g, 0.20 mmol), potassium carbonate (0.41 g, 2.98 mmol), and tris(dibenzylidene-BASEacetone)dipalladium (0.11 g, 0.19 mmol) were added to dry 1,4-dioxane (16 mL). The obtained mixture was purged with nitrogen for 2 min and then reacted under microwave at 120°C for 1 hr. After the reaction was completed, the filtrate was concentrated, and then ethyl acetate (20 mL) and water (20 mL) were added for dissolution. Liquid separation was performed, and the aqueous phase was adjusted to neutral pH with saturated ammonium chloride, and extracted with ethyl acetate (20 mL × 3), The organic

phases were combined, dried over anhydrous sodium sulfate, and then concentrated. The residue was purified by column chromatography (petroleum ether/ethyl acetate = 3/2), and then subjected to chiral SFC resolution to obtain compound 46 (retention time: 2.08 min). Resolution method: instrument name: Waters 150 Prep-SFC F; chromatographic column: Chiralcel AD column; mobile phase: A for $CO_2$ and B for MeOH (0.1%$NH_3 \cdot H_2O$); gradient: B 40%; flow rate: 100 mL/min; column pressure: 100 bar; column temperature: 25°C; absorption wavelength: 220 nm cycle time: 3.8 min). Sample preparation: the compound was dissolved in acetonitrile at a concentration of 10 mg/mL; injection: 3.5 mL/injection; treatment: after the separation, the obtained product was concentrated by a rotary evaporator at 35°C, and then the solvent was dried by a lyophilizer at -80°C to obtain compound 46 (0.18 mg, yield 18%).

LCMS m/z = 497.8 $[M+H]^+$;
$^1H$ NMR (400 MHz, DMSO-$d_6$) δ 9.28 (s, 1H), 8.10-8.09 (d, 1H), 8.02-7.99 (d, 1H), 7.78-7.77 (d, 1H), 7.62 - 7.58 (m, 2H), 7.50-7.46 (m, 1H), 3.79 (s, 4H), 2.10-2.06 (t, 4H), 1.75 - 1.67 (m, 2H).

**Example 47**

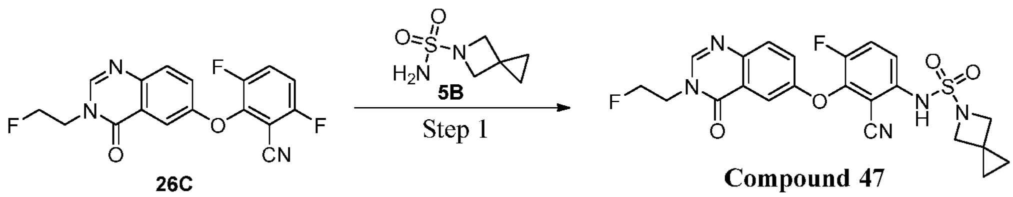


Step 1: Compound 5B (0.55 g, 3.39 mmol) was dissolved in dry N,N-dimethylformamide (10 mL) in a 250 mL single-neck flask. After the addition of cesium carbonate (1.42 g, 4.36 mmol), the obtained mixture was reacted at 50°C for 0.5 h. Then a solution of 26C (1 g, 2.90 mmol) in N,N-dimethylformamide (10 mL) was added dropwise. After the addition, the reaction liquid was stirred at 85°C for 12 h. After the reaction was completed, ethyl acetate (100 mL) was added. Then washing was performed with water (70 mL × 2). The organic layer was dried over anhydrous sodium sulfate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 25%-65% acetonitrile; cycle time: 15 min) to obtain compound 47 (700 mg, yield 49%).

LCMS m/z = 488.30 $[M+H]^+$;
$^1H$ NMR (400 MHz, DMSO-$d_6$) δ 10.47 (s, 1H), 8.33 (s, 1H), 7.88 (t, 1H), 7.80 (d, 1H), 7.76-7.68 (m, 1H), 7.61-7.56 (m, 1H), 7.41 (d, 1H), 4.79-4.62 (m, 2H), 4.36-4.25 (m, 2H), 3.97 (s, 4H), 0.61 (s, 4H).

**Example 48**

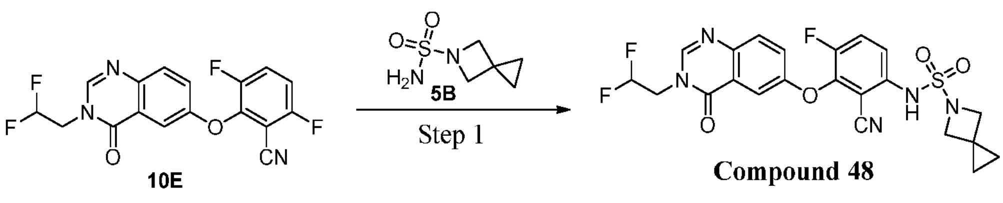


Step 1: Compound 5B (8.2 g, 50.55 mmol) was dissolved in dry N,N-dimethylformamide (50 mL) in a 250 mL single-neck flask. After the addition of cesium carbonate (16.6 g, 7.41 mmol), the obtained mixture was stirred at 50°C for 0.5 h. Then a solution of 9E (2.5 g,6.88 mmol) in N,N-dimethylformamide (15 mL) was added dropwise. After the addition, the reaction liquid was stirred at 85°C for 12 h. After the reaction was completed, filtration was performed. The filtrate was concentrated, and the obtained residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-70% acetonitrile; cycle time: 10 min) to obtain compound 48 (250 mg, yield 7%).

LCMS m/z = 506.90 $[M+H]^+$;
$^1$H NMR (400 MHz, DMSO-$d_6$) δ 8.34 (s, 1H), 7.93 (t, 1H), 7.86-7.76 (m, 3H), 7.74-7.69 (m, 1H), 7.48 (d, 1H), 6.54-6.18 (m, 1H), 4.53-4.43 (m, 1H), 3.55 (s, 2H), 3.25 (s, 2H), 0.64 (s, 4H).

**Example 49**

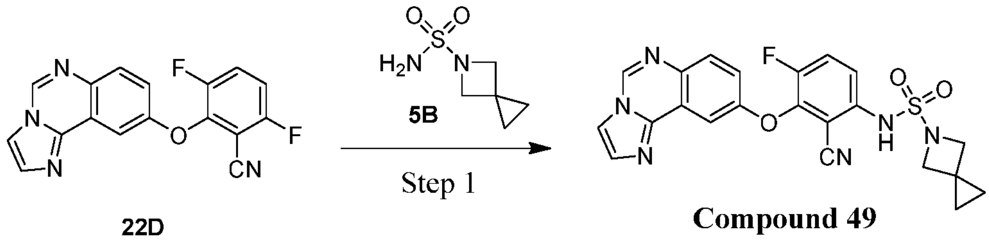


Step 1: Raw material 5B (0.45 g, 2.77 mmol) was dissolved in DMF (10 mL). Then cesium carbonate (0.91 g, 2.79 mmol) was added. The reaction liquid was heated to 50°C and stirred for 30 min. Then raw material 22D (0.6g, 1.86mmol) was added, and the reaction liquid was heated to 85°C and stirred overnight. After the reaction was completed, the reaction mixture was filtered through diatomaceous earth, and the filtrate was concentrated. The resulting residue was purified by slurrying with EA to obtain 0.50 g of a white solid. Then, the white solid was purified through a silica gel column (dichloromethane : methanol = 20 : 1) to obtain compound 49 (0.38 g, 43.99%).
LCMS m/z = 465.3 $[M+H]^+$.
$^1$H NMR (400 MHz, DMSO-$d_6$) δ 9.27 (s, 1H), 8.09 (d, 1H), 8.01 (d, 1H), 7.74 (t, 1H), 7.63 (d, 1H), 7.60 - 7.52 (m, 3H), 3.89 (s, 4H), 0.57 (s, 4H).

**Example 50**

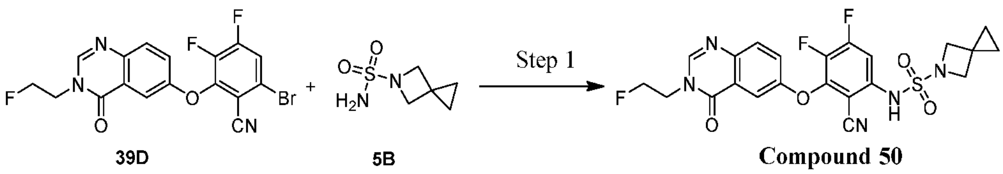


Step 1: 39D (500 mg, 1.18 mmol), 5B (230 mg, 1.42 mmol), 2-di-tert-butylphosphino-2',4',6'-triisopropylbiphenyl (100 mg, 0.24 mmol), allylpalladium chloride (65 mg, 0.18 mmol), and potassium carbonate (571 mg, 4.13 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction liquid was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 30%-80% acetonitrile; cycle time: 20 min) to obtain compound 50 (72 mg, yield: 12%).

LCMS m/z = 506.1 $[M+H]^+$;
$^1$H NMR (400 MHz, DMSO-$d_6$) δ 8.33 (s, 1H), 7.79 (d, 1H), 7.74 (dd, 1H), 7.57 (d, 1H), 7.51 (dd, 1H), 4.71 (dt, 2H), 4.32 (dt, 2H), 3.93 (s, 4H), 0.59 (s, 4H).

**Example 51**

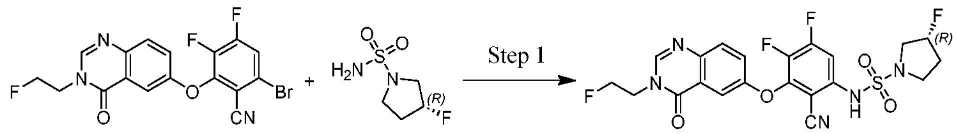


**39D** **Compound 51**

Step 1: 39D (500 mg, 1.18 mmol), (3R)-3-fluoropyrrolidine-1-sulfonamide (238 mg, 1.42 mmol), 2-di-tert-butylphosphino-2',4',6'-triisopropylbiphenyl (100 mg, 0.24 mmol), allylpalladium chloride (65 mg, 0.18 mmol), and potassium carbonate (571 mg, 4.13 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction liquid was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: gradient elution using 20%-80% acetonitrile; cycle time: 15 min) to obtain compound 51 (210 mg, yield: 31%).

LCMS m/z = 512.1 $[M+H]^+$;

$^1H$ NMR (400 MHz, DMSO-$d_6$) $\delta$ 8.34 (s, 1H), 7.80 (d, 1H), 7.74 (dd, 1H), 7.58 (d, 1H), 7.52 (dd, 1H), 5.40 (s, 1H), 5.26 (s, 1H), 4.72 (dt, 2H), 4.32 (dt, 2H), 3.57 - 3.31 (m, 4H), 2.23 - 1.99 (m, 2H).

**Example 52**

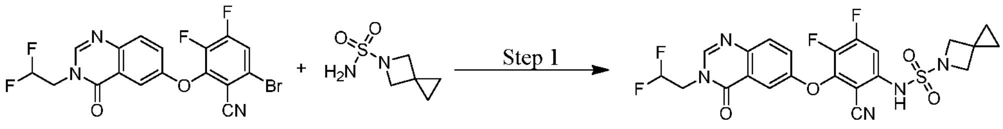


43D 5B Compound 52

Step 1: 43D (800 mg, 1.81 mmol), 5B (352 mg, 2.17 mmol), 2-di-tert-butylphosphino-2',4',6'-triisopropylbiphenyl (80 mg, 0.18 mmol), and potassium carbonate (876 mg, 6.33 mmol) were dissolved in dry 1,4-dioxane (40 mL). Allylpalladium chloride (33 mg, 0.09 mmol) was added. After the addition, nitrogen replacement was performed. The obtained mixture was reacted under stirring at 85°C for 10 h. After the reaction was completed as monitored by TLC (dichloromethane : methanol = 20 : 1 (v/v)), the reaction liquid was filtered, the filtrate was concentrated, and the crude product was purified by column chromatography (eluent: dichloromethane : methanol = 50 : 1 (v/v)) to obtain the target compound 52(340 mg, yield: 36%).

LCMS *m/z*= 524.2 $[M+1]^+$.

$^1H$ NMR (400 MHZ, DMSO-$d_6$) $\delta$ 8.35 (s, 1H), 7.81 (d, 1H), 7.77 (dd, 1H), 7.62 (d, 1H), 7.57 (dd, 1H), 6.37 (tt, 1H), 4.49 (td, 2H), 3.99 (s, 4H), 0.61 (s, 4H).

**Example 53**

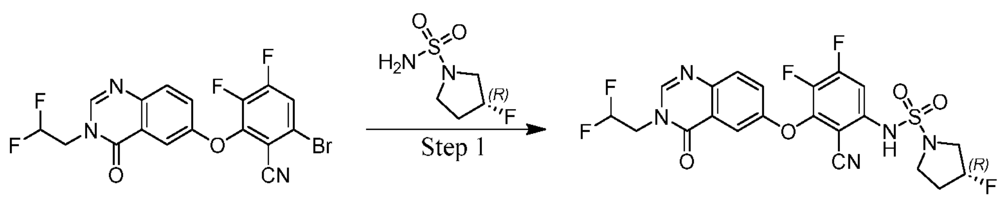


43D Compound 53

Step 1: 43D (800 mg, 1.81 mmol), (3R)-3-fluoropyrrolidine-1-sulfonamide (365 mg, 2.17 mmol), 2-di-tert-butylphosphino-2',4',6'-triisopropylbiphenyl (80 mg, 0.18 mmol), and potassium carbonate (876 mg, 6.33 mmol) were dissolved in dry 1,4-dioxane (40 mL). Allylpalladium chloride (33 mg, 0.09 mmol) was added. After the addition, nitrogen

replacement was performed. The obtained mixture was reacted under stirring at 85°C for 10 h. After the reaction was completed as monitored by TLC (dichloromethane : methanol = 20 : 1 (v/v)), the reaction liquid was filtered, the filtrate was concentrated, and the crude product was purified by column chromatography (eluent: dichloromethane : methanol = 65 : 1 (v/v)) to obtain the target compound 53(650 mg, yield: 68%).

LCMS *m/z* = 530.1 $[M+1]^+$.

$^1$H NMR (400 MHZ, DMSO-$d_6$) $\delta$ 8.35 (s, 1H), 7.81 (d, 1H), 7.75 (dd, 1H), 7.60 (d, 1H), 7.52 (dd, 1H), 6.37 (tt, 1H), 5.33 (d, 1H), 4.49 (td, 2H), 3.58 - 3.34 (m, 4H), 2.21 - 2.01 (m, 2H).

**Biological test example 1:** $BRAF_{V600E}$ enzyme activity test

$BRAF_{V600E}$ (ABCAM, ab204154) and $MEK1_{K97R}$ (USBio, M2865-06J) proteins were diluted at an appropriate dilution factor using 1 × Assay buffer (PH = 7.4 Tris-HCl buffer, supplemented with 10 mM $MgCl_2$) such that the final concentration of $BRAF_{V600E}$ was 10 ng/μL and the final concentration of substrate $MEK1_{K97R}$ was 1 μM. 1 μL of $BRAF_{V600E}$, 1 μL of compound serial dilution (final concentration starting from 2 μM, 5-fold dilution, 8 concentrations), and 6 μL of Assay buffer were pipetted to a 384-well reaction plate with a capacity of 20 μL. The plate was pre-incubated in a constant temperature incubator at 37°C for 30 min. 1 μL of 200 μM ATP and 1 μM $MEK1_{K97R}$ were added to the reaction wells corresponding to the compound incubation, mixed evenly by vortexing, and preincubation was performed in a constant temperature incubator at 37°C for 60 min for enzymatic reaction. After the reaction was completed, 5 μL of the above reaction product was pipetted to another 384-well plate, and 5 μL of formulated ADP-Glo™ Reagent (Promega, V9101) was added and mixed evenly by pipetting, and then the plate was placed at room temperature for 40 min. 10 μL Kinase Detection Reagent was added to the 384-well plate and incubated at room temperature for 40 min. Finally, a microplate reader (BMG LRBTECH) was used and Luminescence module was selected to detect the LUM fluorescence value of each well (the gain value was fixed at 3600), and the formula

$$\text{Inhibition rate\%} = \left(1 - \frac{LUM\ Compound - LUM\ Background}{LUM\ Solvent - LUM\ Background}\right) * 100\%$$

was used to calculate the inhibition rate of the compound on $BRAF_{V600E}$. The Graphpad software log(inhibitor) vs. response -- Variable slope (four parameters) equation was used to perform fitting analysis and calculate the $IC_{50}$value of the sample.

The compounds of the present invention, such as the compounds in the examples, have very good enzymatic activities, with $IC_{50} \leq 100$ nM. The inhibitory activities of some compounds on $BRAF_{V600E}$ are shown in Table 1.

Table 1 Inhibitory activities of compounds on BRAFV600E

| Compound No. | $IC_{50}$/nM |
|---|---|
| Compound 1 | B |
| Compound 9 | B |
| Compound 10 | A |
| Compound 11 | B |
| Compound 26 | A |
| Compound 39 | A |
| Compound 40 | A |

A represents $IC_{50} \leq 10$ nM, B represents 10 nM < $IC_{50} \leq 50$ nM, C represents 50 nM < $IC_{50} \leq 100$ nM.

Conclusion: the compounds of the present invention, such as the compounds in the examples, show high inhibitory activities on $BRAF_{V600E}$.

**Biological test example 2:** A375 cell proliferation inhibition

A375 cells (ATCC, CRL-1619) were cultured in DMEM complete medium (+10% FBS) in a $CO_2$ incubator at 37°C for 48 h. The cells were trypsinized and counted, and then the density was adjusted to 1.67 × $10^4$ cells/mL. 90 μL (1500 cells) of cells were inoculated into each well of a 96-well transparent bottom plate, and the plate was transferred to a $CO_2$ incubator and the cells were cultured overnight at 37°C. After the cells were incubated overnight, 10 μL of the diluted

compound (final concentration starting from 10 μM, 3-fold dilution, 11 concentrations) was added to each well using a multi-channel pipette. The positive control was a serum-free medium containing DMSO. After mixing evenly, the cells were placed in a $CO_2$ incubator at 37°C for 72 h. After the incubation, the CellCounting-Lite®2.0 kit detection solution (Vazyme, DD1101-03) was taken out and returned to room temperature. 100 μL of CellCounting-Lite2.0 detection solution was added to each well, the plate was sealed with a sealing film, and the plate was placed on a shaker for shaking for 15 min (the whole process should be kept away from light). The Luminescence module of the microplate reader (BMG LRBTECH) was used to detect the fluorescence signal value LUM of each well. The formula

$$\text{Inhibition rate\%} = \left(\frac{LUM\ Solvent - LUM\ Compound}{LUM\ Solvent}\right) * 100\%$$

was used to calculate the inhibition rate of the compound. The Graphpad software log(inhibitor) vs. response -- Variable slope (four parameters) equation was used to perform fitting analysis and calculate the $IC_{50}$value of the sample. The ordinate of the data is the percentage of inhibition rate, and the abscissa is the logarithm of the sample concentration ($Log_{10}$).

The compounds of the present invention, such as the compounds in the examples, have very good cell activities, with $IC_{50} \leq 100$ nM. The inhibitory activities of some compounds on A357 cells are shown in Table 2.

Table 2 Inhibitory activities of compounds on A375 cells

| Compound No. | $IC_{50}$/nM |
|---|---|
| Compound 1 | A |
| Compound 4 | A |
| Compound 5 | A |
| Compound 9 | A |
| Compound 10 | A |
| Compound 14 | A |
| Compound 17 | A |
| Compound 22 | A |
| Compound 26 | A |
| Compound 33 | A |
| Compound 39 | A |
| Compound 40 | A |
| Compound 42 | A |
| Compound 44 | A |
| Compound 46 | A |
| Compound 47 | A |
| Compound 49 | A |
| Compound 50 | A |
| Compound 51 | A |

A represents $IC_{50} \leq 10$ nM, B represents 10 nM < $IC_{50} \leq 50$ nM, C represents 50 nM < $IC_{50} \leq 100$ nM.

Conclusion: the compounds of the present invention, such as the compounds in the examples, show high inhibitory activities at the cellular level.

**Biological test example 3:** mouse pharmacokinetic test

**1.1 Experimental animals:** male ICR mice, 20-25 g, 12 mice/compound, purchased from Chengdu Ddossy Experimental Animals Co., Ltd.

**1.2 Experimental design:** on the day of the test, 12 ICR mice were randomly grouped according to their body

weights. The animals were fasted with water available for 12 to 14 h one day before the administration and were fed 4 h after the administration.

Table 3. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Control compound 1 | 2.5 | 0.5 | 5 | Plasma | Intravenous administration |
| G2 | 9 | | 10 | 1 | 10 | Plasma | Intragastric administration |
| G3 | 3 | Compound 1 | 2.5 | 0.5 | 5 | Plasma | Intravenous administration |
| G4 | 9 | | 10 | 1 | 10 | Plasma | Intragastric administration |
| G5 | 3 | Compound 4 | 2.5 | 0.5 | 5 | Plasma | Intravenous administration |
| G6 | 9 | | 10 | 1 | 10 | Plasma | Intragastric administration |
| G7 | 3 | Compound 5 | 2.5 | 0.5 | 5 | Plasma | Intravenous administration |
| G8 | 9 | | 10 | 1 | 10 | Plasma | Intragastric administration |
| G9 | 3 | Compound 7 | 2.5 | 0.5 | 5 | Plasma | Intravenous administration |
| G10 | 9 | | 10 | 1 | 10 | Plasma | Intragastric administration |
| G11 | 3 | Compound 8 | 2.5 | 0.5 | 5 | Plasma | Intravenous administration |
| G12 | 9 | | 10 | 1 | 10 | Plasma | Intragastric administration |
| G13 | 3 | Compound 10 | 2.5 | 0.5 | 5 | Plasma | Intravenous administration |
| G14 | 9 | | 10 | 1 | 10 | Plasma | Intragastric administration |
| G15 | 3 | Compound 11 | 2.5 | 0.5 | 5 | Plasma | Intravenous administration |
| G16 | 9 | | 10 | 1 | 10 | Plasma | Intragastric administration |
| G17 | 3 | Compound 26 | 2.5 | 0.5 | 5 | Plasma | Intravenous administration |
| G18 | 9 | | 10 | 1 | 10 | Plasma | Intragastric administration |

(continued)

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G19 | 3 | Compound 44 | 2.5 | 0.5 | 5 | Plasma | Intravenous administration |
| G20 | 9 | | 10 | 1 | 10 | Plasma | Intragastric administration |
| Note: Control compound 1 was compound Example 1 in the document WO 2021116055 A1; vehicle for intravenous administration: 5%DMA+5%HS-15+90%NS; vehicle for intragastric administration: 0.5% MC | | | | | | | |

Before and after administration, 0.06 mL of blood was taken from the orbit under isoflurane anesthesia and placed in an EDTAK2 centrifuge tube for centrifugation at 5000 rpm at 4°C for 10 min, followed by plasma collection. The blood collection time points for the intravenous administration group and intragastric administration group were 0, 5 min, 15 min, 30 min, 1 h, 2 h, 4 h, 7 h and 24 h. Before analysis and detection, all samples were stored at -80°C, and a quantitative analysis of samples was performed using LC-MS/MS.

Table 4. Pharmacokinetic parameters of test compounds in plasma of mice

| Test compound | Mode of administration | CL (mL/min/kg) | $Vd_{ss}$ (L/kg) | $AUC_{0-t}$ (hr×ng/mL) | $T_{1/2}$ (h) | F (%) | brain/plasma ratio |
|---|---|---|---|---|---|---|---|
| Control compound 1 | i.v. (2.5 mg/kg) | 0.532 | 0.195 | 75119 | 5.32 | - | - |
| | i.g. (10 mg/kg) | - | - | 240899 | 2.46 | 80.2 | 0.00292 |
| Compound 1 | i.v. (2.5 mg/kg) | 0.133 | 0.148 | 223249 | 14.8 | - | - |
| | i.g. (10 mg/kg) | - | - | 861406 | 10.7 | 96.5 | 0.0130 |
| Compound 4 | i.v. (2.5 mg/kg) | 0.37 | 0.147 | 107297 | 5.27 | - | - |
| | i.g. (10 mg/kg) | - | - | 282018 | 4.02 | - | 0.053 |
| Compound 5 | i.v. (2.5 mg/kg) | 0.0627 | 0.159 | 289734 | 30.2 | - | - |
| | i.g. (10 mg/kg) | - | - | 1084669 | 19.2 | 93.6 | 0.0181 |
| Compound 7 | i.v. (2.5 mg/kg) | 0.144 | 0.111 | 239760 | 9.79 | - | - |
| | i.g. (10 mg/kg) | - | - | 792179 | 13.7 | 92.6 | 0.0425 |
| Compound 8 | i.v. (2.5 mg/kg) | 0.147 | 0.0941 | 246506 | 8.59 | - | - |
| | i.g. (10 mg/kg) | - | - | 1303073 | 12.9 | ≥ 98 | 0.0173 |
| Compound 10 | i.v. (2.5 mg/kg) | 0.117 | 0.111 | 272309 | 12 | - | - |
| | i.g. (10 mg/kg) | - | - | 914322 | 11.8 | 83.9 | 0.0107 |
| Compound 11 | i.v. (2.5 mg/kg) | 0.0822 | 0.0837 | 376040 | 12.8 | - | - |
| | i.g. (10 mg/kg) | - | - | 848012 | 11.74 | - | 0.00950 |
| Compound 26 | i.v. (2.5 mg/kg) | 0.119 | 0.114 | 266055 | 12.8 | - | - |
| | i.g. (10 mg/kg) | - | - | 831553 | 8.68 | 78.1 | 0.01785 |
| Compound 44 | i.v. (2.5 mg/kg) | 0.335 | 0.135 | 125977 | 7.5 | - | - |
| | i.g. (10 mg/kg) | - | - | 442783 | 14.2 | 87.9 | 0.0162 |
| Note: Control compound 1 was compound Example 1 in the document WO 2021116055 A1;<br>-: not applicable.<br>Conclusion: The compounds of the present invention, such as compounds in the examples, have good pharmacokinetic characteristics in mice and have better brain-penetrating properties than the control compounds. | | | | | | | |

**Biological test example 4: Beagle dog pharmacokinetic test**

**Experimental animals:** male beagle dogs, about 8-11 kg, 6 beagle dogs/compound, purchased from Beijing Marshall Biotechnology Co., Ltd.

**Experimental method:** on the day of the experiment, 6 beagle dogs were randomly grouped according to their body weights. The animals were fasted with water available for 12 to 14 h one day before the administration and were fed 4 h after the administration. Administration was performed as per Table 1.

Table 5. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Control compound 1 | 1 | 1 | 1 | Plasma | Intravenous administration |
| G2 | 3 | Control compound 1 | 3 | 0.6 | 5 | Plasma | Intragastric administration |
| G3 | 3 | Compound 1 | 0.5 | 0.25 | 2 | Plasma | Intravenous administration |
| G4 | 3 | Compound 1 | 3 | 0.6 | 5 | Plasma | Intragastric administration |
| G5 | 3 | Compound 10 | 0.5 | 0.25 | 2 | Plasma | Intravenous administration |
| G6 | 3 | Compound 10 | 3 | 0.6 | 5 | Plasma | Intragastric administration |
| G7 | 3 | Compound 39 | 0.5 | 0.25 | 2 | Plasma | Intravenous administration |
| G8 | 3 | Compound 39 | 3 | 0.6 | 5 | Plasma | Intragastric administration |
| Note: Vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: Methylcellulose solution ) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for the intravenous group and intragastric group were both 0 min, 5 min, 15 min, 30 min, 1 h, 2 h, 4 h, 6 h, 8 h, 10 h, 12 h, 24 h, 48 h, and 72 h. Before analysis and detection, all samples were stored at -80°C. The samples were analyzed quantitatively by LC-MS/MS.

Table 6. Pharmacokinetic parameters of test compounds in plasma of beagle dogs

| Test compound | Mode of administration | CL (mL/min/kg) | $Vd_{ss}$ (L/kg) | $AUC_{0-t}$ (hr×ng/mL) | F (%) |
|---|---|---|---|---|---|
| Control compound 1 | i.v. (1 mg/kg) | 0.686 | 0.189 | 24350 | - |
| Control compound 1 | i.g. (3 mg/kg) | - | - | 53143 | 72.7 |
| Compound 1 | i.v. (0.5 mg/kg) | 0.492 | 0.156 | 17680 | - |
| Compound 1 | i.g. (3 mg/kg) | - | - | 116724 | ≥ 98 |
| Compound 10 | i.v. (0.5 mg/kg) | 0.425 | 0.155 | 40879 | - |
| Compound 10 | i.g. (3 mg/kg) | - | - | 97760 | 79.7 |

(continued)

| Test compound | Mode of administration | CL (mL/min/kg) | $Vd_{ss}$ (L/kg) | $AUC_{0-t}$ (hr×ng/mL) | F (%) |
|---|---|---|---|---|---|
| Compound 39 | i.v. (0.5 mg/kg) | 0.541 | 0.08 | 31095 | - |
| Compound 39 | i.g. (3 mg/kg) | - | - | 84281 | 90.3 |
| Note: Control compound 1 was compound Example 1 in the document WO 2021116055 A1;<br>-: not applicable. | | | | | |

**Conclusion:** The compounds of the present invention, such as the compounds in the examples, have good pharmacokinetic characteristics in beagle dogs.

**Biological test example 5: Rat pharmacokinetic test**

5.1 Experimental animals: male SD rats, about 220 g, 6-8 weeks old, 6 rats/compound, purchased from Chengdu Ddossy Experimental Animals Co., Ltd.

5.2 Experimental design: on the day of the experiment, 6 SD rats were randomly grouped according to their body weights; the animals were fasted with water available for 12 to 14 h one day before the administration and were fed 4 h after the administration.

Table 7 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Control compound 1 | 2.5 | 0.5 | 5 | Plasma | Intravenous administration |
| G2 | 3 | Control compound 1 | 10 | 1 | 10 | Plasma | Intragastric administration |
| G3 | 3 | Compound 47 | 2.5 | 0.5 | 5 | Plasma | Intravenous administration |
| G4 | 3 | Compound 47 | 10 | 1 | 10 | Plasma | Intragastric administration |
| Vehicle for administration: 0.5% MC | | | | | | | |

Before and after the administration, 0.1 ml of blood was taken from the orbit of the animals under isoflurane anesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h, and 24 h; blood sampling time points for the intragastric administration group: 0, 5 min, 15 min, 30 min, 1 h, 2 h, 4 h, 6 h, 8 h and 24 h. Before analysis and detection, all samples were stored at -80°C.

Table 8 Pharmacokinetic parameters of test compounds in plasma of rats

| Test compound | Mode of administration | CL (mL/min/kg) | $Vd_{ss}$ (L/kg) | $AUC_{0-t}$ (hr×ng/mL) | $T_{1/2}$ (h) | F (%) |
|---|---|---|---|---|---|---|
| Control compound 1 | Intravenous administration (2.5 mg/kg) | 0.171 | 0.142 | 199410 | 10.5 | - |
| Control compound 1 | Intragastric administration (10 mg/kg) | - | - | 634644 | 9.73 | 79.6 |
| Compound 47 | Intravenous administration (2.5 mg/kg) | 0.036 | 0.156 | 1156844 | 50.9 | - |

(continued)

| Test compound | Mode of administration | CL (mL/min/kg) | $Vd_{ss}$ (L/kg) | $AUC_{0-t}$ (hr×ng/mL) | $T_{1/2}$ (h) | F (%) |
|---|---|---|---|---|---|---|
| Compound 47 | Intragastric administration (10 mg/kg) | - | - | 4256852 | 60.6 | 92 |
| Note: Control compound 1 was compound Example 1 in the document WO 2021116055 A1;<br>-: not applicable.<br>Conclusion: The compounds of the present invention, such as the compounds in the examples, have good bioavailability and pharmacokinetic characteristics in rats. | | | | | | |

## Claims

**1.** A compound represented by formula I, and a stereoisomer, deuterated product or pharmaceutically acceptable salt thereof,

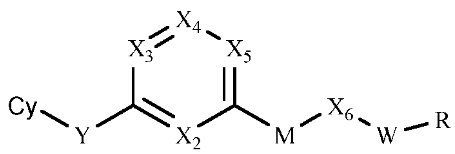

I

**characterized in that**, Cy is selected from P1, P2, P3, P4, P5, P6, P7 or P8;

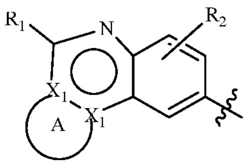

P1 ,

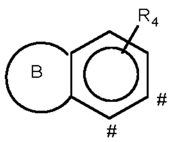

P2 ,

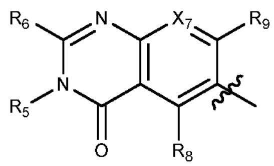

P3 ,

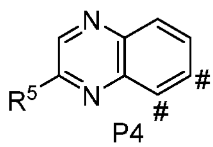

P4 ,

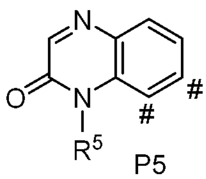

P5 ,

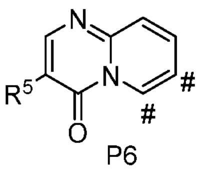

P6 ,

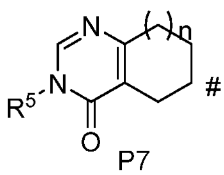

P7 ,

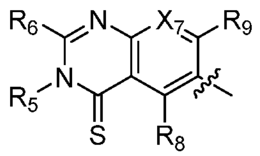

P8 ;

ring A is 5-membered or 6-membered heteroaryl containing 1 to 3 heteroatoms selected from N, S, and O, and the heteroaryl is optionally substituted with 1 or 2 groups selected from halogen, $C_{1-4}$ alkoxy, $-NHC_{1-4}$ alkyl, $-N(C_{1-4}$ alkyl$)_2$, CN, $C_{1-4}$ alkyl, halo$C_{1-4}$ alkoxy and halo$C_{1-4}$ alkyl;
ring B is 5-membered heterocyclic ring containing 1 to 3 heteroatoms selected from N, S, and O, and the heterocyclic ring is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;
# means that one point is selected to connect to Y;
n = 0 or 1;
each $X_1$ is independently N or C;
$X_2$ is N or $CR_3$;
$X_3$ is N or $CR_{31}$;
$X_4$ is N or $CR_{32}$;
$X_5$ is N or $CR_{33}$;
$X_6$ is C(O), S(O) or $S(O)_2$;

$X_7$ is $CR_7$ or N;

$R_1$, $R_2$ and $R_4$ are independently H, halogen, OH, -NH$C_{1-4}$ alkyl, -N($C_{1-4}$ alkyl)$_2$, CN, $C_{1-4}$ alkyl, $C_{1-4}$ alkoxy, halo$C_{1-4}$ alkoxy, halo$C_{1-4}$ alkyl, $C_{2-4}$ alkenyl or $C_{2-4}$ alkynyl;

$R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -NH$C_{1-4}$ alkyl, -N($C_{1-4}$ alkyl)$_2$, CN or $C_{3-6}$ cycloalkyl, alternatively, $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, -NH$C_{1-4}$ alkyl, -N($C_{1-4}$ alkyl)$_2$, $C_{1-4}$ alkoxy, halo$C_{1-4}$ alkoxy, halo$C_{1-4}$ alkyl, CN, $C_{1-4}$ alkyl or =O;

$R_6$ is H, halogen, $C_{1-4}$ alkyl or halo$C_{1-4}$ alkyl;

alternatively, $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -NH$C_{1-4}$ alkyl, -N($C_{1-4}$ alkyl)$_2$, CN and $C_{1-4}$ alkyl;

$R_7$, $R_8$, and $R_9$ are independently H, halogen, -NH$C_{1-4}$ alkyl, -N($C_{1-4}$ alkyl)$_2$, CN, $C_{1-4}$ alkyl, $C_{2-4}$ alkenyl, $C_{2-4}$ alkynyl, halo$C_{1-4}$ alkyl, $C_{1-4}$ alkoxy or halo$C_{1-4}$ alkoxy;

Y is $C_{1-2}$ alkylene, O or $NR_y$;

$R_y$ is H or $C_{1-4}$ alkyl;

M is $C_{1-2}$ alkylene, O or $NR_m$;

W is a bond, O or $NR_w$;

$R_m$ and $R_w$ are independently H or $C_{1-4}$ alkyl;

R is selected from $C_{1-4}$ alkyl, $C_{3-8}$ cycloalkyl, 4-membered to 10-membered heterocyclic ring containing 1 to 3 heteroatoms selected from N, S, and O, or -O-$C_{3-6}$ cycloalkyl, and the alkyl, cycloalkyl or heterocyclic ring is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl;

when Cy is P2, R is 4-membered to 10-membered saturated heterocycloalkyl containing 1 to 3 heteroatoms selected from N, S, and O, and is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl.

2. The compound, and the stereoisomer, the deuterated product, or the pharmaceutically acceptable salt thereof of claim 1,

**characterized in that**, ring A is 5-membered heteroaryl, and the heteroaryl is optionally substituted with 1 or 2 groups selected from halogen, $C_{1-4}$ alkoxy, -NH$C_{1-4}$ alkyl, -N($C_{1-4}$ alkyl)$_2$, CN, $C_{1-4}$ alkyl, halo$C_{1-4}$ alkoxy and halo$C_{1-4}$ alkyl;

when Cy is P3, the compound meets at least one of the following conditions:

(1) $R_5$ is substituted or unsubstituted $C_{3-6}$ cycloalkyl, substituted or unsubstituted 4-membered to 7-membered heterocycloalkyl containing 1-3 heteroatoms selected from N, S, and O, substituted or unsubstituted $C_{3-6}$ cycloalkyloxy, substituted or unsubstituted $C_{1-4}$ alkoxy or substituted or unsubstituted halo$C_{1-4}$ alkoxy, R is $C_{3-8}$ cycloalkyl, 4-membered to 10-membered heterocyclic ring containing 1 to 3 heteroatoms selected from N, S, and O, or -O-$C_{3-6}$ cycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, -NH$C_{1-4}$ alkyl, -N($C_{1-4}$ alkyl)$_2$ and halo$C_{1-4}$ alkyl;

(2) $R_5$ and $R_6$ together with the atoms to which they are attached form 5-membered or 6-membered heterocyclic ring containing 1 to 3 heteroatoms selected from N, S, and O, and the heterocyclic ring is optionally substituted with 1 to 3 groups selected from halogen, OH, $NH_2$, -NH$C_{1-4}$ alkyl, -N($C_{1-4}$ alkyl)$_2$, CN and $C_{1-4}$ alkyl;

(3) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN, W is O;

(4) $R_{31}$ and $R_{32}$, or $R_{32}$ and $R_{33}$ together with the atoms to which they are attached form $C_{3-6}$ carbocyclic ring or 5-membered or 6-membered heterocyclic ring containing 1 to 3 heteroatoms selected from N, S, and O, and the carbocyclic ring or the heterocyclic ring is optionally substituted with 1 to 3 groups selected from halogen, $C_{1-4}$ alkyl, halo$C_{1-4}$ alkyl, $C_{1-4}$ alkoxy, OH, $NH_2$ and CN;

(5) $X_2$ is N or $CR_3$, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -NH$C_{1-4}$ alkyl, -N($C_{1-4}$ alkyl)$_2$ or CN, W is $NR_w$, R is $C_{3-8}$ cycloalkyl or 4-membered to 10-membered

heterocyclic ring containing 1 to 3 heteroatoms selected from N, S, and O, and is optionally substituted with 1 to 3 groups selected from halogen, $C_{1-4}$ alkyl, $C_{1-4}$ alkoxy, $C_{1-4}$ haloalkoxy, OH, $NH_2$, - $NHC_{1-4}$ alkyl, -N($C_{1-4}$ alkyl)$_2$ and halo$C_{1-4}$ alkyl;

(6) $X_2$ is N or $CR_3$, $X_6$ is SO or $SO_2$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN, R is $C_{3-4}$ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl)$_2$ and halo$C_{1-4}$ alkyl;

(7) $X_6$ is CO, $X_2$ is N or $CR_3$, W is a bond, $R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN;

(8) at least one of $X_2$, $X_3$, $X_4$, and $X_5$ is N, when $X_2$ is $CR_3$, $R_3$ is H, $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl)$_2$ or CN;

(9) $X_2$ is N or $CR_3$, $R_3$ is H, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -$NHC_{1-4}$ alkyl, -N($C_{1-4}$ alkyl)$_2$ or CN, $R_8$ is halogen, - $NHC_{1-4}$ alkyl, -N($C_{1-4}$ alkyl)$_2$, CN, $C_{1-4}$ alkoxy or halo$C_{1-4}$ alkoxy.

**3.** The compound, and the stereoisomer, the deuterated product, or the pharmaceutically acceptable salt thereof of claim 2,

**characterized in that**, $R_3$, $R_{31}$, $R_{32}$, and $R_{33}$ are independently H, halogen, $C_{1-4}$ alkyl, halo$C_{1-4}$ alkyl, $C_{2-4}$ alkenyl, $C_{2-4}$ alkynyl, $C_{1-4}$ alkoxy, halo$C_{1-4}$ alkoxy, -$NHC_{1-4}$ alkyl, -N($C_{1-4}$ alkyl)$_2$, CN or $C_{3-6}$ cycloalkyl, alternatively, $R_{31}$ and $R_{32}$, or $R_{32}$ and $R_{33}$ together with the atoms to which they are attached form $C_{3-6}$ cycloalkyl or 5-membered or 6-membered heterocycloalkyl containing 1 to 3 heteroatoms selected from N, S, and O, and the cycloalkyl or the heterocycloalkyl is optionally substituted with 1 to 3 groups selected from halogen, $C_{1-4}$ alkyl, halo$C_{1-4}$ alkyl, $C_{1-4}$ alkoxy, OH, $NH_2$ and CN;

$R_5$ is $C_{1-4}$ alkyl, halo$C_{1-4}$ alkyl, $C_{3-6}$ cycloalkyl, 4-membered to 7-membered heterocycloalkyl containing 1-3 heteroatoms selected from N, S, and O, $C_{1-4}$ alkoxy, halo$C_{1-4}$ alkoxy or $C_{3-6}$ cycloalkyloxy, and is optionally substituted with 1 to 3 groups selected from halogen, -$NHC_{1-4}$ alkyl, -N($C_{1-4}$ alkyl)$_2$, $C_{1-4}$ alkoxy, halo$C_{1-4}$ alkoxy, halo$C_{1-4}$ alkyl, CN, $C_{1-4}$ alkyl and =O;

$R_6$ is H, halogen, $C_{1-4}$ alkyl or halo$C_{1-4}$ alkyl;

alternatively, $R_5$ and $R_6$ together with the atoms to which they are attached form 5-membered or 6-membered heterocycloalkyl containing 1 to 3 heteroatoms selected from N, S, and O, and the heterocycloalkyl is optionally substituted with 1 to 3 groups selected from halogen, OH, $NH_2$, -$NHC_{1-4}$ alkyl, -N($C_{1-4}$ alkyl)$_2$, CN and $C_{1-4}$ alkyl;

R is selected from $C_{1-4}$ alkyl, $C_{3-8}$ cycloalkyl, 4-membered to 10-membered heterocycloalkyl containing 1 to 3 heteroatoms selected from N, S, and O, or -O-$C_{3-6}$ cycloalkyl, and the alkyl, the cycloalkyl or the heterocycloalkyl is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl)$_2$ and halo$C_{1-4}$ alkyl.

**4.** The compound, and the stereoisomer, the deuterated product, or the pharmaceutically acceptable salt thereof of claim 3, **characterized in that** the compound has the structure as shown in formula I-1,

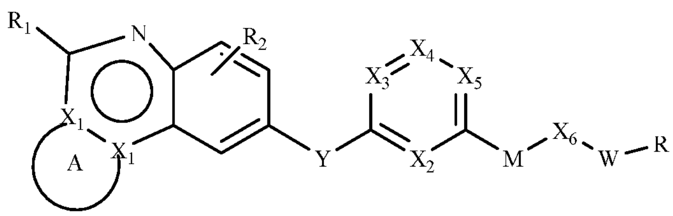

I-1

wherein, R is selected from $C_{1-4}$ alkyl, $C_{3-8}$ cycloalkyl or 4-membered to 7-membered monocyclic heterocycloalkyl containing 1 to 3 heteroatoms selected from N, S, and O, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl)$_2$ and halo$C_{1-4}$ alkyl.

**5.** The compound, and the stereoisomer, the deuterated product, or the pharmaceutically acceptable salt thereof of claim 3, **characterized in that** the compound has the structure as shown in formula 1-2,

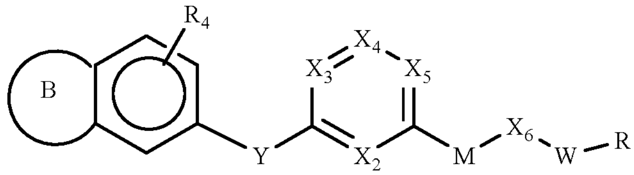

I-2

wherein, ring B is 5-membered heteroaryl or 5-membered heterocycloalkyl containing 1 to 3 heteroatoms selected from N, S, and O, and is optionally substituted with 1 to 3 groups selected from =O, halogen, $C_{1-4}$ alkyl, halo$C_{1-4}$ alkyl, $C_{1-4}$ alkoxy, OH and halo$C_{1-4}$ alkoxy;
R is saturated 4-membered to 7-membered monocyclic heterocycloalkyl containing 1 to 3 heteroatoms selected from N, S, and O, saturated 5-membered to 10-membered fused heterocycloalkyl, saturated 5-membered to 10-membered bridged heterocycloalkyl or saturated 6-membered to 10-membered spirocyclic heterocycloalkyl, and is optionally substituted with 1 to 3 groups selected from halogen, $C_{1-4}$ alkyl, $C_{1-4}$ alkoxy, halo$C_{1-4}$ alkoxy, OH, $NH_2$, -$NHC_{1-4}$ alkyl, -N($C_{1-4}$ alkyl$)_2$ and halo$C_{1-4}$ alkyl.

**6.** The compound, and the stereoisomer, the deuterated product, or the pharmaceutically acceptable salt thereof of claim 3, **characterized in that** the compound has the structure as shown in formula 1-3,

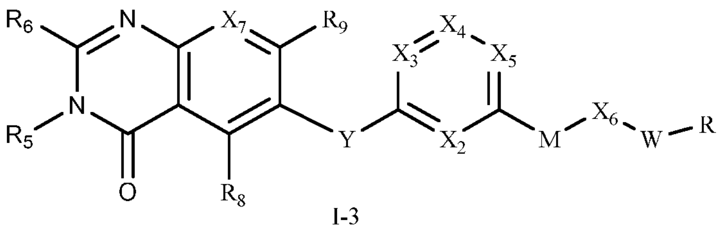

I-3

.

**7.** The compound, and the stereoisomer, the deuterated product, or the pharmaceutically acceptable salt thereof of claim 6, **characterized in that**

$R_6$ and $R_9$ are H; Y is O; M is NH;
$X_2$ is N or $CR_3$, $X_6$ is SO or $SO_2$, and W is a bond;
$R_3$ is $C_{1-4}$ alkyl, halo$C_{1-4}$ alkyl, $C_{1-4}$ alkoxy, halo$C_{1-4}$ alkoxy, $C_{2-4}$ alkenyl, $C_{2-4}$ alkynyl, -$NHC_{1-4}$ alkyl, -N($C_{1-4}$ alkyl$)_2$ or CN; R is 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl, 6-membered to 10-membered spirocyclic heterocycloalkyl or -O-$C_{3-6}$ cycloalkyl, and the 4-membered monocyclic heterocycloalkyl is substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl$)_2$ and halo$C_{1-4}$ alkyl, the 6-membered to 8-membered monocyclic heterocycloalkyl, the 5-membered or 6-membered monocyclic heteroaryl, the 5-membered to 10-membered fused heterocycloalkyl, the 5-membered to 10-membered bridged heterocycloalkyl or the 6-membered to 10-membered spirocyclic heterocycloalkyl is optionally substituted with 1 to 3 groups selected from halogen, $C_{1-4}$ alkyl, $C_{1-4}$ alkoxy, OH, $NH_2$, -$NHC_{1-4}$ alkyl, -N($C_{1-4}$ alkyl$)_2$ and halo$C_{1-4}$ alkyl.

**8.** The compound, and the stereoisomer, the deuterated product, or the pharmaceutically acceptable salt thereof of claim 7, **characterized in that**

$X_2$ is $CR_3$; $X_3$ is $CR_{31}$; $X_4$ is $CR_{32}$; $X_5$ is CH; $X_6$ is $SO_2$; $X_7$ is CH;
$R_3$ is $C_{1-2}$ alkyl, halo$C_{1-2}$ alkyl, $C_{1-2}$ alkoxy, halo$C_{1-2}$ alkoxy, $C_{2-4}$ alkenyl, $C_{2-4}$ alkynyl, -$NHC_{1-2}$ alkyl, -N($C_{1-2}$ alkyl$)_2$ or CN;
each of $R_{31}$ and $R_{32}$ is independently H, F, Cl, $C_{1-2}$ alkyl, halo$C_{1-2}$ alkyl, $C_{2-4}$ alkenyl, $C_{2-4}$ alkynyl, $C_{1-2}$ alkoxy, halo$C_{1-2}$ alkoxy, -$NHC_{1-2}$ alkyl, -N($C_{1-2}$ alkyl$)_2$, CN or $C_{3-6}$ cycloalkyl;

$R_5$ is $C_{1-2}$ alkyl, CN or =O substituted $C_{1-2}$ alkyl, halo$C_{1-2}$ alkyl, $C_{3-4}$ cycloalkyl, 4-membered to 7-membered heterocycloalkyl containing 1-3 heteroatoms selected from N, S, and O, $C_{1-2}$ alkoxy, halo$C_{1-2}$ alkoxy or $C_{3-4}$ cycloalkyloxy;
$R_8$ is selected from H, F, Cl, CN, $C_{1-2}$ alkyl, $C_{2-4}$ alkenyl, $C_{2-4}$ alkynyl, halo$C_{1-2}$ alkyl, $C_{1-2}$ alkoxy or halo$C_{1-2}$ alkoxy;
R is 4-membered or 5-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl, 6-membered to 10-membered spirocyclic heterocycloalkyl or -O-$C_{3-6}$ cycloalkyl, and the 4-membered monocyclic heterocycloalkyl is substituted with 1 to 3 groups selected from F, Cl, $C_{1-2}$ alkyl, $C_{1-2}$ alkoxy, OH, $NH_2$, -NH$C_{1-2}$ alkyl, -N($C_{1-2}$ alkyl)$_2$ and halo$C_{1-2}$ alkyl, the 6-membered to 8-membered monocyclic heterocycloalkyl, the 5-membered or 6-membered monocyclic heteroaryl, the 5-membered to 10-membered fused heterocycloalkyl, the 5-membered to 10-membered bridged heterocycloalkyl, the 6-membered to 10-membered spirocyclic heterocycloalkyl or the $C_{3-6}$ cycloalkyl is optionally substituted with 1 to 3 groups selected from F, Cl, $C_{1-2}$ alkyl, $C_{1-2}$ alkoxy, OH, $NH_2$, -NH$C_{1-2}$ alkyl, -N($C_{1-2}$ alkyl)$_2$ and halo$C_{1-2}$ alkyl.

**9.** The compound, and the stereoisomer, the deuterated product, or the pharmaceutically acceptable salt thereof of claim 8, **characterized in that**,

$R_3$ is CN;
each of $R_{31}$ and $R_{32}$ is independently H, F, Cl or $C_{3-6}$ cycloalkyl;
$R_5$ is selected from $C_{1-2}$ alkyl, halo$C_{1-2}$ alkyl, $C_{1-2}$ alkoxy, or CN or =O substituted $C_{1-2}$ alkyl;
$R_8$ is H;
R is 6-membered to 10-membered spirocyclic heterocycloalkyl containing 1 to 3 heteroatoms selected from N, S, and O, 4-membered or 5-membered monocyclic heterocycloalkyl containing 1 to 3 heteroatoms selected from N, S, and O, or -O-$C_{3-6}$ cycloalkyl, and the 6-membered to 10-membered spirocyclic heterocycloalkyl, the 4-membered or 5-membered monocyclic heterocycloalkyl or the $C_{3-6}$ cycloalkyl is optionally substituted with 1 to 3 groups selected from F, Cl, $C_{1-2}$ alkyl or $C_{1-2}$ alkoxy.

**10.** The compound, and the stereoisomer, the deuterated product, or the pharmaceutically acceptable salt thereof of claim 1, **characterized in that** the compound is selected from one of the structures in Table 1 or Table 2.

**11.** A pharmaceutical composition or pharmaceutical preparation, **characterized in that** the pharmaceutical composition or pharmaceutical preparation comprises the compound, and the stereoisomer, the deuterated product or the pharmaceutically acceptable salt thereof of any one of claims 1 to 10, and a pharmaceutically acceptable carrier and/or excipient.

**12.** The pharmaceutical composition or pharmaceutical preparation of claim 11, **characterized in that** the pharmaceutical composition or pharmaceutical preparation comprises 1 mg-1500 mg of the compound, and the stereoisomer, the deuterated product or the pharmaceutically acceptable salt thereof of any one of claims 1 to 10, and the pharmaceutically acceptable carrier and/or excipient.

**13.** The compound, and the stereoisomer, the deuterated product or the pharmaceutically acceptable salt thereof of any one of claims 1 to 10 for use in the preparation of a drug for the treatment/prevention of a BRAF-mediated disease.

**14.** A method for treating a disease in a mammal, **characterized in that** the method comprises administering to a subject a therapeutically effective amount of the compound, and the stereoisomer, the deuterated product or the pharmaceutically acceptable salt thereof of any one of claims 1 to 10, wherein the therapeutically effective amount is preferably 1 mg-1500 mg, and the disease is preferably a tumor, more preferably a brain tumor.

**INTERNATIONAL SEARCH REPORT**

International application No.
**PCT/CN2023/108142**

**A. CLASSIFICATION OF SUBJECT MATTER**

C07D471/04(2006.01)i; C07D 487/00(2006.01)i; C07D 487/04(2006.01)i; A61K 31/513(2006.01)i; A61K 31/517(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D471/-, C07D487/-, A61K31/-, A61P35/-

Documentation searched other than minimum documentation to the extent that such documents are included in the fields searched

Electronic data base consulted during the international search (name of data base and, where practicable, search terms used)

CNKI; CNTXT; WPABS; DWPI; ENTXT; STN(CAPLUS, REGISTRY, MARPAT): 西藏海思科, BRAF, 激酶, 抑制剂, 肿瘤, 癌症, kinase, inhibitor, cancer, tumor, tumour, 依据权利要求1式(I)化合物中Cy为P1, P3, P8的结构进行亚结构检索, sub-structure search according to the structure in which Cy is P1, P3 and P8 in the compound of formula (I) in claim 1

**C. DOCUMENTS CONSIDERED TO BE RELEVANT**

| Category* | Citation of document, with indication, where appropriate, of the relevant passages | Relevant to claim No. |
|---|---|---|
| X | CN 111247152 A (ABM THERAPEUTICS, INC.) 05 June 2020 (2020-06-05)<br>embodiments 1-13, 16-18, 21, 27, 37-44, 46, 48-49, 51-56, claims 1, 3-44 | 1-4, 10-14 |
| A | CN 111247152 A (ABM THERAPEUTICS, INC.) 05 June 2020 (2020-06-05)<br>embodiments 1-13, 16-18, 21, 27, 37-44, 46, 48-49, 51-56, claims 1, 3-44 | 6-9 |
| X | WO 2012118492 A1 (ARRAY BIOPHARMA INC.; GENENTECH, INC.; GRINA JONAS; HANSEN JOSHUA D.; LAIRD ELLEN; MATHIEU SIMON; MORENO DAVID; REN, Li; RUDOLPH JOACHIM; WENGLOWSKY STEVEN MARK;) 07 September 2012 (2012-09-07)<br>claims 21-25, description, embodiments 1-6, 25-37, 39, 44-47, 49 | 1-3, 6-14 |
| A | WO 2012118492 A1 (ARRAY BIOPHARMA INC.; GENENTECH, INC.; GRINA JONAS; HANSEN JOSHUA D.; LAIRD ELLEN; MATHIEU SIMON; MORENO DAVID; REN, Li; RUDOLPH JOACHIM; WENGLOWSKY STEVEN MARK;) 07 September 2012 (2012-09-07)<br>claims 21-25, description, embodiments 1-6, 25-37, 39, 44-47, 49 | 4 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2023** | **02 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/108142**

**C. DOCUMENTS CONSIDERED TO BE RELEVANT**

| Category* | Citation of document, with indication, where appropriate, of the relevant passages | Relevant to claim No. |
|---|---|---|
| X | WO 2021116055 A1 (F. HOFFMANN-LA ROCHE AG; HOFFMANN-LA ROCHE INC.;) 17 June 2021 (2021-06-17)<br>description, page 1 paragraphs 5-7, page 5 paragraphs 10-15, claims 1-4, 8, 12 | 1-3, 6-14 |
| A | WO 2021116055 A1 (F. HOFFMANN-LA ROCHE AG; HOFFMANN-LA ROCHE INC.;) 17 June 2021 (2021-06-17)<br>description, page 1 paragraphs 5-7, page 5 paragraphs 10-15, claims 1-4, 8, 12 | 4 |
| X | Steve Wenglowsky et al. "Highly Potent and Selective 3-N-methylquinazoline-4(3H)-one Based Inhibitors of B-RafV600E Kinase"<br>*Bioorganic & Medicinal Chemistry Letters,* Vol. 24, No. (8), 13 March 2014 (2014-03-13), pp. 1923-1927<br>ISSN: 0960-894x,<br>abstract, page 1925, Table 4 | 1-3, 6-14 |
| A | Steve Wenglowsky et al. "Highly Potent and Selective 3-N-methylquinazoline-4(3H)-one Based Inhibitors of B-RafV600E Kinase"<br>*Bioorganic & Medicinal Chemistry Letters,* Vol. 24, No. (8), 13 March 2014 (2014-03-13), pp. 1923-1927<br>ISSN: 0960-894x,<br>abstract, page 1925, Table 4 | 4 |

Form PCT/ISA/210 (second sheet) (July 2022)

**INTERNATIONAL SEARCH REPORT**

International application No.
**PCT/CN2023/108142**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

Claim 14 relates to a treatment method implemented on a human or animal body (PCT Rule 39.1(iv)). In spite of this, a search is still performed on the basis of the technical subject matter of the use of the compound in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The present International Searching Authority finds two inventions in the international application, namely,

the present application sets forth a compound represented by formula (I), a stereoisomer, deuterated compound, or pharmaceutically acceptable salt thereof

(claims 1-10), a pharmaceutical composition or pharmaceutical preparation thereof (claims 11-12), and a pharmaceutical use thereof (claims 13-14).

On the basis of the content disclosed in the description, the technical solutions described in the claims of the present application can be divided into the following two groups:

Group 1: a compound of formula (I) in claims 1-13 with Cy having a P1 structure, a stereoisomer, deuterated compound or pharmaceutically acceptable salt thereof, a pharmaceutical composition or pharmaceutical preparation thereof, and a pharmaceutical use thereof.

Group 2: a compound of formula (I) in claims 1-13 with Cy having P2-P8 structures, a stereoisomer, deuterated compound or pharmaceutically acceptable salt thereof, a pharmaceutical composition or pharmaceutical preparation thereof, and a pharmaceutical use thereof.

When the Markush grouping is for alternatives of chemical compounds, they are regarded as being of a similar nature where the following criteria are fulfilled: (A) all alternatives have a common property or activity; and (B) (1) a common structure is present, that is, a significant structural element is shared by all of the alternatives, or (B) (2) in cases where the common structure cannot be the unifying criteria, all alternatives belong to a recognized class of chemical compounds in the art to which the invention pertains.

The group definition of the compound of formula (I) in claim 1 of the present application is broad, and a common structure thereof is not a significant structural element, for example, see document WO 2021116055 A1 (publication date: 17 June 2021), which discloses a compound having the same use.

In addition, when Cy in formula (I) has different structures, the compounds represented thereby of formula (I) does not belong to a recognized class of chemical compounds in the art to which the invention pertains.

Therefore, the compounds of the two inventions cannot satisfy the above-mentioned criteria. Therefore, these inventions lack unity of invention, and do not comply with PCT Rule 13.

**INTERNATIONAL SEARCH REPORT**

International application No.
**PCT/CN2023/108142**

**Box No. III Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/108142**

| Patent document cited in search report | | | Publication date (day/month/year) | Patent family member(s) | | | Publication date (day/month/year) |
|---|---|---|---|---|---|---|---|
| CN | 111247152 | A | 05 June 2020 | BR | 112020005455 | A2 | 24 September 2020 |
| | | | | KR | 20200055034 | A | 20 May 2020 |
| | | | | EP | 3684772 | A1 | 29 July 2020 |
| | | | | RU | 2020113707 | A | 20 October 2021 |
| | | | | RU | 2020113707 | A3 | 23 December 2021 |
| | | | | US | 2022251089 | A1 | 11 August 2022 |
| | | | | WO | 2019060611 | A1 | 28 March 2019 |
| | | | | CA | 3076202 | A1 | 28 March 2019 |
| | | | | AU | 2018338098 | A1 | 07 May 2020 |
| | | | | MX | 2020003126 | A | 01 October 2020 |
| | | | | JP | 2020534373 | A | 26 November 2020 |
| | | | | US | 2020247813 | A1 | 06 August 2020 |
| | | | | US | 11254680 | B2 | 22 February 2022 |
| WO | 2012118492 | A1 | 07 September 2012 | None | | | |
| WO | 2021116055 | A1 | 17 June 2021 | CO | 2022008968 | A2 | 19 July 2022 |
| | | | | JP | 2022124458 | A | 25 August 2022 |
| | | | | ZA | 202204675 | B | 21 December 2022 |
| | | | | CL | 2022001529 | A1 | 10 February 2023 |
| | | | | AU | 2020403443 | A1 | 12 May 2022 |
| | | | | AU | 2020403443 | B2 | 23 February 2023 |
| | | | | TW | 202136245 | A | 01 October 2021 |
| | | | | AR | 122351 | A1 | 07 September 2022 |
| | | | | CR | 20220251 | A | 11 July 2022 |
| | | | | PE | 20221778 | A1 | 16 November 2022 |
| | | | | US | 2022298119 | A1 | 22 September 2022 |
| | | | | IL | 292161 | A | 01 June 2022 |
| | | | | BR | 112022011123 | A2 | 23 August 2022 |
| | | | | KR | 20220110554 | A | 08 August 2022 |
| | | | | JP | 2022531609 | A | 07 July 2022 |
| | | | | JP | 7108146 | B2 | 27 July 2022 |
| | | | | MX | 2022006783 | A | 11 July 2022 |
| | | | | CA | 3162883 | A1 | 17 June 2021 |
| | | | | EP | 4073065 | A1 | 19 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021116050 A1
- WO 20171660 A1
- WO 201960611 A1
- WO 2021116055 A1